# EUROPEAN PATENT SPECIFICATION

(11) **EP 4 380 921 B1**
(45) Date of publication and mention of the grant of the patent: **28.01.2026**
(21) Application number: 22761746.1
(22) Date of filing: 03.08.2022
(51) Int. Cl.: C07D 207/416, A61P 25/18, A61P 25/30, A61K 31/40

(54) **D3 DOPAMINE RECEPTOR-SELECTIVE NEGATIVE ALLOSTERIC MODULATORS**
D3-DOPAMINREZEPTORSELEKTIVE NEGATIVE ALLOSTERISCHE MODULATOREN
MODULATEURS ALLOSTÉRIQUES NÉGATIFS SÉLECTIFS DU RÉCEPTEUR D3 DE LA DOPAMINE

(30) Priority: 03.08.2021 US 202163228660 P
(43) Date of publication of application: 12.06.2024
(73) Proprietor: The United States of America, as represented by The Secretary, Department of Health and Human Services, Bethesda, MD 20892-7788 (US); The University of North Carolina at Chapel Hill, Chapel Hill, NC 27514 (US)
(72) Inventor: GANDHI, Disha, Carlsbad, California 92011 (US); FRANKOWSKI, Kevin, Pittsboro, North Carolina 27312 (US); FERRER, Marc, Bethesda, Maryland 20892-7788 (US); FREE, R. Benjamin, Bethesda, Maryland 20892-7788 (US); SIBLEY, David R., Bethesda, Maryland 20892-7788 (US); MORITZ, Amy E., Bethesda, Maryland 20892-7788 (US); SOUTHALL, Noel Terrence, Bethesda, Maryland 20892-7788 (US); HU, Xin, Bethesda, Maryland 20892-7788 (US); KELLEY, Amber, Grayslake, Illinois 60030 (US)
(74) Representative: Harris, Oliver John Richard
(86) International application number: PCT/US2022/039245
(87) International publication number: WO 2023/014768

(56) References cited:
- WO-A1-2012/162249
- WO-A1-2016/090313
- WO-A1-2017/160552
- WO-A2-2014/144606
- VIVEK KUMAR ET AL: "Highly Selective Dopamine D 3 Receptor (D 3 R) Antagonists and Partial Agonists Based on Eticlopride and the D 3 R Crystal Structure: New Leads for Opioid Dependence Treatment", JOURNAL OF MEDICINAL CHEMISTRY, vol. 59, no. 16, 25 August 2016 (2016-08-25), US, pages 7634 - 7650, XP055370771, ISSN: 0022-2623, DOI: 10.1021/acs.jmedchem.6b00860
- JIANYONG CHEN ET AL: "Tranylcypromine Substituted cis -Hydroxycyclobutylnaphthamides as Potent and Selective Dopamine D 3 Receptor Antagonists", JOURNAL OF MEDICINAL CHEMISTRY, vol. 57, no. 11, 12 June 2014 (2014-06-12), pages 4962 - 4968, XP055179856, ISSN: 0022-2623, DOI: 10.1021/jm401798r

## Description

### STATEMENT OF GOVERNMENT SUPPORT

This invention was made in part with government support from the US Department of Health and Human Services, National Institutes of Health. The government has certain rights in this invention.

### BACKGROUND

G-protein coupled receptors (GPCRs) are among the most intensely investigated drug targets in the pharmaceutical industry. Over 40% of all FDA approved drugs target these important receptor proteins. Unfortunately, many of the ligands that are used as drugs or pharmacological tools are not selective and exhibit some unintended activity on non-target GPCRs or other proteins. This is because the orthosteric binding site, where the endogenous ligand binds, is highly conserved among closely related types of GPCRs.

Dopamine receptors (DARs) are members of the G protein-coupled receptor (GPCR) superfamily which play a critical role in cell signaling processes, especially modulating the transfer of information within the nervous system. Members of the DAR subfamilies share high sequence homology, especially the D2 and D3 DARs. Therefore, most currently available dopaminergic drugs, including antipsychotics and anti-Parkinson's disease (PD) medications, cross-react with both subtypes to varying degrees. In addition, most D2/D3 DAR compounds also cross-react with other related GPCRs creating the potential for profound off-target side effects.

As the dopaminergic system is intricately involved in the regulation of mood, pleasure and reward, selective modulation of DAR signaling, particularly that of the D3 DAR, could in theory alleviate components of substance use disorder (SUD) pathology (Newman, AH; et al., (2012) Medication discovery for addiction: Translating the dopamine D3 receptor hypothesis. Biochem. Pharmacol. 84, 882-890). As such, a number of D3 DAR antagonists and partial agonists are being investigated as a therapeutic approach to treating substance abuse, both in disrupting drug seeking motivation and preventing relapse (Heidbreder, CA, et al., (2005) The role of central dopamine D3 receptors in drug addiction: a review of pharmacological evidence. Brain Res. Rev. 49, 77-105). D3 DAR antagonists and partial agonists have shown promise in both diminishing drug-seeking behavior and preventing relapse incidents in cocaine-, methamphetamine, nicotine-, and heroin-conditioned animals. Recent studies suggest that D3 DAR antagonism may be broadly effective in treating opioid use disorder (OUD) (Boateng, CA; et al., (2015)). High affinity dopamine D3 receptor (D3 DAR)-selective antagonists attenuate heroin self-administration in wild-type but not D3 DAR knockout mice. (J. Med. Chem. 58, 6195-6213). Further, D3 DAR-preferring ligands have clinical potential in treating Parkinson's disease and related syndromes, dyskinesia, especially dyskinesias secondary to treating Parkinson's disease with L-DOPA, neurodegenerative disorders such as Alzheimer's disease and dementia, restless legs syndrome, depression, schizophrenia, cognitive dysfunction, and substance use disorders, including addiction to alcohol, nicotine, cocaine, methamphetamine, and opioids.

Despite the promise of this drug target, concerns of off-target side effects have tempered interest in the clinical utility of currently available D3 DAR antagonists as they lack selectivity. D2 and D3 DARs share 74% amino acid homology in their transmembrane spanning domains and are 94% identical in their orthosteric binding sites, where DA binds. Therefore, it may not be surprising that current compounds targeting either the D2 DAR or D3 DAR, including antipsychotics, anti-Parkinson's disease medications, and known research tool compounds, lack high selectivity not only between these subtypes but frequently with many other GPCRs as well.

Drug overdose deaths have increased 3.6-fold in the past decade driven largely by rising opioid abuse, underscoring the urgent need to identify new SUD therapies (Hedegaard, H., et al., (2018) NCHS Data Brief 329, 1-8). The comorbidity of substance abuse and neuropsychiatric disorders is known-people with SUD are ~5-fold more likely to suffer mood disorders, further complicating treatment regimens (Regier, DA; et al., (1990) JAMA 264, 2511-2518; Brady, KT; et al., (2005) Subst. Use Misuse 40, 2021-2041). Thus, in a complementary approach to treating SUDs, D3 DAR selective antagonists have also been proposed as therapeutics for psychiatric conditions, as most antipsychotic agents possessing D3 DAR antagonism in addition to the intended D2 DAR antagonism. Thus, highly selective D3 DAR antagonists may also have utility in the treatment of schizophrenia, potentially with reduced side effects, including dyskinesia and metabolic dysregulation, which are typically observed with D2 DAR blockade using current antipsychotic drugs.

This disclosure fulfills an unmet need for highly selective D3 DAR allosteric antagonists that may provide significant advantages over currently known compounds for the treatment of a variety of dopaminergic related pathologies. Note that WO2016090313A1 discloses compounds for modulating inclusion formation and stress granules in cells related to the onset of neurodegenerative diseases, musculoskeletal diseases, cancer, ophthalmological diseases, and viral infections, WO2012162249A1 discloses methods comprising contacting a cell with an inclusion inhibitor and methods for screening for modulators of TDP-43 aggregation, and WO2014144606 discloses methods for treating muscular dystrophy.

### SUMMARY

The claimed invention provides a compound of Formula I or a pharmaceutically acceptable salt thereof, 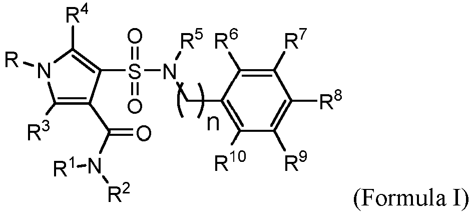 wherein in Formula I:
n is 0 or 1;
R is hydrogen;
R¹ is chosen from hydrogen, methyl, and ethyl, and R² is chosen from methyl and ethyl; or
R¹ and R² are joined to form a heterocycloalkyl ring optionally containing 1 additional heteroatom chosen from N, O, and S, and the heterocycloalkyl ring is unsubstituted or substituted by 1 or more substituents independently chosen from halogen, hydroxyl, amino, C₁-C₃alklyl, C₁-C₃alkoxy, C₁-C₂haloalkyl, and C₁-C₂haloalkoxy;
R³ and R⁴ are independently chosen from hydrogen, methyl, ethyl, and trifluoromethyl;
R⁵ is chosen from hydrogen, methyl, and ethyl, and
R⁶, R⁷, R⁸, R⁹, and R¹⁰ are independently chosen from hydrogen, halogen, hydroxyl, amino, - SF₅, C₁-C₄alkyl, C₁-C₄alkoxy, C₃-C₆cycloalkyl, C₁-C₄alkylester, C₁-C₂haloalkyl, and C₁-C₂haloalkoxy; wherein at least one of R⁷, R⁸, and R⁹ is not hydrogen. Described, but not according to the claimed invention, is Formula I above wherein R is methyl or ethyl.

Described, but not according to the claimed invention, is a compound of Formula II and a pharmaceutically acceptable salt thereof, 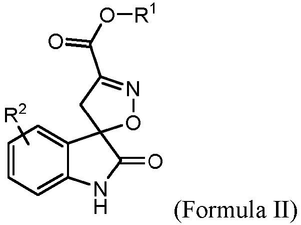 wherein in Formula II,
R¹ is C₁-C₄ alkyl;
R² is 1 or more optional substituents independently chosen from halogen, hydroxyl, amino, cyano, or C₁-C₆alkyl, in which any one CH₂ in the C₁-C₆alkyl is optionally replaced by NR³, O, S, or SO₂ and which C₁-C₆alkyl is optionally substituted by one or more substituents independently chosen from halogen, hydroxyl, amino, oxo, and cyano; and
R³ is hydrogen or C₁-C₄alkyl.

Also described, but not according to the claimed invention, is a compound of Formula III and a pharmaceutically acceptable salt thereof, 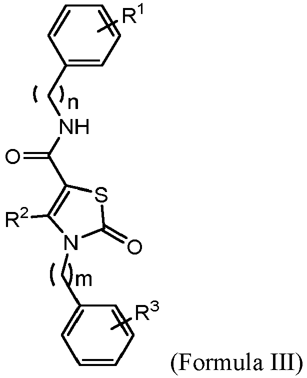 wherein in Formula III,
n is 0, 1, or 2;
m is 0, 1, or 2;
R¹ and R³ are each 1 or more optional substituents independently chosen from halogen, hydroxyl, amino, cyano, or C₁-C₆alkyl, in which any one CH₂ in the C₁-C₆alkyl is optionally replaced by NR⁴, O, S, or SO₂ and which C₁-C₆alkyl is optionally substituted by one or more substituents independently chosen from halogen, hydroxyl, amino, oxo, and cyano; and
R² and R⁴ are each independently hydrogen or C₁-C₄alkyl.

Also disclosed but not as such according to the claimed invention, is a method of treating central nervous system disorders, comprising Parkinson's disease and related syndromes, dyskinesias, neurodegenerative disorders, schizophrenia, cognitive dysfunction, or substance use disorder and/or withdrawal syndrome in a patient, comprising a step of providing to a patient in need thereof a therapeutic agent, wherein the therapeutic agent is a compound or salt thereof of any one of compounds of Formula I, Formula II, or Formula III.

### BRIEF DESCRIPTION OF THE DRAWINGS

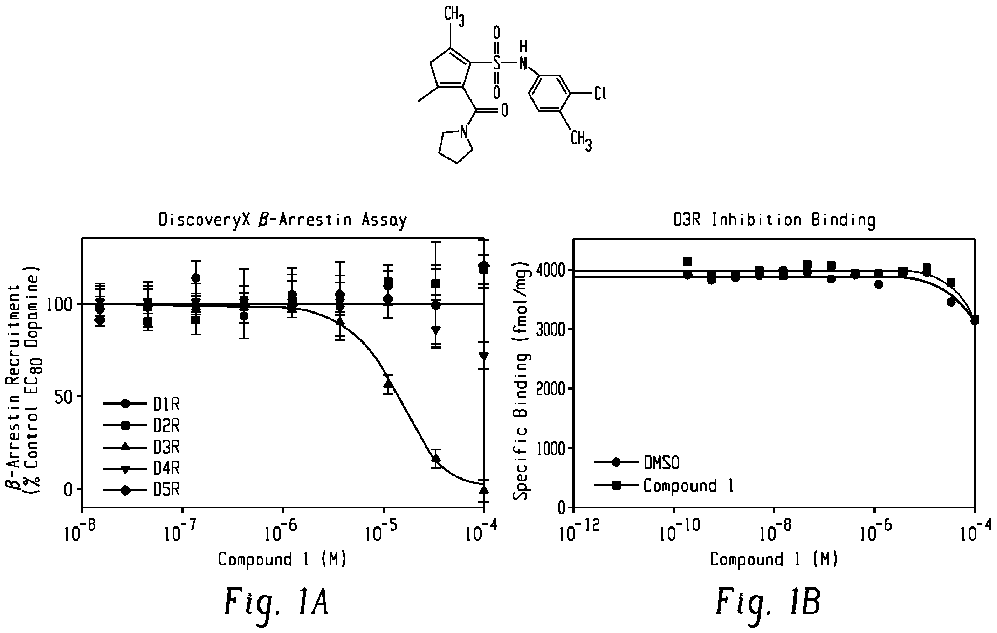

FIGURE 1: Cmp. 1 is a selective D3 DAR NAM (Negative Allosteric Modulator), FIG. 1A shows that Cmp. 1 is a selective for the D3 DAR vs. other dopamine receptors (DARs) as assessed in the DiscoverX β-arrestin assay; FIG. 1B shows that Cmp. 1 lacks the ability to inhibit the binding of the orthosteric antagonist [³H]-methylspiperone to the D3 DAR, supporting its allosteric mechanism of action;

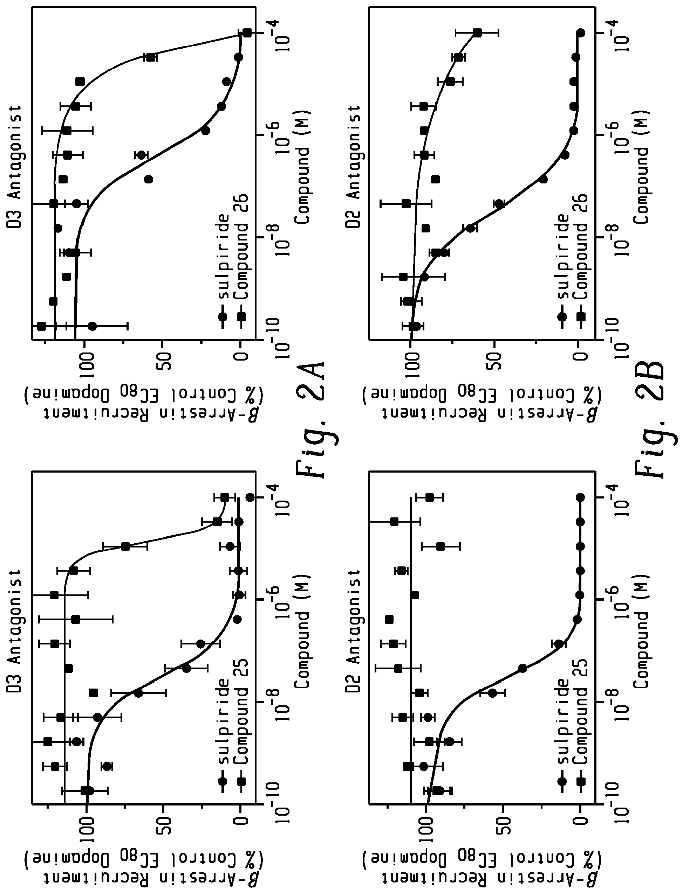

FIGURE 2: Cmp. 25 and Cmp. 26 (not according to the claimed invention) are selective D3 DAR NAMs as compared with the non-selective orthosteric antagonist sulpiride, FIG. 2A shows that Cmp. 25 (left) and Cmp. 26 (right) inhibit the D3 DAR as assessed in the DiscoverX β-arrestin assay, respectively; FIG. 2B shows, Cmp. 25 (left) and Cmp. 26 (right) lack ability to antagonize dopamine activation of the D2R as assessed in the DiscoverX β-arrestin assay;

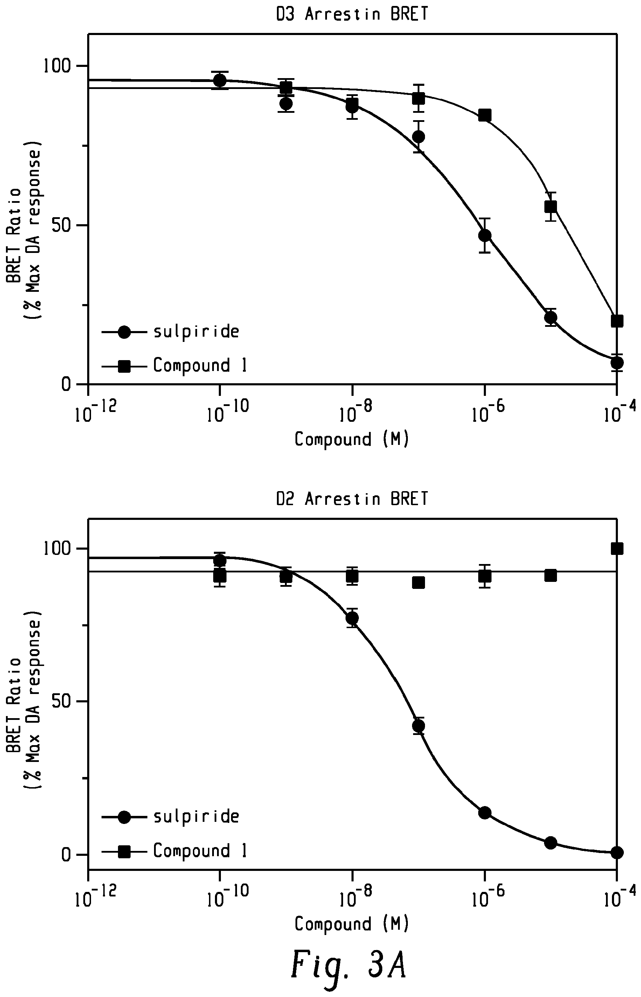

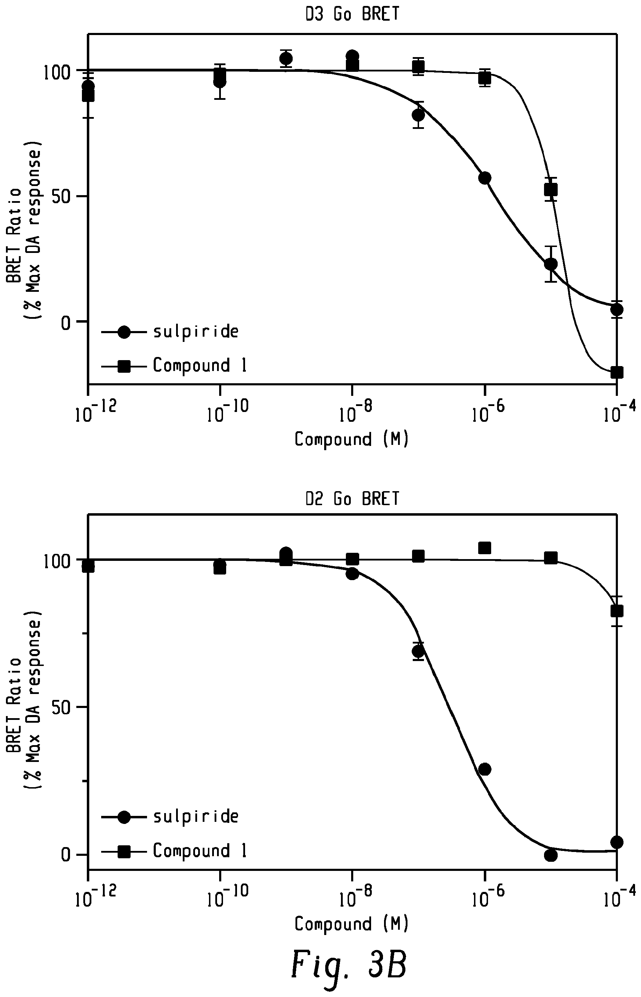

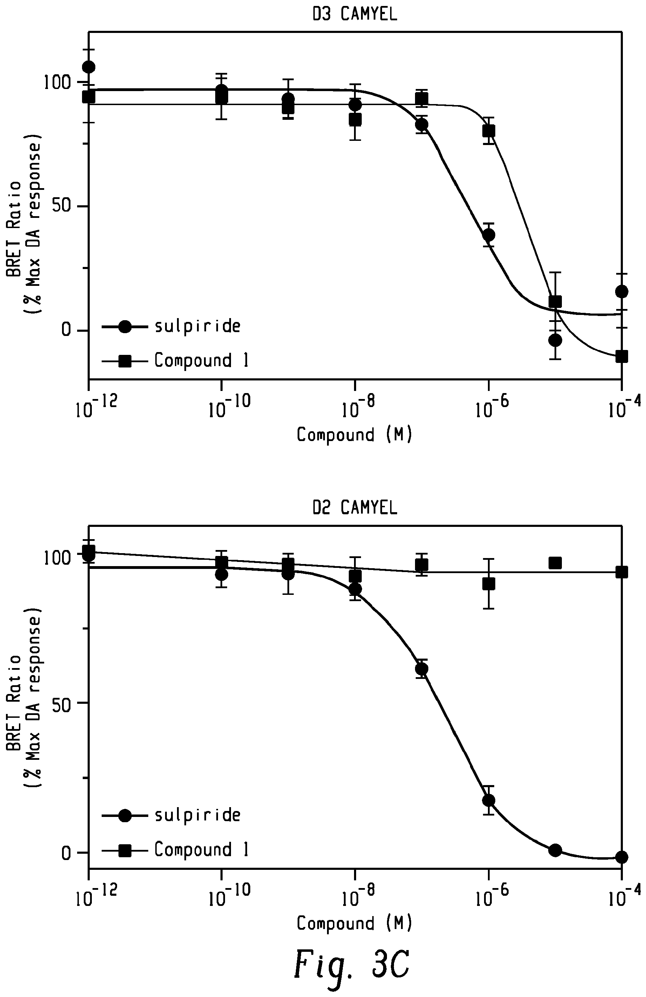

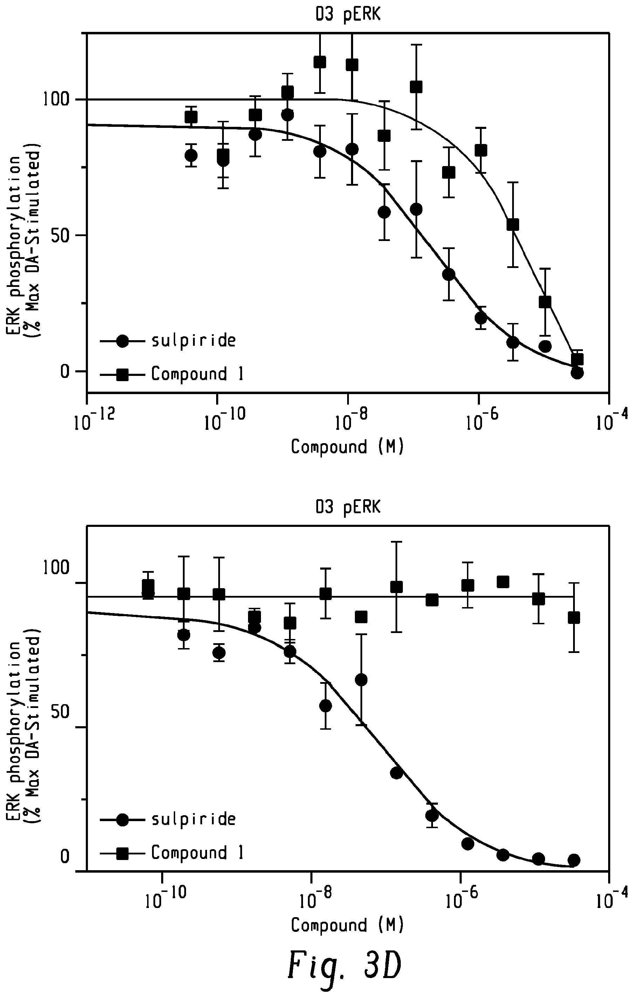

FIGURE 3: Cmp. 1 is a selective D3 DAR antagonist as assessed in multiple signaling outputs as compared with the non-selective orthosteric antagonist sulpiride, FIG. 3A shows a BRET-based β-arrestin assay; FIG. 3B shows a BRET-based assay of Go activation; FIG. 3C shows a BRET-based CAMYEL cAMP accumulation assay; and FIG. 3D shows an antibody capture assay used to assess pERK levels;

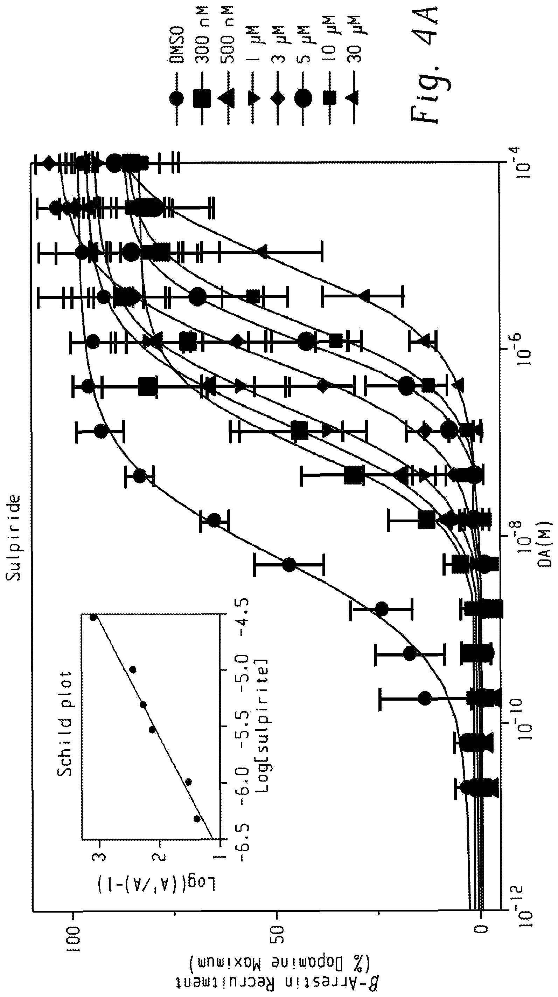

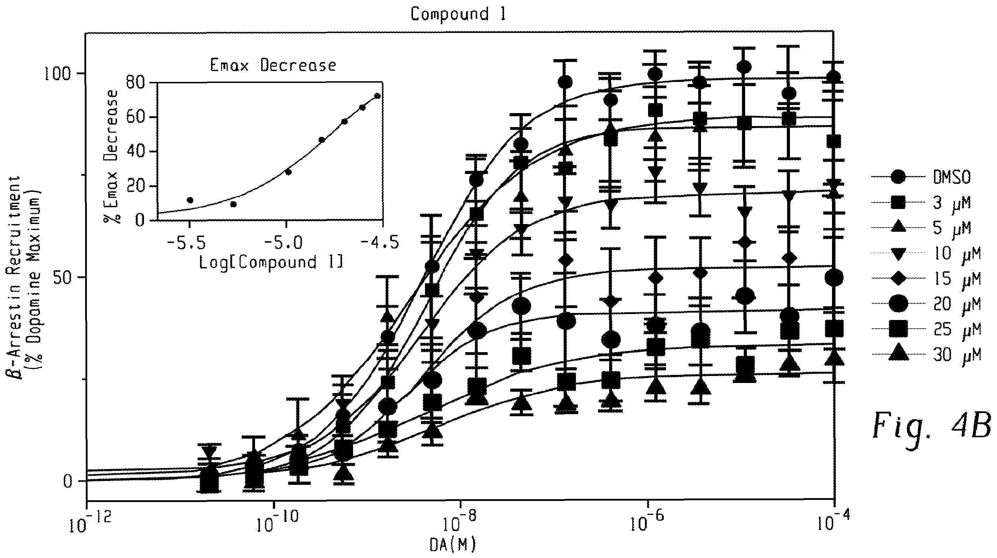

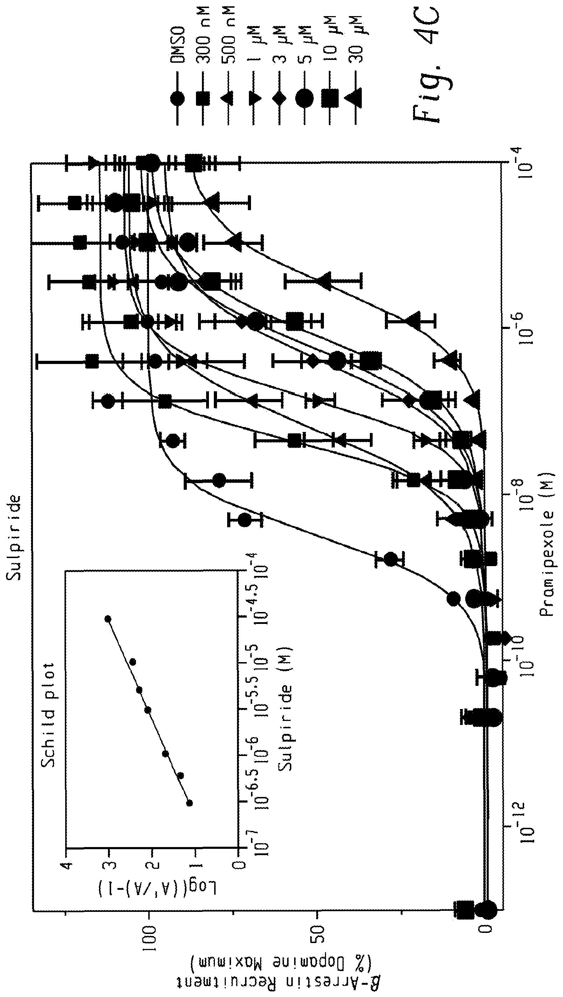

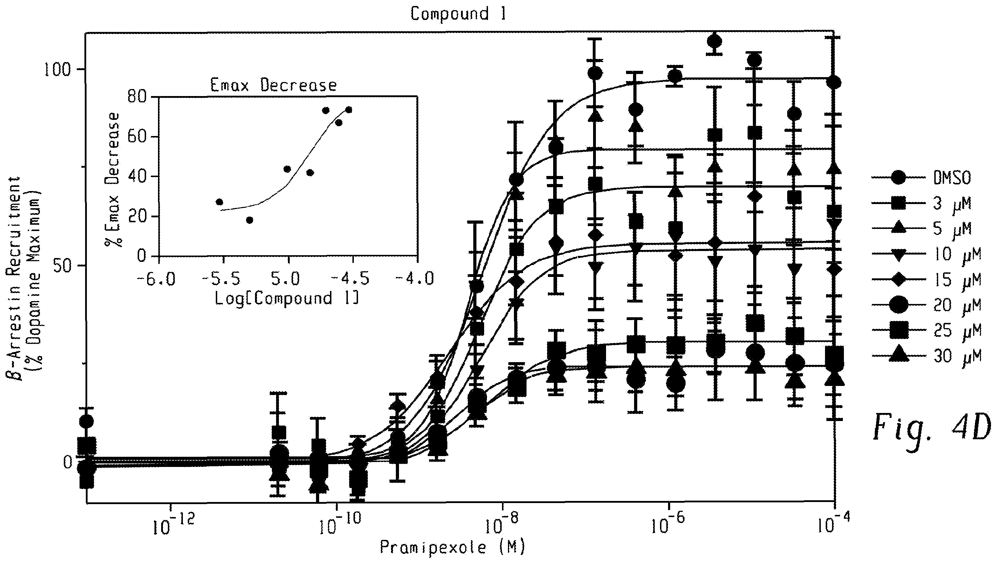

FIGURE 4: functional curve shift assays for Cmp. 1 indicating that it behaves as a noncompetitive antagonist at the D3 DAR Cells expressing the D3R were incubated with dopamine (FIG. 4A and 4B) or pramipexole (FIG. 4C and 4D) in the presence or absence of increasing concentrations of either the orthosteric antagonist sulpiride (FIG. 4A and 4C) or Cmp. 1 (FIG. 4B and 4D). The data and Schild regression plots (*insets,* *Fig. 4A**,* C) indicate that sulpiride behaves in a competitive manner with dopamine and pramipexole while Cmp. 1 behaves in a non-competitive manner with both agonists (*insets show Emax decrease as a function of Cmp.1 concentration,* *Fig. 4B**, D).* Average curves are shown (mean ± SEM from at least 3 experiments each performed in triplicate);

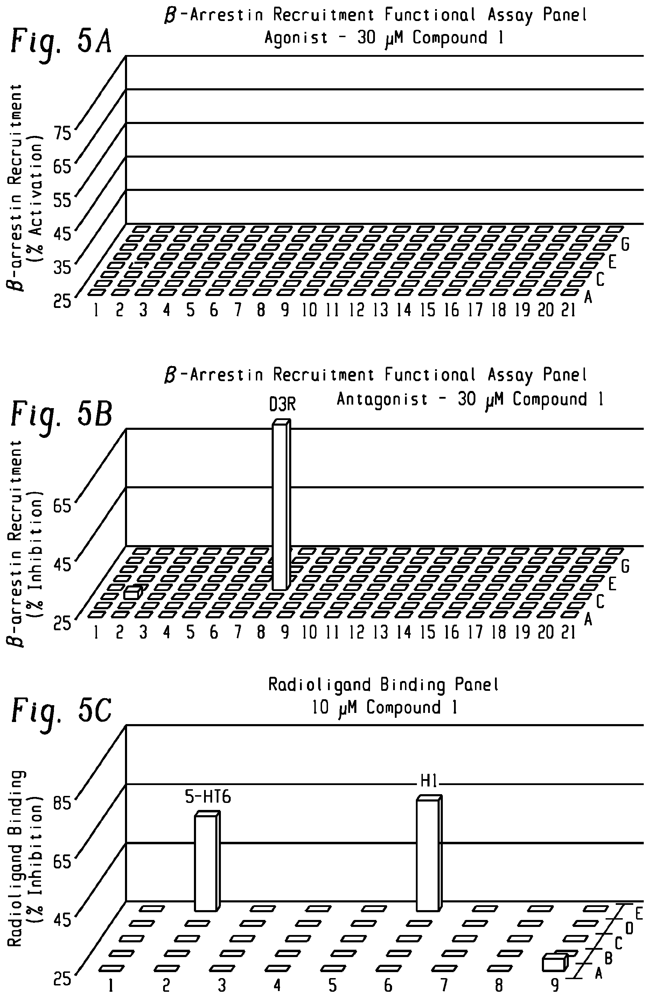

FIGURE 5: Cmp. 1 is highly selective for the D3 DAR when screened in the DiscoverX gpcrMAX^{™} β-arrestin recruitment assay panel of 168 unique GPCRs (each bar represents a different GPCR. The key is shown below the graph, along with the corresponding % activation (FIG 5A) or inhibition (FIG 5B)); FIG. 5A shows Cmp. 1 tested as an agonist; FIG. 5B shows Cmp. 1 tested as an antagonist, and FIG. 5C shows that Cmp. 1 also showed limited off-target affinity in a radioligand binding panel of 45 different GPCRs, monoamine transporters and ion channels;

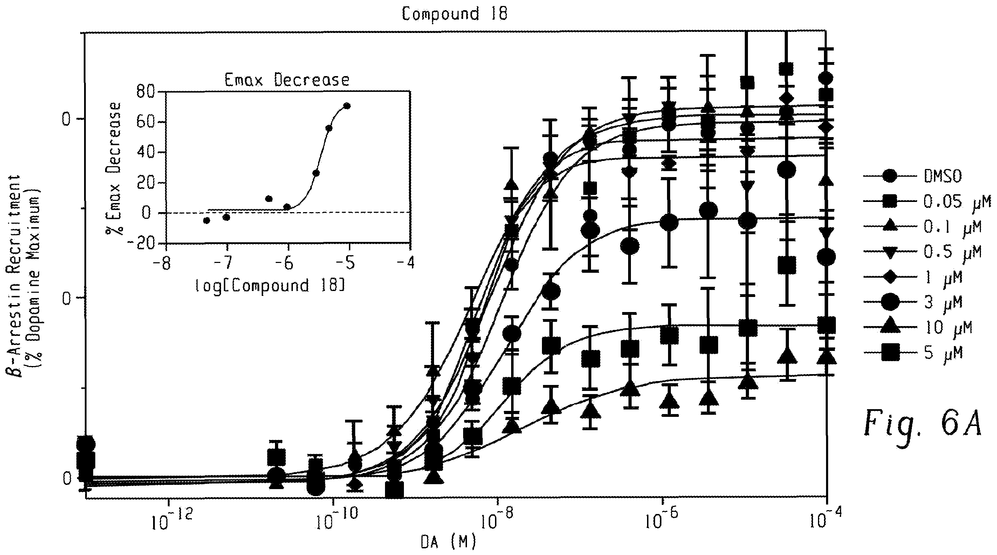

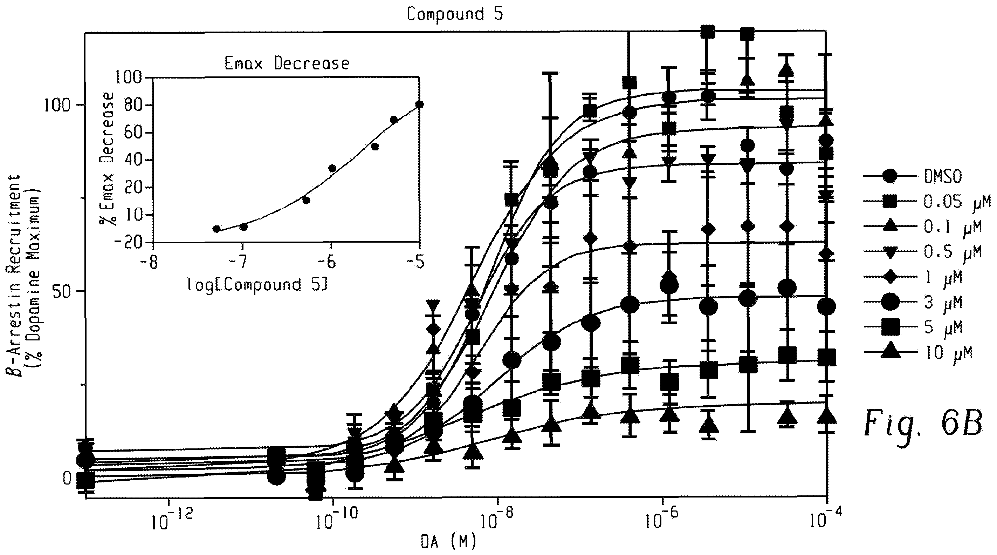

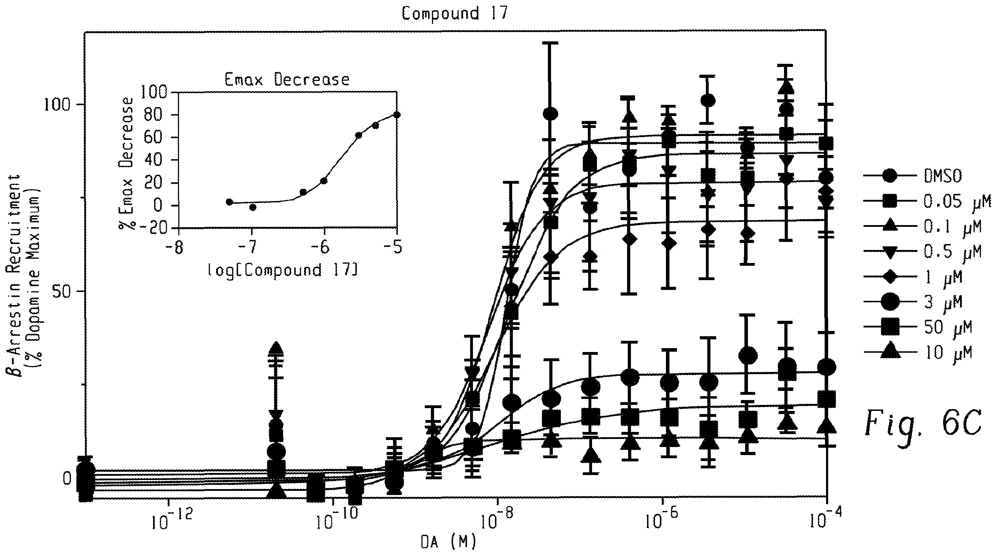

FIGURE 6: functional curve shift assays for Cmp. 18 (FIG. 6A), Cmp. 5 (FIG. 6B), and Cmp. 17 (FIG. 6C) showing that they exhibit noncompetitive antagonism of the D3 DAR consistent with an allosteric mechanism of action.

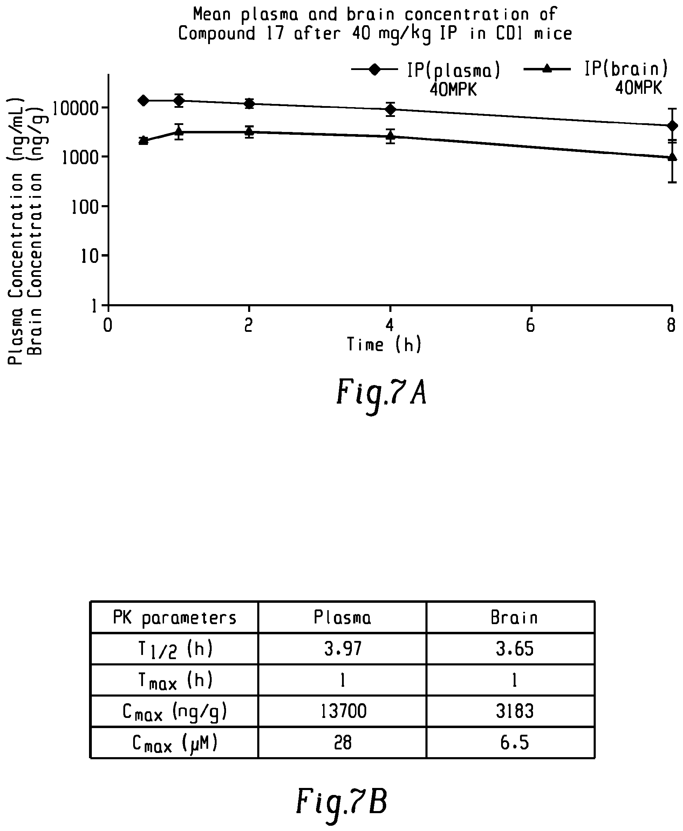

FIGURE 7: Pharmacokinetic data showing cmp 17 is brain penetrant and achieves concentrations that should antagonize the D3 DAR in vivo.

### DETAILED DESCRIPTION

### TERMINOLOGY

Compounds of the present disclosure are generally described using standard nomenclature.

The terms "a" and "an" do not denote a limitation of quantity, but rather denote the presence of at least one of the referenced item. The term "or" means "and/or." The open-ended transitional phrase "comprising" encompasses the intermediate transitional phrase "consisting essentially of" and the close-ended phrase "consisting of." Claims reciting one of these three transitional phrases, or with an alternate transitional phrase such as "containing" or "including" can be written with any other transitional phrase unless clearly precluded by the context or art. Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable. All methods described herein can be performed in a suitable order unless otherwise indicated herein or otherwise clearly contradicted by context. The use of any and all examples, or exemplary language (e.g., "such as"), is intended merely to better illustrate the disclosure and does not pose a limitation on its scope unless otherwise claimed. No language in the specification should be construed as indicating any non-claimed element as essential to the practice of the invention as used herein. Unless defined otherwise, technical and scientific terms used herein have the same meaning as is commonly understood by one of skill in the art to which this disclosure belongs.

Formulae I, II, and III include all salts of Formulae I, II, and III and all subformulae such as Formula I-A.

In certain situations, the compounds of Formulae I, II, and III may contain one or more asymmetric elements such as stereogenic centers, stereogenic axes and the like, e.g. asymmetric carbon atoms, so that the compounds can exist in different stereoisomeric forms. Formulae I, II, and III include all stereoisomeric forms, including racemates, optically enriched, and optically pure forms. In addition, compounds with carbon-carbon double bonds may occur in Z- and E-forms, with all isomeric forms of the compounds being included in the present disclosure. In these situations, the single enantiomers, i.e., optically active forms can be obtained by asymmetric synthesis, synthesis from optically pure precursors, or by resolution of the racemates. Resolution of the racemates can also be accomplished, for example, by conventional methods such as crystallization in the presence of a resolving agent, or chromatography, using, for example, a chiral HPLC column.

The disclosure of Formulae I, II, and III include all isotopes of atoms occurring in the present compounds. Isotopes include those atoms having the same atomic number but different mass numbers. By way of general example, and without limitation, isotopes of hydrogen include tritium and deuterium and isotopes of carbon include ¹¹C, ¹³C, and ¹⁴C and isotopes of fluorine including ¹⁹F.

Certain compounds are described herein using a general formula that includes variables, e.g., R¹, R², R³, R⁴, R⁵, R⁶, R⁷, R⁸, and R⁹. Unless otherwise specified, each variable within such a formula is defined independently of other variables. Thus, if a group is said to be substituted, e.g., with 0-2 R*, then said group may be substituted with up to two R* groups and R* at each occurrence is selected independently from the definition of R*. When a group is substituted by an "oxo" substituent a carbonyl bond replaces two hydrogen atoms on a carbon. An "oxo" substituent on an aromatic group or heteroaromatic group destroys the aromatic character of that group, e.g., a pyridyl substituted with oxo is a pyridone.

Combinations of substituents and/or variables are permissible only if such combinations result in stable compounds. A stable compound or stable structure is meant to imply a compound that is sufficiently robust to survive isolation from a reaction mixture, and subsequent formulation into an effective therapeutic agent.

The term "substituted" means that any one or more hydrogen atoms bound to the designated atom or group is replaced with a selection from the indicated group, provided that the designated atom's normal valence is not exceeded. When the substituent is oxo (i.e., =O), then 2 hydrogens on the atom are replaced. When aromatic moieties are substituted by an oxo group, the aromatic ring is replaced by the corresponding partially unsaturated ring. For example, a pyridyl group substituted by oxo is a pyridone. Combinations of substituents and/or variables are permissible only if such combinations result in stable compounds or useful synthetic intermediates. A stable compound or stable structure is meant to imply a compound that is sufficiently robust to survive isolation from a reaction mixture, and subsequent formulation into an effective therapeutic agent. Unless otherwise specified substituents are named into the core structure. For example, it is to be understood that when (cycloalkyl)alkyl is listed as a possible substituent the point of attachment of this substituent to the core structure is in the alkyl portion.

Substituents are named into the ring unless otherwise indicated. A dash ("-") or a double bond ("=") that is not between two letters or symbols indicates the point of attachment for a substituent. For example, -CONH₂ is attached through the carbon atom.

An "active agent" means a compound (including a compound disclosed herein), element, or mixture that when administered to a patient, alone or in combination with another compound, element, or mixture, confers, directly or indirectly, a physiological effect on the subject. The indirect physiological effect may occur via a metabolite or other indirect mechanism. The "active agent" may also potentiate or make more active another active agent. For example, the compounds of Formulae I, II, and III potentiate the activity of other active agents when given in combination with another active agent, for example by lowering the effective dose of the other active agent.

An "aliphatic group" is a non-aromatic hydrocarbon group having the indicated number of carbon atoms. Aliphatic groups may be saturated, unsaturated, or cyclic.

"Alkyl" includes both branched and straight-chain saturated aliphatic hydrocarbon groups, having the specified number of carbon atoms. Thus, the term C₁-C₆alkyl includes alkyl groups having from 1 to about 6 carbon atoms. When C₀-Cₙalkyl is used herein in conjunction with another group, for example, (cycloalkyl)C₀-C₂ alkyl, the indicated group, in this case cycloalkyl, is either directly bound by a single covalent bond (C₀), or attached by an alkyl chain having the specified number of carbon atoms, in this case from 1 to about 2 carbon atoms. Examples of alkyl include, but are not limited to, methyl, ethyl, n-propyl, isopropyl, n-butyl, t-butyl, n-pentyl, and sec-pentyl. C₁-C₄alkyl includes alkyl groups having 1, 2, 3, or 4 carbon atoms.

"Alkoxy" is an alkyl group as defined above with the indicated number of carbon atoms attached through an oxygen bridge. Examples of alkoxy include, but are not limited to, methoxy, ethoxy, n-propoxy, i-propoxy, n-butoxy, 2-butoxy, t-butoxy, n-pentoxy, 2-pentoxy, 3-pentoxy, isopentoxy, neopentoxy, n-hexoxy, 2-hexoxy, 3-hexoxy, and 3-methylpentoxy. Similarly, an "Alkylthio" or a "thioalkyl" group is an alkyl group as defined above with the indicated number of carbon atoms covalently bound to the group it substitutes by a sulfur bridge (-S-).

"Haloalkyl" includes both branched and straight-chain saturated aliphatic hydrocarbon groups, having the specified number of carbon atoms, substituted with 1 or more halogen atoms, generally up to the maximum allowable number of halogen atoms. Thus, the term C₁- C₆haloalkyl includes haloalkyl groups having from 1 to about 6 carbon atoms. When C₀-Cₙhaloalkyl is used herein in conjunction with another group, for example, (cycloalkyl)C₀-C₂ haloalkyl, the indicated group, in this case cycloalkyl, is either directly bound by a single covalent bond (C₀), or attached by an haloalkyl chain having the specified number of carbon atoms, in this case from 1 to about 2 carbon atoms, substituted with 1 or more halogen atoms, generally up to the maximum allowable number of halogen atoms. Examples of haloalkyl include, but are not limited to, trifluoromethyl, difluoromethyl, 2-fluoroethyl, chloromethyl, chloroethyl, and penta-fluoroethyl. C₁-C₂alkyl includes alkyl groups having 1or 2 carbon atoms, substituted with 1 or more halogen atoms, generally up to the maximum allowable number of halogen atoms.

"Haloalkoxy" is an haloalkyl group as defined above with the indicated number of carbon atoms attached through an oxygen bridge. Examples of haloalkoxy include, but are not limited to, chloromethoxy, chloroethoxy, bromo-n-propoxy, bromo-i-propoxy, iodo-n-butoxy, iodo-2-butoxy, or chloro-n-pentoxy.

"Cycloalkyl" is a saturated hydrocarbon ring groups, having the specified number of carbon atoms, usually from 3 to 7 carbon atoms. Examples of cycloalkyl groups include cyclopropyl, cyclobutyl, cyclopentyl, or cyclohexyl as well as bridged or caged saturated ring groups such as norborane or adamantane. In the term "(cycloalkyl)alkyl," cycloalkyl and alkyl are as defined above, and the point of attachment in on the alkyl group.

"Halo" or "halogen" as used herein refers to fluoro, chloro, bromo, or iodo.

"Heterocycloalkyl" is a stable monocyclic ring having the indicated number of ring atoms which contains from 1 to 3, or in some embodiments from 1 to 2, heteroatoms chosen from N, O, and S, with remaining ring atoms being carbon, or a stable bicyclic or tricyclic system containing at least one 5- to 7-membered cyclic ring which contains from 1 to 3, or in some embodiments from 1 to 2, heteroatoms chosen from N, O, and S, with remaining ring atoms being carbon. Monocyclic heterocycloalkyl groups typically have from 5 to 7 ring atoms. The stable monocyclic heterocycloalkyl may have 3-10 ring atoms which contains from 1 to 3, or in some embodiments from 1 to 2, heteroatoms chosen from N, O, and S, with remaining ring atoms being carbon. In some embodiments bicyclic heterocycloalkyl groups are 9- to 10-membered heterocycloalkyl groups, that is, groups containing 9 or 10 ring atoms in which one 5- to 7-member cyclic ring is fused to a second aromatic or non-aromatic ring. It is preferred that the total number of S and O atoms in the heteroaryl group is not more than 2. Examples of heterocycloalkyl groups include, but are not limited to, azetidinyl, pyrrolidinyl, piperidinyl, or piperazinyl.

"Pharmaceutical compositions" are compositions comprising at least one active agent, such as a compound or salt of Formula I or Formula II or Formula III, or a pharmaceutically acceptable salt, derivative, or solvate thereof, and at least one other excipient, such as a carrier. "Carriers" are any inactive materials, including excipients and diluents, which may be added to the pharmaceutical compositions including carriers and diluents. Pharmaceutical compositions meet the U.S. FDA's GMP (good manufacturing practice) standards for human or non-human drugs. Also included are any and all solvents, dispersion media, coatings, antibacterial and antifungal agents, and isotonic and absorption delaying agents. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions. A "pharmaceutically acceptable carrier" includes both one and more than one such carrier.

"Pharmaceutically acceptable salt" includes derivatives of the disclosed compounds wherein the parent compound is modified by making non-toxic acid or base salts thereof, and further refers to pharmaceutically acceptable hydrates or solvates of such compounds and such salts. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts and the quaternary ammonium salts of the parent compound formed, for example, from non-toxic inorganic or organic acids. For example, conventional non-toxic acid salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxylmaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH₂)ₙ-COOH where n is 0-4, and the like. Lists of additional suitable salts may be found, e.g., in G. Steffen Paulekuhn, et al., Journal of Medicinal Chemistry 2007, 50, 6665 and Handbook of Pharmaceutically Acceptable Salts: Properties, Selection and Use, P. Heinrich Stahl and Camille G. Wermuth Editors, Wiley-VCH, 2002.

The term "carrier" applied to pharmaceutical compositions/ combinations of the invention refers to a diluent, excipient, or vehicle with which an active compound is provided. A "pharmaceutically acceptable carrier" means a substance, e.g., excipient, diluent, or vehicle, that is useful in preparing a pharmaceutical composition that is generally safe, non-toxic and neither biologically nor otherwise undesirable, and includes a carrier that is acceptable for veterinary use as well as human pharmaceutical use. A "pharmaceutically acceptable carrier" includes both one and more than one such carrier.

A "patient" or a "subject" is a human or non-human animal in need of medical treatment. Medical treatment can include treatment of an existing condition, such as a disease or disorder or diagnostic treatment. Optionally the patient is a human patient.

"Providing" means giving, administering, selling, distributing, transferring (for profit or not), manufacturing, compounding, or dispensing.

"Treatment" or "treating" means providing an active compound to a patient in an amount sufficient to measurably reduce any existing condition or slow existing condition progression.

A significant change is any detectable change that is statistically significant in a standard parametric test of statistical significance such as Student's T-test, where p <0.05.

The term "therapeutically effective amount" of a compound of Formulae I, II, and III, or a related formula, means an amount effective, when administered to a patient, to provide a therapeutic benefit such as an amelioration of symptoms, e.g., an amount effective to decrease the symptoms of a central nervous system disorder, and including an amount sufficient to reduce the symptoms of Parkinson's disease, restless legs syndrome, bipolar disorder, hyperprolactinemia, depression, Huntington's chorea, Alzheimer's disease, or the cravings associated with substance abuse. Thus, a therapeutically effective amount of a compound is also an amount sufficient to significantly reduce the indicia of the disease or condition being treated. A significant reduction is any detectable negative change that is statistically significant in a standard parametric test of statistical significance, such as Student's t-test, in which p < 0.05.

"Administering" means giving, providing, applying, or dispensing by any suitable route. Administration of a combination of active agents includes administration of the combination in a single formulation or unit dosage form, administration of the individual active agents of the combination concurrently but separately, or administration of the individual active agents of the combination sequentially by any suitable route. The dosage of the individual active agents of the combination may require more frequent administration of one of the active agent(s) as compared to the other active agent(s) in the combination. Therefore, to permit appropriate dosing, packaged pharmaceutical products may contain one or more dosage forms that contain the combination of active agents, and one or more dosage forms that contain one of the combination of active agents, but not the other active agent(s) of the combination.

"Treatment" or "treating" means providing an active agent (compound) to a subject in an amount effective to measurably reduce a central nervous system disorder symptom, slow progression of the central nervous system disorder, or minimize the risk of developing the central nervous system disorder symptom. Optionally, treatment of the central nervous system disorder symptom may be initiated before the subject presents symptoms of the disease.

The term "combination therapy" refers to the administration of two or more therapeutic (active) agents to treat a therapeutic condition or disorder described in the present disclosure. Such administration encompasses co-administration of these therapeutic agents in a substantially simultaneous manner, such as in a single dosage form having a fixed ratio of active ingredients or in separate dosage forms for each active ingredient. In addition, such administration also encompasses administration of each therapeutic agent in a sequential manner, either at approximately the same time or at different times. In either case, the treatment regimen will provide the beneficial effects of each therapeutic agent in the drug combination in treating the conditions or disorders described herein.

The disclosure includes deuterated compounds and salts of Formula I, II, and III. The compounds may be deuterated at any position chemically accessible for deuteration. Certain compounds are deuterated at positions indicated by a "D" in which each D is deuterium with a deuterium enrichment of at least 50%. In certain embodiments the level of deuteration (or deuterium enrichment) at 2 of the 3 D, at least, is greater than 90%. In certain embodiments the level of deuteration at each D is greater than 90%. In certain embodiments the level of deuteration (or deuterium enrichment) at 1 of the 3 D, at least, is greater than 95%. In certain embodiments the level of deuteration (or deuterium enrichment) at 2 of the 3 D, at least, is greater than 95%. In certain embodiments the level of deuteration (or deuterium enrichment) at each D is greater than 95%.

### CHEMICAL DESCRIPTION

This disclosure describes three series of novel small molecule D3 DAR-selective negative allosteric modulators. Lead analogs in these series exhibit improved D3 DAR potency while maintaining a lack of D2 DAR antagonism. In a panel of GPCR binding assays and a functional screen of 168 different GPCRs, the disclosed compounds show extreme selectivity for the D3 DAR. Furthermore, the series of molecules showed promising *in vitro* ADME data. These chemical scaffolds have great potential to be further developed into drug-like molecules for the treatment of CNS diseases such as substance use disorder (SUD), psychosis, and Parkinson's disease (PD).

The claimed invention provides a compound of Formula I 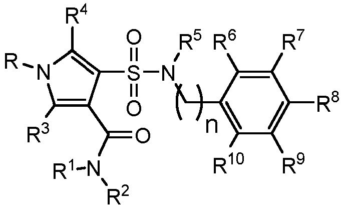 or a pharmaceutically acceptable salt thereof, wherein:
n is 0 or 1;
R is H;
R¹ is chosen from H, methyl, and ethyl, and R² is chosen from methyl and ethyl; or
R¹ and R² are joined to form a heterocycloalkyl ring optionally containing 1 additional heteroatom chosen from N, O, and S, and the heterocycloalkyl ring is unsubstituted or substituted by 1 or more substituents independently chosen from halogen, hydroxyl, amino, C₁-C₃alklyl, C₁-C₃alkoxy, C₁-C₂haloalkyl, and C₁-C₂haloalkoxy;
R³ and R⁴ are independently chosen from hydrogen, methyl, ethyl, and trifluoromethyl;
R⁵ is chosen from H, methyl, and ethyl; and
R⁶, R⁷, R⁸, R⁹, and R¹⁰ are independently chosen from hydrogen, halogen, hydroxyl, amino, -SF₅, C₁-C₄alkyl, C₁-C₄alkoxy, C₃-C₆cycloalkyl, C₁-C₄alkylester, C₁-C₂haloalkyl, and C₁-C₂haloalkoxy; wherein at least of R⁷, R⁸, and R⁹ is not hydrogen. Described, but not according to the claimed invention, is Formula I above wherein R is methyl or ethyl.

Formula I includes embodiments in which the variables, e.g. R¹, R², R³, R⁴, R⁵, R⁶, R⁷, R⁸, R⁹, R¹⁰, and n, carry the definitions set forth below. The variable definitions can be combined in any combination that results in a stable compound.
(a) R¹ and R² are both methyl.
(b) R¹ is methyl and R² is ethyl, or R¹ is hydrogen and R² is ethyl.
(c) R¹ and R² are joined to form a heterocycloalkyl ring selected from an azetidinyl, pyrrolidinyl, piperidinyl, or piperazinyl ring and the heterocycloalkyl ring is unsubstituted or substituted by 1 or more substituents independently chosen from halogen, hydroxyl, amino, C₁-C₃alkyl, C₁-C₃alkoxy, C₁-C₂haloalkyl, and C₁-C₂haloalkoxy.
(d) R¹ and R² are joined to form a heterocycloalkyl ring selected from an azetidinyl, pyrrolidinyl, piperidinyl, or piperazinyl ring is unsubstituted or substituted with one or two halogen or methyl substituents.
(e) R³ and R⁴ are both methyl.
(g) R⁵ is hydrogen.
(h) n is 1 or n is (0).
(i) R⁶, R⁹, and R¹⁰ are all hydrogen.
(j) R⁷ and R⁸ are independently chosen from hydrogen, fluoro, chloro, bromo, methyl, ethyl, and trifluoromethyl, where at least one of R⁷ and R⁸ is not hydrogen.

In a preferred embodiment, Formula I has the structure of Formula I-A

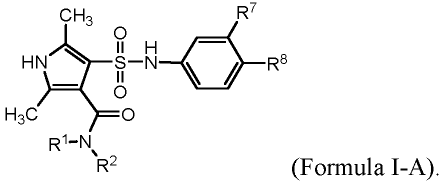

In certain embodiments the variables shown in Formula I-A carry the following definitions:
R¹ and R² are both methyl, or R¹ is hydrogen and R² is ethyl, or R¹ is methyl and R² is ethyl; or
R¹ and R² are joined to form a heterocycloalkyl ring selected from an azetidinyl, pyrrolidinyl, piperidinyl, or piperazinyl ring and the heterocycloalkyl ring is unsubstituted or substituted by 1 or more substituents independently chosen from halogen, hydroxyl, amino, C₁-C₃alkyl, C₁-C₃alkoxy, C₁-C₂haloalkyl, and C₁-C₂haloalkoxy (or preferably one or more methyl or fluoro substituents); and
R⁷ and R⁸ are independently chosen from hydrogen, fluoro, chloro, bromo, methyl, ethyl, cyclopropyl, SF₅ and trifluoromethyl, where at least one of R⁷ and R⁸ is not hydrogen.

The disclosure includes the following compounds of Formula I shown in TABLE 1, and their pharmaceutically acceptable salts, noting that compounds 22 and 31 are not according to the claimed invention.

In a preferred embodiment, Formula I has the structure of Formula I-B 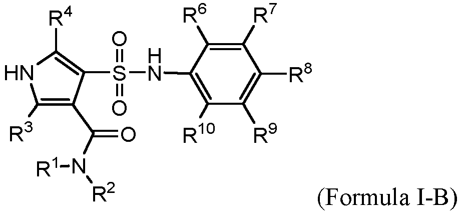 and the pharmaceutically acceptable salts thereof.

Within Formula I-B the variables can have the following values:
R¹ is methyl and R² is ethyl, or R¹ is hydrogen and R² is ethyl, or R¹ is methyl and R² is ethyl; or R¹ and R² are joined to form a heterocycloalkyl ring selected from an azetidinyl, pyrrolidinyl, piperidinyl, or piperazinyl ring, which ring is unsubstituted or substituted with or more methyl or fluoro substituents; and
R³ and R⁴ are both methyl, or one of R³ and R⁴ is hydrogen and the other is trifluoromethyl, or oner of R³ and R⁴ is methyl and the other is trifluoromethyl; and R⁶, R⁷, R⁸, R⁹, and R¹⁰ are independently chosen from hydrogen, halogen, C₁-C₂alkyl, C₁-C₂alkoxy, cyclopropyl, SF₅, and CF₃, wherein at least one of R⁶, R⁷, R⁸, R⁹, and R¹⁰ is not hydrogen and no more than 4 of R⁶, R⁷, R⁸, R⁹, and R¹⁰ are non-hydrogen.

| TABLE 1 | | | |
|---|---|---|---|
| Cmp. No. | Structure | Cmp. No. | Structure |
| 1 | 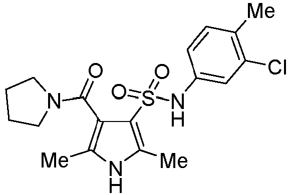 | 22 | 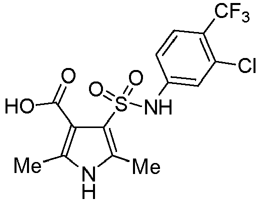 |
| 2 | 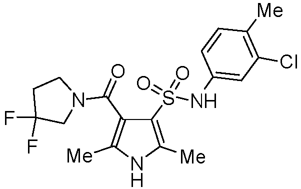 | 23 | 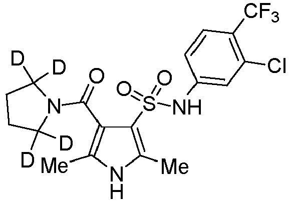 |
| 3 | 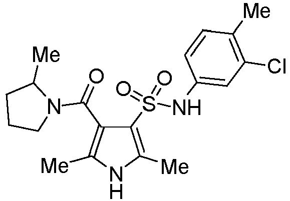 | 24 | 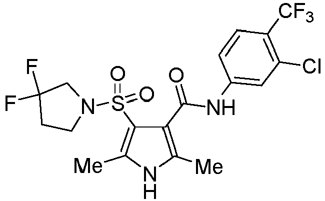 |
| 4 | 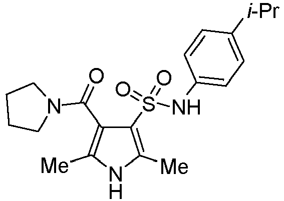 | 27 | 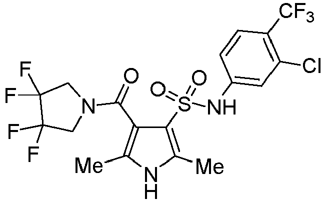 |
| 5 | 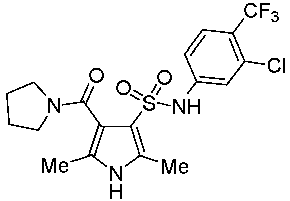 | 28 | 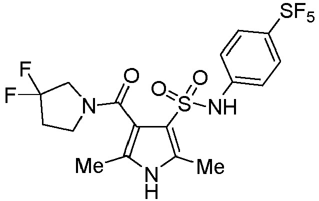 |
| 6 | 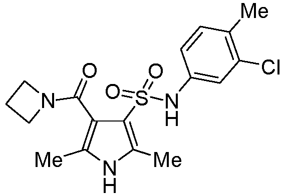 | 29 | 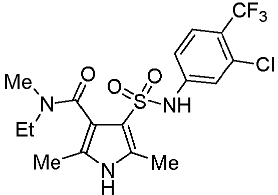 |
| 7 | 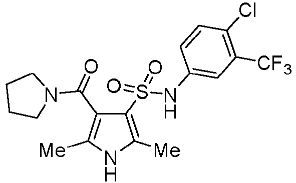 | 30 | 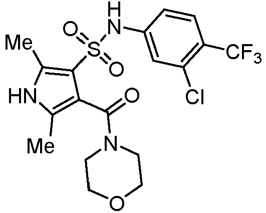 |
| 8 | 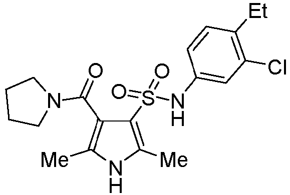 | 31 | 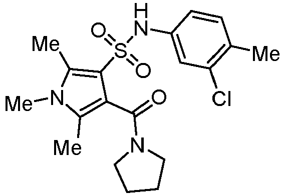 |
| 9 | 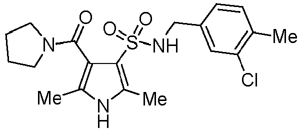 | 32 | 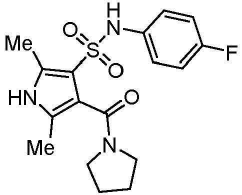 |
| 10 | 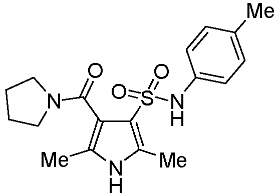 | 33 | 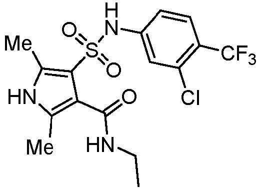 |
| 11 | 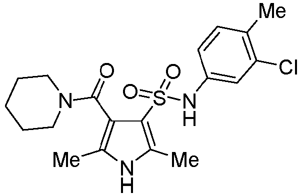 | 34 | 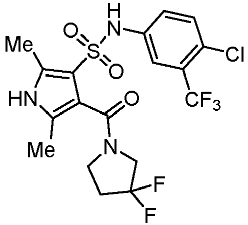 |
| 12 | 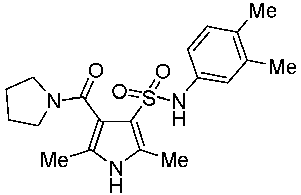 | 35 | 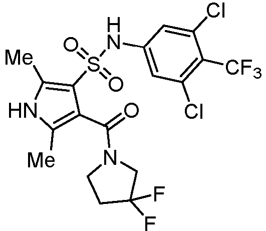 |
| 13 | 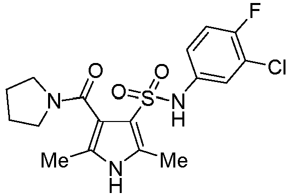 | 36 | 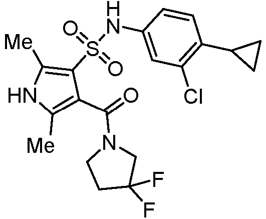 |
| 14 | 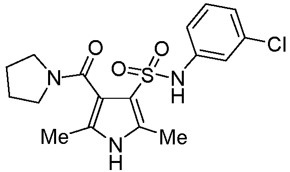 | 37 | 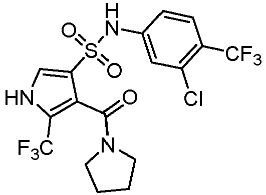 |
| 15 | 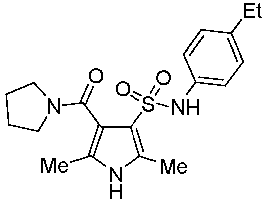 | 38 | 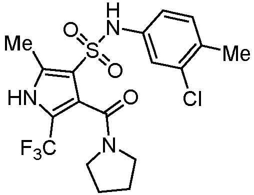 |
| 16 | 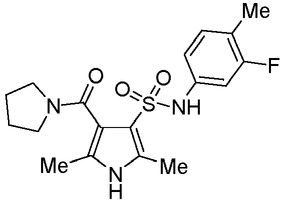 | 39 | 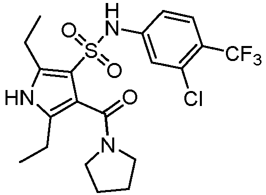 |
| 17 | 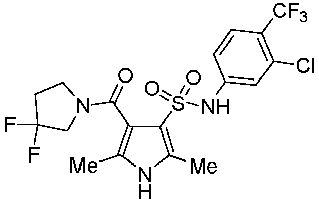 | 40 | 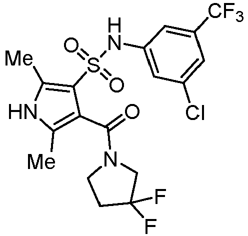 |
| 18 | 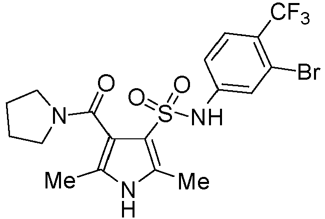 | 41 | 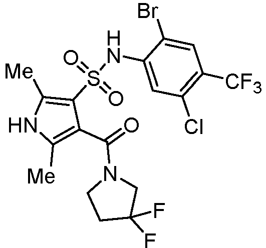 |
| 19 | 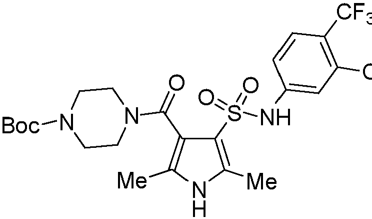 | 42 | 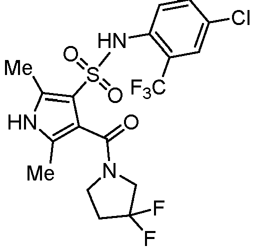 |
| 20 | 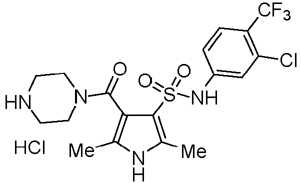 | 43 | 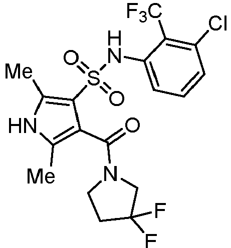 |
| 21 | 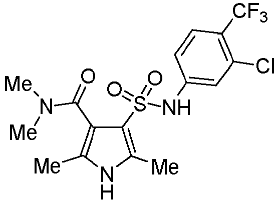 | | |

Described, but not according to the claimed invention, are compounds of Formula II, which comprises Formula II-A and II-B, 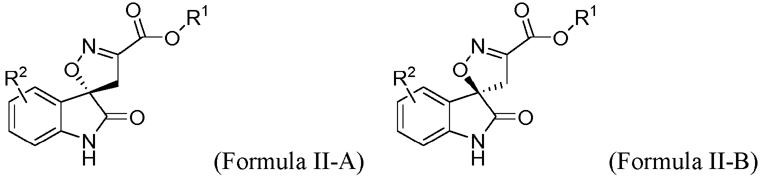 or a pharmaceutically acceptable salt thereof, wherein
R¹ is C₁-C₄alkyl;
R² is 1 or more optional substituents independently chosen from halogen, hydroxyl, amino, cyano, or C₁-C₆alkyl, in which any one CH₂ in the C₁-C₆alkyl is optionally replaced by NR³, O, S, or SO₂ and which C₁-C₆alkyl is optionally substituted by one or more substituents independently chosen from halogen, hydroxyl, amino, oxo, and cyano; and
R³ is hydrogen or C₁-C₄alkyl.

Optionally, the compound of Formula II is Compound 25, or a pharmaceutically acceptable salt thereof:

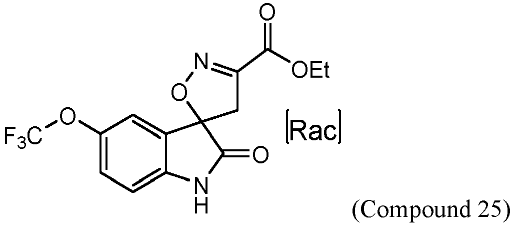

Optionally, the compound of Formula II is Compound 44, or a pharmaceutically acceptable salt thereof.

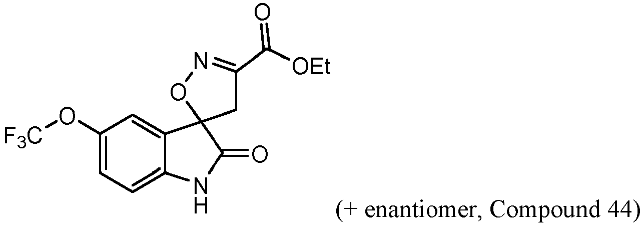

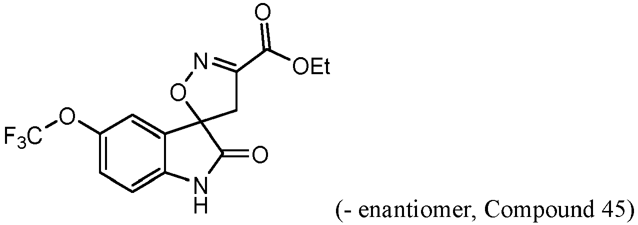

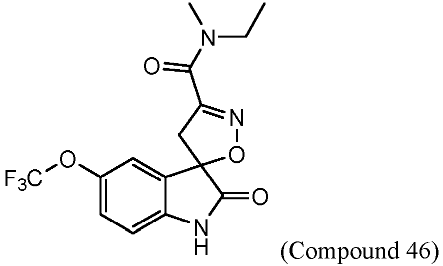

Described, but not according to the claimed invention, are compounds of Formula III, 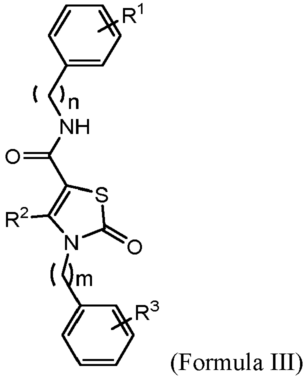 or a pharmaceutically acceptable salt thereof, wherein
n is 0, 1, or 2;
m is 0, 1, or 2;
R¹ and R³ are each 1 or more optional substituents independently chosen from halogen, hydroxyl, amino, cyano, or C₁-C₆alkyl, in which any one CH₂ in the C₁-C₆alkyl is optionally replaced by NR⁴, O, S, or SO₂ and which C₁-C₆alkyl is optionally substituted by one or more substituents independently chosen from halogen, hydroxyl, amino, oxo, and cyano; and
R² and R⁴ are each independently hydrogen or C₁-C₄alkyl.

Optionally, the compound of Formula III is Compound 26:
Compound 26, 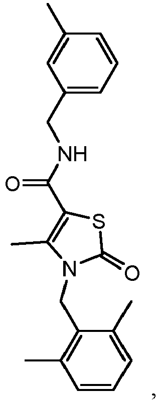 or a pharmaceutically acceptable salt thereof.

Compounds were identified that showed antagonist activity a high-throughput D3 DAR β-arrestin recruitment assay. These hit compounds were then triaged by counter screening against the closely related D2 DAR to identify compounds that were active at the D3 DAR but not at the D2 DAR. From the resulting pool of D3 DAR-selective antagonists, putative negative allosteric modulator (NAM) ligands were identified by conducting radioligand competition binding assays with [³H]-methylspiperone, an antagonist compound known to interact with the D3 DAR orthosteric binding pocket. Any compound that did not inhibit the binding of the orthosteric [³H]-methylspiperone ligand was assumed to act by binding to an ectopic allosteric site on the D3 DAR.

Three separate scaffolds are identified, Compound 1 (Cmp. 1, a Formula I compound), Compound 25 (Cmp. 25, a Formula II compound), and Compound 26 (Cmp. 26, a Formula III compound) that exhibited a D3 DAR NAM chemotype (Compounds 25 and 26 not according to the claimed invention). Cmp. 1 inhibits dopamine-stimulated β-arrestin recruitment to the D3 DAR with a potency of ~16 µM as assessed using the DiscoverX β-arrestin recruitment assay while displaying no activity at other dopamine receptor subtypes **(****FIGURE 1). FIG. 1A** shows that Cmp. 1 is fully selective for the D3 DAR vs. other DARs as assessed in the DiscoverX arrestin assay with an IC₅₀ of 16 µM ± 2.5 µM, average curves are shown (mean ± SEM from at least 3 experiments performed in triplicate). **FIG. 1B** shows Cmp. 1 lacks ability to inhibit the binding of the orthosteric antagonist [³H]-methylspiperone to the D3R, indicating it does not bind to the orthosteric site where dopamine binds. Homogenates from cells expressing the D3 DAR were incubated with indicated concentrations of Cmp. 1 or DMSO vehicle and [³H]-methylspiperone. Non-specific binding was determined in the presence of 4 µM (+)-butaclamol. These results indicate that the Cmp. 1 does not bind to the conserved orthosteric binding site on the D3 DAR, supporting an allosteric mechanism of action. Cmp. 25 and Cmp. 26 were found to inhibit dopamine-stimulated β-arrestin recruitment to the D3 DAR with potencies of ~13 µM and ~30 µM, respectively, while displaying no activity at the D2R **(****FIGURE 2****).** Importantly, neither compound exhibited measurable ability to inhibit the binding of the orthosteric antagonist [³H]-methylspiperone to the D3 DAR when using single high 40 µM concentrations of each compound (data not shown), which is consistent with an allosteric mechanism of action. **FIG. 2A** (left) shows that Cmp. 25 inhibits the D3 DAR as assessed in the DiscoverX β-arrestin assay with an IC₅₀ of ~15 µM,**FIG. 2A** (right) shows Cmp. 26 inhibits the D3 DAR as assessed in the DiscoverX beta-arrestin assay with an IC₅₀ of ~30 µM. **FIG. 2B** (left) shows Cmp. 25 lacks ability to antagonize dopamine activation of the D2 DAR as assessed in the DiscoverX β-arrestin assay, and **FIG 2B** (right) shows Cmp. 26 lacks ability to antagonize dopamine activation of the D2 DAR as assessed in the DiscoverX β-arrestin assay. Average curves from two experiments, each performed in triplicate are shown.

In **FIGURE 3****,** Cmp. 1 was further examined for its ability to antagonize various functional pathways of D3 DAR-mediated signaling including dopamine-stimulated β-arrestin recruitment (using a different assay technique than in **FIGURE 1****),** G protein (Go) activation, cAMP inhibition, and pERK stimulation, while failing to block these same signaling pathways mediated by the closely related D2 DAR. **FIG. 3A** (left) shows Cmp. 1 is a selective D3 DAR antagonist as assessed in a BRET-based β-arrestin assay (IC₅₀ = 13 µM ± 2 µM), **FIG. 3B** (left) shows Cmp. 1 is a selective D3 DAR antagonist as assessed in a BRET-based assay of Go activation (IC₅₀ = 16 µM ± 2.5 µM), **FIG. 3C** (left) shows Cmp. 1 is a selective D3 DAR antagonist as assessed in a BRET-based CAMYEL cAMP accumulation assay (IC₅₀ = 2.9 µM ± 0.7 µM), and **FIG. 3D** (left) shows Cmp. 1 is a selective D3 DAR antagonist as assessed in an antibody capture assay used to assess pERK levels (IC₅₀ = 5.6 µM ± 1.6 µM). Cmp. 1 lacked antagonist activity at the D2 DAR in all of these assays **(****FIGs 3A to 3D** right hand panels). Average curves are shown (mean ± SEM from at least 3 experiments each performed in triplicate). These findings indicate that Cmp. 1 is a selective antagonist for all D3 DAR-mediated signaling pathways that have been tested to-date.

To further assess the mechanism of action of Cmp. 1, curve-shift assays were conducted using the DiscoverX β-arrestin recruitment assay while using the orthosteric competitive antagonist sulpiride as a comparator. **FIG. 4** shows cells expressing the D3 DAR that were incubated with either dopamine **(****FIGs. 4A** **and** **4B****)** or the D3 DAR agonist pramipexole **(****FIGs. 4C** **and** **4D****)** in the presence or absence of increasing concentrations of either the orthosteric competitive antagonist sulpiride **(****FIGs 4A** and **4C****)** or Cmp. 1 **(****FIGs 4B** and **4D****).** Results and Schild plot analyses **(****FIGs 4A** **and** **C,** *insets)* indicate that sulpiride behaves in a competitive manner with dopamine and pramipexole (increasing concentrations of antagonist produces an apparent decrease in DA potency (increase in EC₅₀) with no effect on efficacy (Emax) while Cmp. 1 behaves in a non-competitive manner with both agonists (increasing concentrations of Cmp. 1 inhibits DA efficacy (Emax) with no effect on DA potency (EC₅₀)). Functional IC₅₀s for Cmp. 1 for decreasing the Emax of dopamine or pramipexole were 17 µM and 14 µM,respectively **(****FIGs 4C** **and** **D,** *insets show Emax decrease as a function of compound concentration).* Average curves are shown (mean ± SEM from at least 3 experiments each performed in triplicate). Increasing concentrations of sulpiride result in right-ward shifts in the agonist concentration-response curves with no effect on the maximum responses as would be expected for a competitive orthosteric antagonist **(****FIG. 4****).** In contrast, increasing concentrations of Cmp. 1 have no effect on the agonist potencies (EC₅₀) but produce a dose-dependent decrease in the maximum responses (Emax) indicative of noncompetitive antagonism **(****FIG. 4****)** as would be expected for a negative allosteric modulator.

As there is no evolutionary pressure to conserve an allosteric receptor site, compounds that interact with these sites offer the potential for greater target selectivity than compounds that bind to orthosteric sites, which are evolutionary conserved to optimally interact with endogenous ligands. The target selectivity of Cmp. 1 was assessed using both the DiscoverX gpcrMAX^{SM} GPCR functional assay panel of 168 unique GPCRs, and the Psychoactive Drug Screening Program (PDSP) binding affinity panel that contains 45 GPCRs, ion channels, and neurotransmitter transporters **(****FIGURE 5****).**

Cmp. 1 was tested as both an agonist **(****FIG. 5A****)** or an antagonist **(****FIG. 5B****)** at a single high concentration (30 µM) on 168 different GPCRs in the DiscoverX gpcrMAX^{™} β-arrestin recruitment assay panel **(****FIGs. 5A** and **5B****).** Assay results are presented as the mean percent activation of indicated GPCRs (for n = 2 replicates). For a full description of the DiscoverX gperMAX^{™}-GPCR assay panel and methods (see: http://www.DiscoverX.com). **FIG. 5C** shows that Cmp. 1 (10 µM) also showed limited off-target affinity in a radioligand binding panel. Data represent mean % inhibition (n = 4) for radioligand binding to various receptor subtypes, transporters, and ion channels. Data were provided by the National Institute of Mental Health (NIMH) Psychoactive Drug Screening Program (PDSP) (University of North Carolina, Chapel Hill, NC). For experimental details including radioligands used and associated Kd values for each individual target, refer to the PDSP website: http://pdsp.med.unc.edu/. While Cmp. 1 displayed affinity for the H1 receptor in the radioligand binding panel, no functional activity was identified in the functional panels. The 5-HT6 receptor was not present in the functional panels, however, subsequent testing found Cmp. 1 displays negligible, if any, antagonism of the 5-HT6 receptor (IC₅₀ > 50 µM) as assessed in a cAMP recruitment assay.

Notably, Cmp. 1 lacked agonist activity at all 168 GPCRs in the functional panel (FIG. 5A), and the only GPCR that was antagonized was the D3 DAR (FIG. 5B), thus describing the most selective D3 DAR antagonist ever identified. In the PDSP binding panel, Cmp. 1 exhibited minimal displacement at only the H1 histamine and 5-HT6 serotonin receptors. Notably, no functional activity at the H1 receptor was identified in the gpcrMAX^{SM} panel suggesting that Cmp. 1 may be negatively cooperative with the H1 histamine receptor radioligand used in the PDSP screen, but not with histamine itself. The 5-HT6 receptor was not represented in the gpcrMAX^{SM} panel, but subsequent functional assays indicated negligible, if any (IC₅₀ >50 µM) antagonism of this receptor. Taken together, these panels highlight the unprecedented selectivity of this compound for antagonizing the D3 DAR.

Analogs of Cmp. 1 have been developed that exhibit improved characteristics, primarily through increasing the scaffold's potency for the D3 DAR. Preliminary data are included in **Table 1.**

Further testing of a select group of more potent analogs in curve-shift assays show that these analogs also behave in a noncompetitive manner with agonists at the D3 DAR **(****FIGURE 6****)** and display up to 10-fold greater potency for antagonizing D3 DAR signaling activity when compared to the parent Cmp.1. **FIGURE 6A** - **C** panels show concentration-response curves of dopamine in the absence or presence of increasing concentrations of compound. Cmp. 1 analogs behave in a non-competitive manner as would be expected for a negative allosteric modulator (*inset shows Emax decrease as a function of compound concentration).* Functional IC₅₀s were calculated from concentration-response curves (insets) and found to be 3.6 µM,2.3 µM,and 1.7 µM for Cmp. 18 **(****FIG. 6A****),** Cmp. 5 **(****FIG. 6B****),** and Cmp. 17 **(****FIG. 6C****),** respectively. Average curves are shown (mean ± SEM from at least 3 experiments, each performed in triplicate).

**FIGURE 7A** shows that cmp 17 is brain penetrant in a pharmacokinetic study conducted in male CD1 mice using a single 40 mg/kg i.p. injection. **FIGURE 7B** displays the calculated pharmacokinetic parameters for cmp 17, indicating that it achieves a sufficient concentration in the brain to antagonize the D3 DAR in vivo and has a half-life of approximately 4 hours.

### PHARMACEUTICAL PREPARATIONS

Compounds disclosed herein can be administered as the neat chemical, but are preferably administered as a pharmaceutical composition. Accordingly, the disclosure describes pharmaceutical compositions comprising a compound or pharmaceutically acceptable salt of Formulae I, II, or III, together with at least one pharmaceutically acceptable carrier. In the claimed invention, the pharmaceutical composition comprises a compound of Formula I of the claimed invention, as detailed above. The pharmaceutical composition/ combination may contain a compound or salt of Formulae I, II, or III as the only active agent or may be combined with one or more additional active agents. In certain embodiments the pharmaceutical composition is in a dosage form that contains from about 0.1 milligrams (mg) to about 2000 mg, from about 10 mg to about 1000 mg, from about 100 mg to about 800 mg, or from about 200 mg to about 600 mg of a compound of Formulae I, II, or III.

Compounds disclosed herein may be administered orally, topically, parenterally, by inhalation or spray, sublingually, transdermally, via buccal administration, or by other means routine in the art for administering pharmaceutical compositions. The pharmaceutical composition may be formulated as any pharmaceutically useful form, e.g., as an aerosol, a cream, a gel, a pill, a capsule, a tablet, a syrup, a transdermal patch, or an ophthalmic solution. Some dosage forms, such as tablets and capsules, are subdivided into suitably sized unit doses containing appropriate quantities of the active components, e.g., an effective amount to achieve the desired purpose.

Carriers include excipients and diluents and must be of sufficiently high purity and sufficiently low toxicity to render them suitable for administration to the patient being treated. The carrier can be inert or it can possess pharmaceutical benefits of its own. The amount of carrier employed in conjunction with the compound is sufficient to provide a practical quantity of material for administration per unit dose of the compound.

Classes of carriers include, but are not limited to binders, buffering agents, coloring agents, diluents, disintegrants, emulsifiers, flavorants, glidants, lubricants, preservatives, stabilizers, surfactants, tableting agents, and wetting agents. Some carriers may be listed in more than one class, for example vegetable oil may be used as a lubricant in some formulations and a diluent in others. Exemplary pharmaceutically acceptable carriers include sugars, starches, celluloses, powdered tragacanth, malt, gelatin; talc, and vegetable oils. Optional active agents may be included in a pharmaceutical composition, which do not substantially interfere with the activity of the compound of the present invention.

The pharmaceutical compositions/combinations can be formulated for oral administration. These compositions contain between 0.1 and 99 weight % (wt.%) of a compound of Formulae I, II, or III and usually at least about 5 wt.% of a compound of Formulae I, II, or III. Some embodiments contain from about 25 wt.% to about 50 wt. % or from about 5 wt.% to about 75 wt.% of the compound of Formulae I, II, or III.

### METHODS OF TREATMENT

Any reference herein to a method of treatment is to be interpreted as a reference to the compound etc for use in a method of such treatment. In addition, please note that only compounds according to the claimed invention (as detailed above) for use in such a method is according to this aspect of the claimed invention.

The disclosure provides methods of treating central nervous system disorders, including Parkinson's disease and related syndromes, dyskinesia, especially dyskinesias secondary to treating Parkinson's disease with L-DOPA, neurodegenerative disorders such as Alzheimer's disease and dementia, Huntington's disease, restless legs syndrome, bipolar disorder, psychosis, and depression, schizophrenia, cognitive dysfunction, and substance use disorder (SUD) and/or withdrawal syndrome. In particular, the disclosure describes methods of treating depressive disorder, bipolar disorders, anxiety disorders, obsessive-compulsive disorders, substance use disorders and/or withdrawal syndrome, trauma- and stressor-related disorder, and disruptive, impulsive-control, and conduct disorder comprising administering an effective amount of a compound of Formulae I, II, or III to a patient having one of these disorders.

A compound of Formulae I, II, or III may be the only active agent administered (monotherapy) or may be combined with one or more other active agents (combination, adjunct, or augmentation therapy).

In another embodiment the invention provides a method of treating substance use disorder comprising (i) diagnosing a patient as having any substance use disorder and (ii) providing an effective amount of compound of Formulae I, II, or III to the patient, wherein the compound of Formulae I, II, or III is provided as the only active agent or is provided together with one or more additional active agents.

Psychosocial intervention may play an important role in treatment of any central nervous system disorder. Psychosocial intervention includes cognitive-behavior therapy, dialectical-behavior therapy, interpersonal therapy, psychodynamic therapy, and group therapy. In another embodiment, the disclosure provides a method of treating a central nervous system disorder in a patient including administration of an effective amount of a compound of Formulae I, II, or III to the patient, the method further including providing psychosocial intervention to the patient.

In another embodiment the disclosure provides a method for lessening the symptoms of depressive disorders, bipolar disorders, psychosis, anxiety disorders, obsessive-compulsive disorders, substance use disorders (SUDs) and/or withdrawal syndrome, trauma- and stressor-related disorders, and disruptive, impulsive-control, and conduct disorders comprising providing an effective amount of compound of Formulae I, II, or III to the patient. In another embodiment the disclosure provides a method for lessening the symptoms of depressive disorders, bipolar disorders, anxiety disorders, obsessive-compulsive disorders, substance use disorders and/or withdrawal syndrome, trauma- and stressor-related disorders, and disruptive, impulsive control, and conduct disorders comprising providing an effective amount of compound of Formulae I, II, or III to the patient and a therapeutically effective quantity of one or more additional active agents to lessen the symptoms of depressive disorders, bipolar disorders, anxiety disorders, obsessive-compulsive disorders, substance use disorders and/or withdrawal syndrome, trauma- and stressor-related disorders, and disruptive, impulsive-control, and conduct disorders.

Further included are methods of treating the SUDs by providing a compound of Formula I, Formula II, or Formula III to a patient wherein the SUD is abuse and dependence, as they relate to substances such as tobacco (nicotine), alcohol, caffeine, opioids, cannabis, stimulants (including amphetamine-type substances, cocaine, and other stimulants), sedatives, inhalants, hypnotics or anxiolytics, and hallucinogen (phencyclidine or similarly acting arylcyclohexylamines, and other hallucinogens, such as LSD).

Methods of treatment include providing certain dosage amounts of a compound of Formula I, Formula II, or Formula III to a patient. Dosage levels of each compound of from about 0.1 mg to about 140 mg per kilogram of body weight per day are useful in the treatment of the above-indicated conditions (about 0.5 mg to about 7 g per patient per day). The amount of compound that may be combined with the carrier materials to produce a single dosage form will vary depending upon the patient treated and the particular mode of administration. Dosage unit forms will generally contain between from about 1 mg to about 500 mg of each active compound. In certain embodiments 25 mg to 500 mg, or 25 mg to 200 mg of a compound of Formula I, Formula II, or Formula III are provided daily to a patient. Frequency of dosage may also vary depending on the compound used and the particular disease treated. However, for treatment of most diseases and disorders, a dosage regimen of 4 times daily or less can be used and in certain embodiments a dosage regimen of 1 or 2 times daily is used.

In an embodiment, a method of treating central nervous system disorders, comprises Parkinson's disease and related syndromes, dyskinesia, neurodegenerative disorders, schizophrenia, psychosis, cognitive dysfunction, or substance use disorder and/or withdrawal syndrome in a patient, comprising a step of providing to a patient in need thereof a therapeutic agent, wherein the therapeutic agent is a compound or salt thereof of any one of compound of Formula I, Formula II, or Formula III.

The method of treating a central nervous system disorders, wherein the disorder is selected from the group comprising of obesity, anxiety, depression (e.g., refractory depression, MDD, or bipolar depression), psychosis (including psychosis associated with dementia such as hallucinations in progressive Parkinson's disease or paranoid ideas), schizophrenia, sleep disorders (in particular sleep disorders associated with schizophrenia and other psychiatric and neurological diseases), sexual disorders, migraines, conditions associated with headaches, social phobias, agitation in dementia (e.g., agitation in Alzheimer's disease), agitation in autism and related autistic disorders, gastrointestinal disorders such as gastrointestinal motility dysfunction, and dementia, such as dementia of Alzheimer's disease or Parkinson's disease; mood disorders; or substance use disorder and/or withdrawal syndrome, for example, opiate dependence and / or alcohol dependence or withdrawal from drug or alcohol dependence (for example, opiate dependence); or compulsive overeating.

In an embodiment, the disorder is the substance use disorder and/or the withdrawal syndrome.

In an embodiment, the substance use disorder is related to substances comprising tobacco, alcohol, caffeine, opioids, cannabis, stimulants, sedatives, inhalants, hypnotics or anxiolytics, or hallucinogen.

In an embodiment, the method further comprises administering to the patient in need thereof at least one additional therapeutic agent.

It will be understood, however, that the specific dose level for any particular patient will depend upon a variety of factors including the activity of the specific compound employed, the age, body weight, general health, sex, diet, time of administration, route of administration, and rate of excretion, drug combination and the severity of the particular disease undergoing therapy.

Methods of treatment provided herein are also useful for treatment of mammals other than humans, including for veterinary applications such as to treat horses and livestock e.g. cattle, sheep, cows, goats, swine and the like, and pets (companion animals) such as dogs and cats.

The term "room temperature" (rt or RT) used herein refers to a temperature of about 25 °C.

### EXAMPLES

Note that, herein, compounds 22, 25, 26, 31, 44, 45, and 46 are not according to the claimed invention.

### ABBREVIATIONS

- BOC or Boc: *tert*-butoxycarbonyl
- Dec: Decomposition
- DIPEA: Diisopropylethylamine
- DCE: Dichloroethane
- DCM: Dichloromethane
- DMAP: 4-(N,N-dimethylamino)pyridine
- DMF: Dimethylformamide
- DMSO: Dimethyl Sulfoxide
- EtOAc: Ethyl Acetate
- HATU: 2-(3H-[1,2,3]triazolo[4,5-b]pyridin-3-yl)-1,1,3,3-tetramethylisouronium hexafluorophosphate (V)
- Mp: Melting point
- NMR: Nuclear Magnetic Resonance
- PyBOP: Bzotriazol-1-yloxytripyrrolidinophosphonium hexafluorophosphate
- rt or RT: Room temperature
- THF: Tetrahydrofuran
- TLC: Thin layer chromatography

### GENERAL METHODS

All air- or moisture-sensitive reactions were performed under positive pressure of argon with oven-dried glassware. All reagents were used as received from the following suppliers: Alfa Aesar, Ark Pharm, Aldrich, and Fisher Scientific. The ¹H and ¹³C NMR spectra were recorded on either a Bruker Avance 400 MHz or 500 MHz spectrometer or Varian MR 400 Megahertz (MHz) spectrometer. Chemical shifts are reported in parts per million and were referenced to residual proton solvent signals. When indicated, ¹³C multiplicities were determined with the aid of an APT (Attached Proton Test) pulse sequence, differentiating the signals for methyl and methane carbons as "d" from methylene and quaternary carbons as "u".

The infrared (IR) spectra were acquired as thin on a PerkinElmer Spectrum One FT-IR spectrometer equipped with a universal Attenuated total reflection (ATR) sampling accessory and the absorption frequencies are reported in cm⁻¹. Flash column chromatography separations were performed using the Teledyne Isco CombiFlash *R_{F}* using RediSep *R_{F}* silica gel columns. TLC was performed on Analtech UNIPLATE silica gel GHLF plates (gypsum inorganic hard layer with fluorescence). TLC plates were visualized under a long wave/short wave ultraviolet (UV) lamp.

Compound purity was measured on the basis of peak integration (area under the curve) from UV/Vis absorbance (at 214 nm), and compound identity was determined on the basis of mass analysis. All compounds used for biological studies have purity >90%.

### SYNTHESIS OF SELECTED COMPOUNDS

### General Synthetic Scheme for the synthesis of pyrrole D3 NAM Analogues

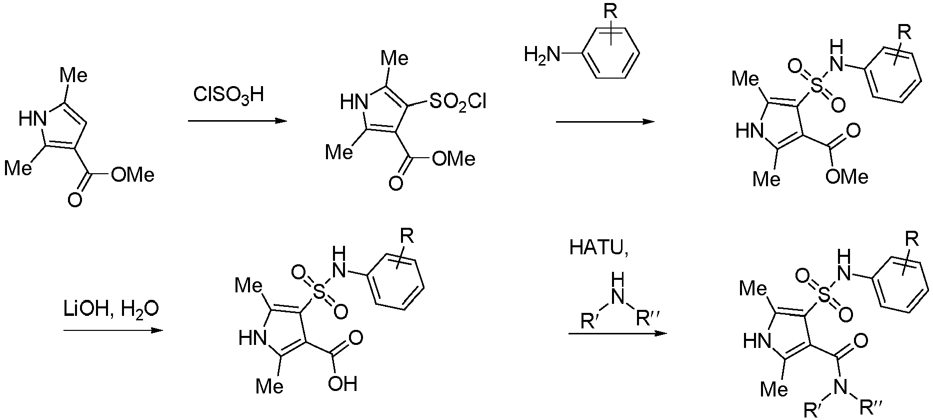

Synthetic Routes for the Synthesis of Compounds 22, 21, 5, 17, 30, 19, 20, 28, 31, 33, 34, 35, 36, 39, 40, 41, 42 and 43

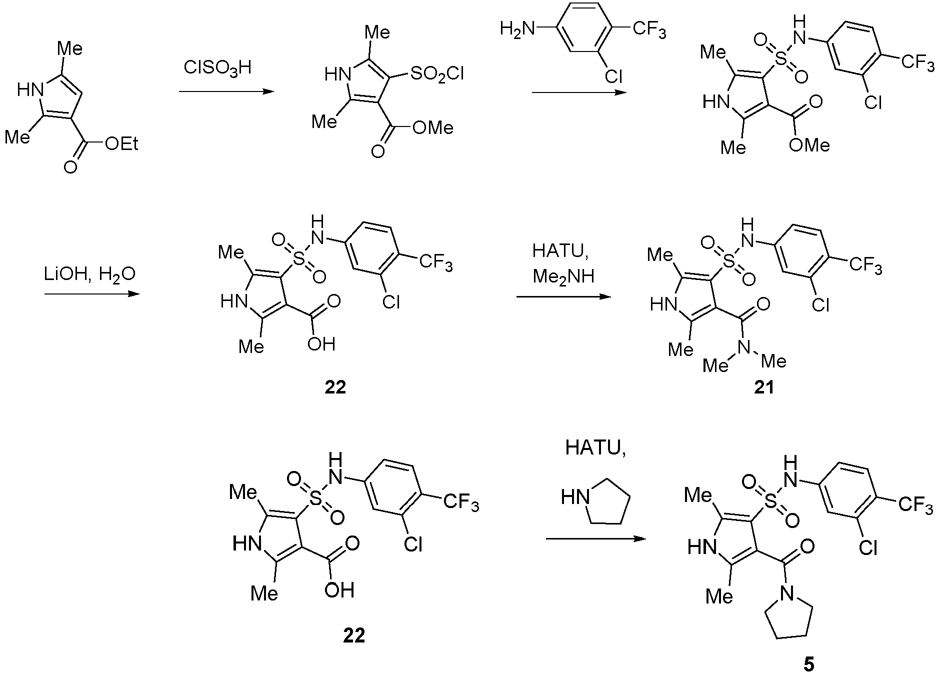

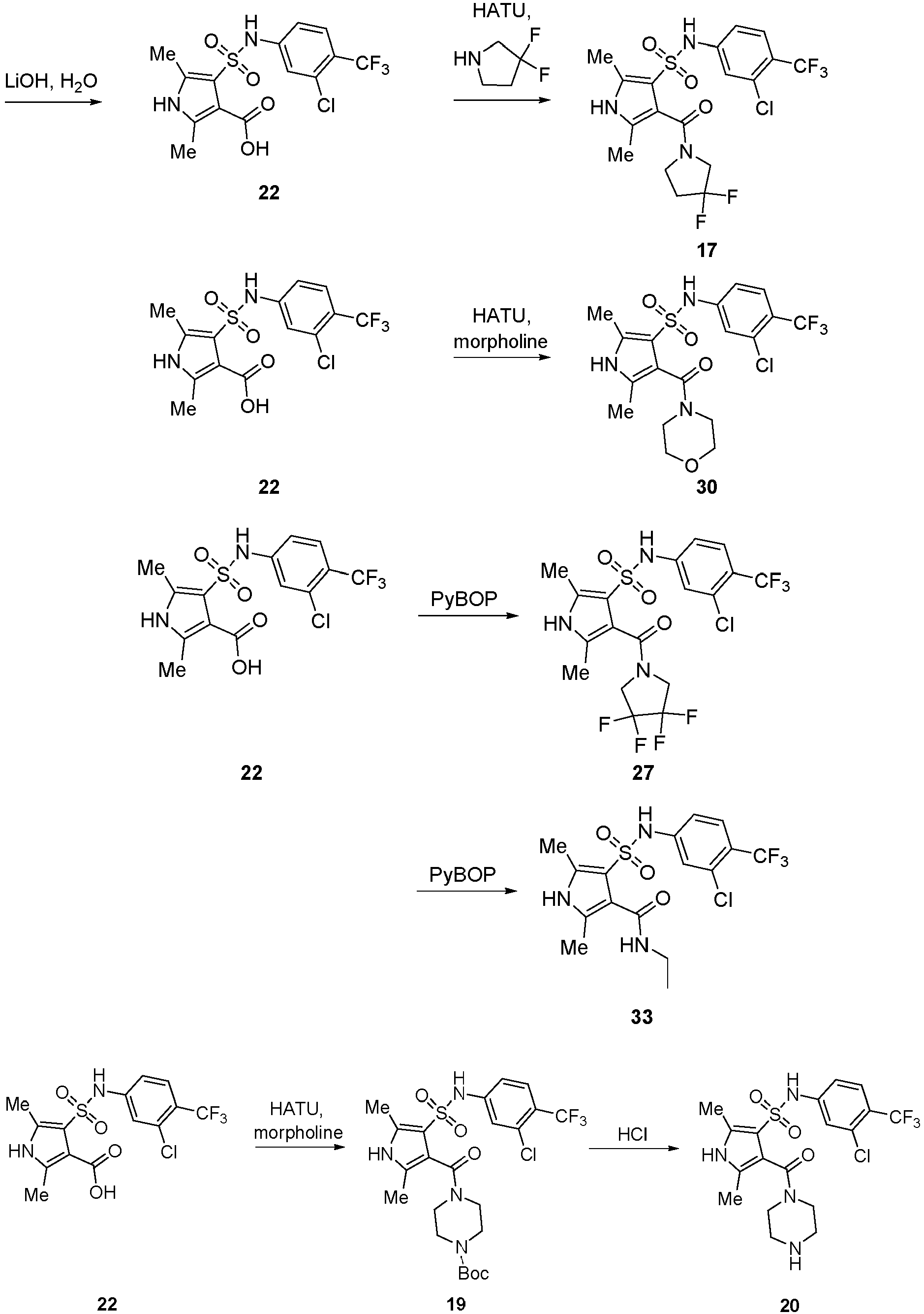

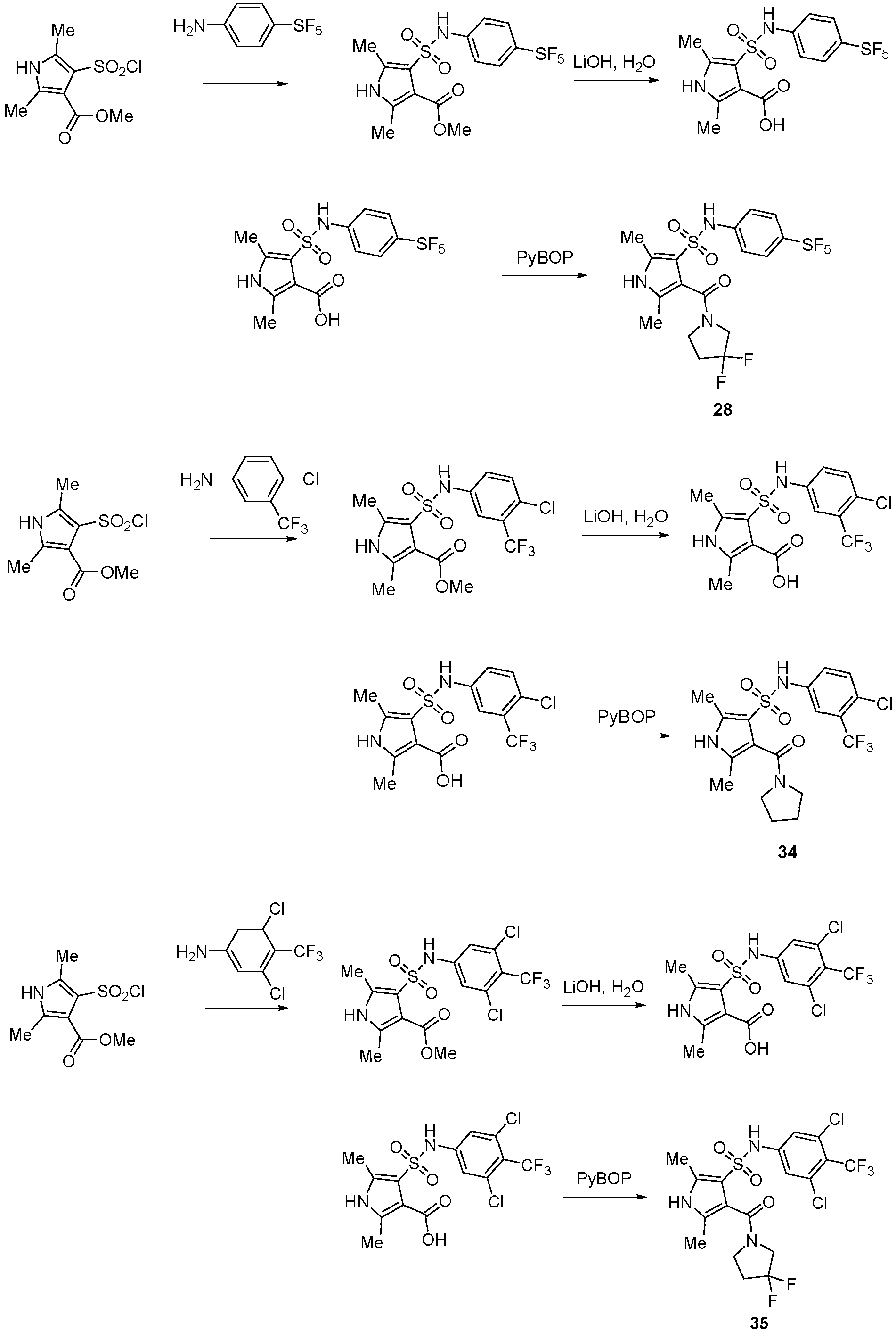

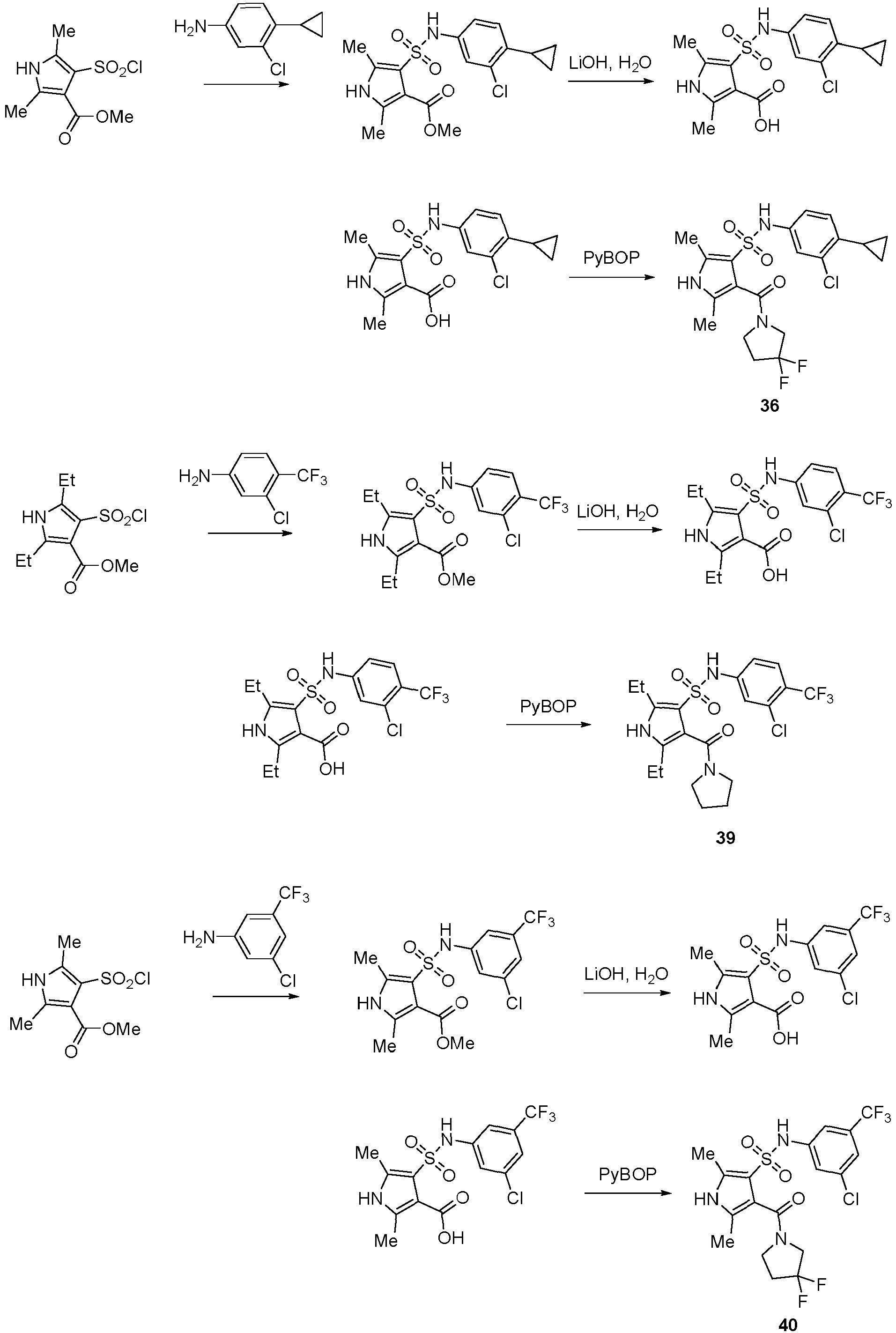

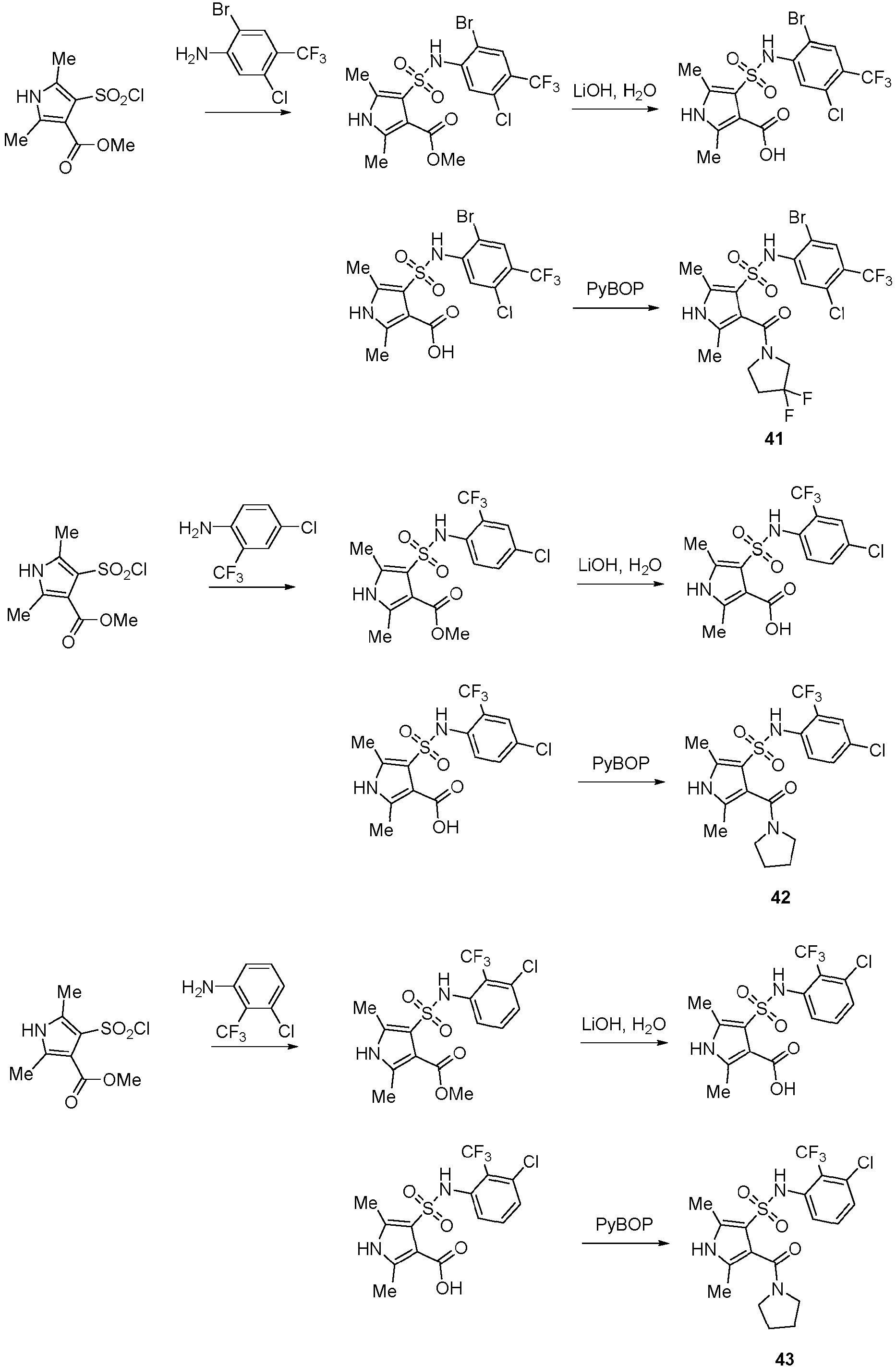

### EXAMPLE 1. SYNTHESIS OF METHYL 4-(N-(3-CHL ORO-4-(TRIFLUOROMETHYL)PHENYL)SULFAMOYL)-2,5-DIMETHYL-1H-PYRROLE-3-CARBOXYLATE

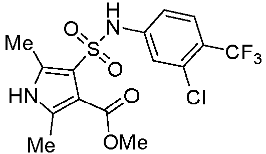

Chlorosulfonic acid (4.090 grams (g), 2.333 milliliters (ml), 35.10 millimoles (mmol), 10 equivalents (equiv.)) was added in small portions to methyl 2,5-dimethyl-14-pyrrole-3-carboxylate (537.7 milligrams (mg), 3.510 mmol, 1 equiv.) at 0 °C under vigorous stirring. After 2.5 hours (h), the reaction was warmed to rt over 15 mins then heated at 60 °C for 14 h. After cooling again to 0 °C, the reaction was quenched by pouring into ice. The solid was filtered and washed with water to afford the crude product as a purple-brown solid, which was dried via toluene azeotrope (four times) and used without further purification (crude yield = 282.8 mg). The above sulfonyl chloride was added to a solution of 4-chloro-3-(trifluoromethyl)aniline (343.2 mg, 1.755 mmol, 0.5 equiv.) in pyridine (6 ml) and stirred at rt overnight. The solvent removed and the residue purified by flash chromatography (hexanes/EtOAc, 0 to 100%) to afford the sulfonamide product as an off-white solid (350.6 mg, 3.510 mmol, 24% yield over two steps). *R_{f}*= 0.48 (50% EtOAc/hexanes); mp = 187-198 °C.

¹H NMR (400 MHz, acetone-d6) δ 2.34 (s, 3H), 2.37 (s, 3H), 3.84 (s, 3H), 7.42 (dd, *J=* 2.7, 8.7 Hz, 1H), 7.51 (d, *J =* 8.7 Hz, 1H), 7.68 (d, *J =* 2.6 Hz, 1H), 8.86 (br s, 1H), 10.74 (br s, 1H); ¹³C NMR (101 MHz, acetone-d6, APT pulse sequence) δ d (CH, CH₃) 11.7, 12.6, 51.0, 118.7 (q, *J =* 5.7 Hz), 124.8, 132.3; u (C, CH₂) 109.1, 116.0, 122.8 (q, *J =* 273.9 Hz), 125.9 (q, *J =* 1.9 Hz), 127.9 (q, *J =* 60.0 Hz), 134.9, 136.0, 138.5, 164.8.

### EXAMPLE 2. SYNTHESIS OF 4-(N-(3-CHLORO-4-(TRIFLUOROMETHYL)PHENYL)SULFAMOYL)-2,5-DIMETHYL-1H-PYRROLE-3-CARBOXYLIC ACID (COMPOUND 22)

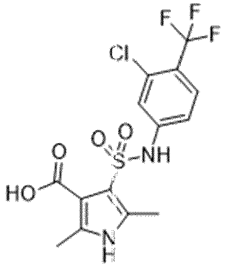

Methyl 4-(*N*-(3-chloro-4-(trifluoromethyl)phenyl)sulfamoyl)-2,5-dimethyl-1*H*-pyrrole-3-carboxylate (400 mg, 0.974 mmol) was dissolved in MeOH (1.5 ml) and THF (4.5 ml). A solution of LiOH (233 mg, 9.74 mmol) in water (1.5 ml) was added and the reaction was heated at 55 °C for 5 h. After cooling to rt, the reaction was acidified with 1 normal (N) aqueous HCl, precipitating the carboxylic acid product. The crude product was collected by filtration, washed with water, dried under vacuum and purified by C-18 flash chromatography to afford Compound 22 as an off-white solid (420 mg, 1.06 mmol, 68% yield).

¹H NMR (400 MHz, DMSO-d6) δ 2.26 (s, 3H), 2.36 (s, 3H), 6.95-7.16 (m, 1H), 7.23 (d, *J= 2.1* Hz, 1H), 7.60 (d, *J =* 8.7 Hz, 1H), 11.52 (s, 1H). ¹³C NMR (101 MHz, DMSO-*d6*) δ 12.8, 13.5, 110.9, 117.1, 117.2, 118.8 (q, *J =* 31.2 Hz), 120.5, 123.7 (q, *J =* 272.8 Hz), 129.0 (q, *J =* 5.3 Hz), 131.4 (q, *J =* 1.9 Hz), 132.7, 135.3, 147.3 (q, *J =* 3.5 Hz), 165.6; ¹⁹F NMR (376 MHz, DMSO-d6) δ -59.77.

### EXAMPLE 3. SYNTHESIS OF 4-(N-(3-CHLORO-4-(TRIFLUOROMETHYL)PHENYL)SULFAMOYL)-N,N,2,5-TETRAMETHYL-1H-PYRROLE-3-CARBOXAMIDE(COMPOUND 21)

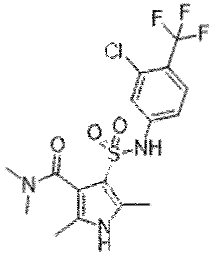

To a solution of 4-(*N*-(3-chloro-4-(trifluoromethyl)phenyl)sulfamoyl)-2,5-dimethyl-1*H*-pyrrole-3-carboxylic acid (50 mg, 0.13 mmol) in DMF (2 mL) was added HATU (58 mg, 0.15 mmol, 1.2 equiv.). After 10 mins, a solution of dimethylamine (40 wt % in THF, 17 mg, 0.15 mmol, 1.2 equiv.) was added followed by DIEA (20 mg, 0.15 mmol, 1.2 equiv). The reaction was stirred at rt for 26 h, the solvent removed and the residue purified by flash chromatography (CH₂Cl₂/MeOH) to afford the amide product as a tan solid (13 mg, 0.031 mmol, 24% yield).

¹H NMR (400 MHz, Methanol-*d₄*) δ 2.05 (s, 3H), 2.32 (s, 3H), 2.91 (s, 3H), 3.08 (s, 3H), 7.19 (dd, *J =* 1.4, 8.7 Hz, 1H), 7.34 (d, *J =* 2.2 Hz, 1H), 7.59 (d, *J =* 8.7 Hz, 1H); ¹³C NMR (100 MHz, methanol-*d₄*) δ 9.6, 10.5, 33.8, 37.8, 113.7, 114.7, 116.6, 120.7, 122.5 (q, *J =* 31.8 Hz), 123.1 (q, *J* = 272.6 Hz), 128.0 (q, *J =* 5.4 Hz), 125.1, 132.1 (q, *J* =1.9 Hz), 132.9, 143.2, 168.0.

### EXAMPLE 4. SYNTHESIS OF N-(3-CHLORO-4-(TRIFLUOROMETHYL)PHENYL)-2,5-DIMETHYL-4-(PYRROLIDINE-1-CARBONYL)-4,5-DIHYDRO-1H-PYRROLE-3-SULFONAMIDE (Compound 5)

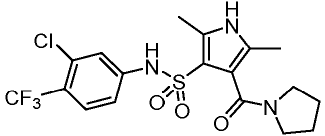

To a solution of 4-(*N*-(3-chloro-4-(trifluoromethyl)phenyl)sulfamoyl)-2,5-dimethyl-1*H*-pyrrole-3-carboxylic acid (60.8 mg, 0.153 mmol) in DMF (2 mL) was added HATU (87.4 mg, 1.5 equiv, 230 micromole (µmol)) and the reaction stirred for 10 min at rt. Pyrrolidine (21.8 mg, 0.306 mmol, 2.0 equiv) was added followed by DIEA (80.1 microliters (µL), 0.460 mmol, 3.0 equiv) and the reaction stirred at rt for 29 h. The solvent was removed and the residue purified by silica then C-18 flash chromatography (EtOAc/hexanes then water/MeOH, respectively) to afford the amide product as a white solid (28.5 mg, 0.634 mmol, 41 % yield). *R_{f}=* 0.09 (50% EtOAc/hexanes); mp = 126-141 °C.

¹H NMR (400 MHz, chloroform-d) δ 1.66 (m, 2 H), 1.74 (m, 2H), 1.90 (s, 3H), 2.03 (s, 3H), 3.13 (m, 2H), 3.39 (m, 2H), 7.03 (m, 1H), 7.27 (m, 2H), 8.07 (bs, 1H), 8.15 (bs, 1H); ¹³C NMR (101 MHz, chloroform-d) δ 11.9, 24.5, 25.7, 45.9, 48.7, 50.7, 114.6, 115.9, 118.8, 122.9 (q, *J* = 273.7 Hz), 123.1, 124.9 (q, *J =* 31.9 Hz), 125.5, 128.0 (q, *J =* 5.4 Hz), 132.7, 132.9, 142.7, 166.3; ¹⁹F NMR (376 MHz, chloroform-d) δ -61.9; IR (neat): 1606, 1576, 1454 cm⁻¹.

### EXAMPLE 5. SYNTHESIS OF N-(3-CHLORO-4-(TRIFLUOROMETHYL)PHENYL)-4-(3,3-DIFLUOROPYRROLIDINE-1-CARB ONYL)-2, 5-DIMETHYL-1H-PYRROLE-3-SULFONAMIDE

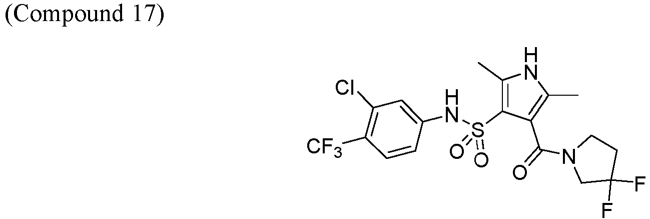

To a solution of 4-(N-(3-chloro-4-(trifluoromethyl)phenyl)sulfamoyl)-2,5-dimethyl-1H-pyrrole-3-carboxylic acid (47.1 mg, 0.119 mmol) in DMF (2 mL) was added HATU (67.7 mg, 0.178 mmol, 1.5 equiv.). After 10 min 3,3-difluoropyrrolidin-1-ium chloride (34.1 mg, 0.237 mmol, 2.0 equiv.) was added followed by DIEA (76 µL, 0.43 mmol, 3.7 equiv.). The reaction was stirred at rt for 28 h, the solvent evaporated and the residue purified by flash chromatography to afford the amide product as a white solid (21.3 mg, 0.0438 mmol, 37 % yield). Rf = 0.22 (50% EtOAc/hexanes).

¹H NMR (400 MHz, Methanol-*d₄*) δ 2.08 (s, 3H), 2.32 (s, 3H), 2.44 (s, 2H), 3.37 (t, *J =* 7.2 Hz, 2H), 3.49 (t, *J =* 12.9 Hz, 2H), 7.14 - 7.20 (m, 1H), 7.31 (d, *J =* 2.3 Hz, 1H), 7.69 (d, *J =* 8.7 Hz, 1H); ¹⁹F NMR (376 MHz, methanol-*d₄*) δ -63.13.

### EXAMPLE 6. SYNTHESIS OF N-(3-CHLORO-4-(TRIFLUOROMETHYL)PHENYL)-2,5-DIMETHYL-4-(MORPHOLINE-4-CARBONYL)-1H-PYRROLE-3-SULFONAMIDE (Compound 30)

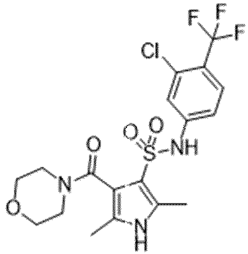

To a solution of 4-(N-(3-chloro-4-(trifluoromethyl)phenyl)sulfamoyl)-2,5-dimethyl-1H-pyrrole-3-carboxylic acid (50 mg, 0.13 mmol) in DMF (2 mL) was added HATU (58 mg, 0.15 mmol, 1.2 equiv.). After 10 mins, a solution of morpholine (13 mg, 0.15 mmol, 1.2 equiv.) in DMF (0.5 mL) was added followed by DIEA (26 µL, 0.15 mmol, 1.2 equiv.). The reaction was stirred at rt for 27 h, the solvent removed and the residue purified by C-18 flash chromatography (water/MeOH) to afford the amide product as an off-white solid (25 mg, 0.054 mmol, 42% yield).

¹H NMR (400 MHz, Chloroform-d) δ 2.50 (d, *J =* 4.0 Hz, 6H), 2.81 - 3.09 (m, 4H), 3.46 - 3.73 (m, 4H), 7.52 - 7.57 (m, 1H), 7.61 (d, *J =* 8.7 Hz, 1H), 7.94 - 8.13 (m, 1H), 8.69 (s, 1H), 9.88 (s, 1H). ¹³C NMR (100 MHz, Chloroform-d) δ 13.3, 13.6, 45.1, 65.9, 110.2, 114.1, 116.6, 121.3, 123.2 (q, *J* = 31.8 Hz), 124.3, 128.2 (q, *J =* 5.3 Hz), 133.2 (q, *J =* 1.9 Hz), 134.9, 135.6, 142.4, 162.3.

### EXAMPLE 7. SYNTHESIS OF TERT-BUTYL 4-(4-(N-(3-CHLORO-4-(TRIFLUOROMETHYL))PHENYL)SULFAMOYL)-2,5-DIMETHYL-1H-PYRROLE-3-CARBONYL)PIPERAZINE-1-CARBOXYLATE (Compound 19)

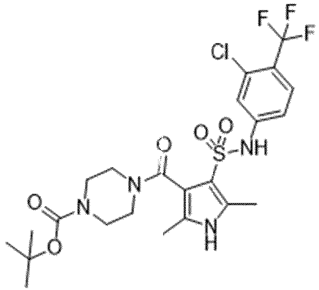

To a solution of 4-(*N*-(3-chloro-4-(trifluoromethyl)phenyl)sulfamoyl)-2,5-dimethyl-1*H*-pyrrole-3-carboxylic acid (60 mg, 0.15 mmol) in DMF (2 mL) was added HATU (69 mg, 0.18 mmol, 1.2 equiv.). After 10 mins, tert-butyl piperazine-1-carboxylate (34 mg, 0.18 mmol, 1.2 equiv.) was added followed by DIEA (40 µL, 0.18 mmol, 1.2 equiv.). The reaction was stirred at rt for 30 h, the solvent removed and the residue purified by C-18 flash chromatography (water/MeOH) to afford the amide product as a tan solid (44.1 mg, 0.078 mmol, 52% yield).

¹H NMR (400 MHz, Chloroform-*d*) δ 1.41 (s, 9H), 2.50 (s, 3H), 2.51 (s, 3H), 2.94 (t, *J =* 5.1 Hz, 4H), 3.35-3.43 (m, 4H), 7.58 (d, *J =* 1.9 Hz, 1H), 7.62 (d, *J =* 8.7 Hz, 1H), 8.03 (s, 1H), 8.84 (br s, 1H), 9.89 (br s, 1H). ¹³C NMR (100 MHz, Chloroform-d) δ 13.3, 13.6, 28.3, 45.0, 80.6, 110.4, 114.0, 116.6, 121.3, 122.9 (q, *J =* 273.4 Hz), 123.5 (q, *J* = 31.9 Hz), 128.2 (q, *J =* 5.3 Hz), 133.1, 134.8, 135.7, 142.5, 154.1, 162.2; ¹⁹F NMR (376 MHz, chloroform-d) δ -61.73.

### EXAMPLE 8. SYNTHESIS OF N-(3-CHLORO-4-(TRIFLUOROMETHYL)PHENYL)-2,5-DIMETHYL-4-(PIPERAZINE-1-CARBONYL)-1H-PYRROLE-3-SULFONAMIDE (Compound 20)

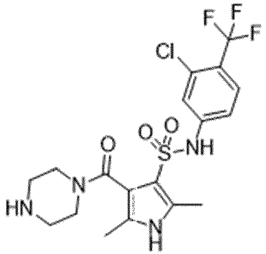

To a solution of tert-Butyl 4-(4-(*N*-(3-chloro-4-(trifluoromethyl)phenyl)sulfamoyl)-2,5-dimethyl-1*H*-pyrrole-3-carbonyl)piperazine-1-carboxylate (20 mg, 0.035 mmol) in CH₂Cl₂ (2 mL) was added concentrated hydrochloric acid (1 mL). The deprotection was stirred at rt for 3 h and neutralized with 1 N sodium hydroxide and extracted with CH₂Cl₂ (3 × 3 mL). The combined organic layers were evaporated and purified by C-18 flash chromatography (water/MeOH) to afford the amide product as an off-white solid (25 mg, 0.054 mmol, 42% yield).

¹H NMR (400 MHz, Methanol-*d₄*) δ 2.37 (s, 3H), 2.44 (s, 3H), 2.73 (t, *J = 5.0* Hz, 4H), 2.95 (t, *J =* 5.0 Hz, 4H), 7.60 (ddd, *J =* 0.9, 2.0, 8.6 Hz, 1H), 7.70 (d, *J =* 8.7 Hz, 1H), 8.09 (d, *J =* 2.0 Hz, 1H); ¹³C NMR (100 MHz, Methanol-*d₄*) δ δ 11.0, 11.5, 44.3, 45.4, 110.0, 114.2, 116.8, 121.0, 122.3 (q, *J =* 31.9 Hz), 123.0 (q, *J* = 272.7 Hz), 127.9 (q, *J =* 5.4 Hz), 132.3 (q, *J =* 1.8 Hz), 133.4, 134.9, 143.1, 164.0.

### EXAMPLE 9. SYNTHESIS OF N-(3-CHLORO-4-(TRIFLUOROMETHYL)PHENYL)-2,5-DIMETHYL-4-(3,3,4,4-TETRAFLUOROPYRROLIDINE-1-CARBONYL)-1H-PYRROLE-3-SUFLONAMIDE (Compound 27)

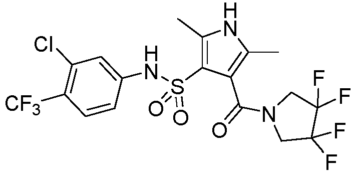

To a solution of 4-(N-(3-chloro-4-(trifluoromethyl)phenyl)sulfamoyl)-2,5-dimethyl-1H-pyrrole-3-carboxylic acid (100 mg, 0.25 mmol) in DMF (2 mL) was added PyBOP (195 mg, 0.38 mmol, 1.5 equiv.). After 10 mins, 2,2,3,3-tetrafluoropyrrolidine (72 mg, 0.50 mmol, 2.0 equiv.) was added. The reaction was stirred at rt for 30 h, the solvent removed and the residue purified by flash chromatography (EtOAc/hexanes) to afford amide as white solid (21 mg, 0.25 mmol, 16% yield).

¹H NMR (400 MHz, CDCl₃) δ 2.21 (s, 3H), 2.30 (s, 3H), 3.78-4.29 (m, 4H), 7.22 (d, *J =* 8.5 Hz, 1H) 7.43 (s, 1H), 7.53 (d, *J =* 7.8 Hz, 1H), 7.63 (s, 1H), 8.02, (s, 1H); HPLC purity = 98%.

### EXAMPLE 10. SYNTHESIS OF 4-(N-(3-CHLORO-4-(TRIFLUOROMETHYL)PHENYL)SULFAMOYL)-N-ETHYL-2,5-DIMETHYL-1H-PYRROLE-3-CARBOXAMIDE (Compound 33).

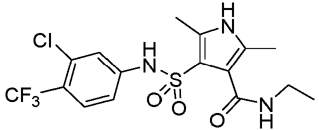

To a solution of 4-(*N*-(3-chloro-4-(trifluoromethyl)phenyl)sulfamoyl)-2,5-dimethyl-1*H*-pyrrole-3-carboxylic acid (60 mg, 0.15 mmol) in DMF (2 mL) was added PyBOP (117 mg, 0.23 mmol, 1.5 equiv.). After 10 mins, ethylamine solution (1 M in THF, 0.3 mL, 0.30 mmol, 2.0 equiv.) was added. The reaction was stirred at rt for 30 h, the solvent removed and the residue purified by flash chromatography (EtOAc/hexanes) to afford amide as white solid (54 mg, 0.13 mmol, 84% yield).

¹H NMR (400 MHz, Acetone-D₆) δ 1.21 (t, *J =* 7.2 Hz, 3H), 2.30 (s, 3H), 2.33 (s, 3H), 3.42 (m, 2H), 7.35 (d, *J =* 8.6 Hz, 1H), 7.51 (d, *J =* 1.8 Hz, 1H), 7.71 (d, *J =* 8.6 Hz, 1H), 9.73 (s, 1H), 10.44 (s, 1H); ¹⁹F NMR (376 MHz) δ -62.2; HPLC purity = 99 %. EXAMPLE 11. SYNTHESIS OF 4-(3,3-DIFLUOROPYRROLIDINE-1-CARBONYL)-2,5-DIMETHYL-N-)4-(PENTAFLUORO-16-SULFANEYL)PHENYL)1H-PYRROLE-3-SULFONAMIDE (Compound 28)

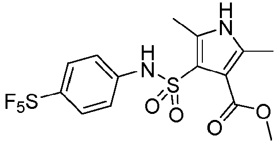

To a solution of methyl-4-(chlorosulfonyl)-2,5-dimethyl-1*H*-pyrrole-3-carboxylate (231 mg, 0.91 mmol) in DCE cooled in an ice bath was added 4-(sulfurpentafluoro)aniline (311 mg, 1.59 mmol, 1 equiv.) and the reaction was stirred for 18 h, slowly warming to rt. The solvents were removed in vacuo and the residue purified by flash chromatography (EtOAc/hexanes) to afford the sulfonamide product as a pink solid (101 mg, 0.92 mmol, 25% yield).

¹H NMR (400 MHz, CDCl₃) δ 2.39 (s, 3H), 2.40 (s, 3H), 3.89 (s, 3H), 7.23 (d, *J =* 8.5 Hz, 2H), 7.60 (d, *J =* 9.0 Hz, 2H), 8.19 (s, 1H), 8.76 (s, 1H).

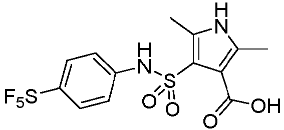

Methyl 2,5-dimethyl-4-(sulfurpentafluoro)phenyl)sulfamoyl)-1*H*-pyrrole-3-carboxylate (450 mg, 1.05 mmol) was dissolved in THF:MeOH (10 mL, 2:1) and lithium hydroxide (50.4 mg, 2.10 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford the carboxylic acid product as a white solid (127 mg, 0.3 mmol, 29% yield).

¹H NMR (400 MHz, MeO-D₄) δ 2.34 (s, 6H), 7.25 (d, *J*=8.0 Hz, 2H), 7.57 (d, *J*=9 Hz, 2H).

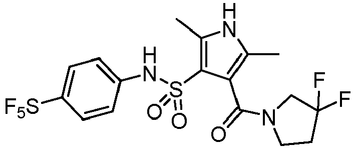

To a solution of 2,5-dimethyl-4-(*N*-(4-(sulfurpentafluoro)phenyl)sulfamoyl)-1*H*-pyrrole-3-carboxylic acid (76 mg, 0.18 mmol) in DMF (2 mL) was added PyBOP (141 mg, 0.27 mmol, 1.5 equiv.). After 10 mins, 3,3-difluoropyrrolidine (39 mg, 0.36 mmol, 2.0 equiv.) was added. The reaction was stirred at rt for 30 h, the solvent removed and the residue purified by flash chromatography (EtOAc/hexanes) to afford the amide product as a white solid (20 mg, 0.04 mmol, 20% yield).

¹H NMR (400 MHz, CDCl₃) δ 2.15 (s, 3H), 2.21 (s, 3H), 2.31-2.51 (bm, 2H), 3.65 (bm, 2H), 3.91 (bs, 2H), 7.32 (d, *J* =8.9 Hz, 2H), 7.60 (d, *J* =9.0 Hz, 2 H), 7.97 (bs, 1H), 8.31 (bs, 1H); ¹³ C NMR (101 MHz, CDCl₃) δ 11.9, 12.0, 34.3, 43.4, 46.2, 52.7, 55.2, 115.2, 127.0, 127.5, 128.4, 130.2, 133.5, 141.1, 150.1, 167.0; HPLC purity = 99%.

### EXAMPLE 12. SYNTHESIS OF N-(3-CHORO-4-METHYLPHENYL)-2,5-DIMETHYL-4-(PYRROLIDNE-1-CARBONYL)-1H-PYRROLE-3-SULFONAMIDE (Compound 31)

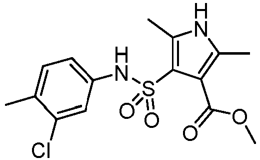

To a solution of Methyl 4-(chlorosulfonyl)-2,5-dimethyl-1H-pyrrole-3-carboxylate (356 mg, 1.4 mmol) in DCE (14 mL) cooled in an ice bath was added 3-chloro-4-methylaniline (198 mg, 1.4 mmol, 1 equiv.) and the reaction was stirred for 18 h, slowly warming to rt. The solvents were removed in vacuo and the residue purified by flash chromatography (EtOAc/hexanes) to afford product as a white solid (465 mg, 1.3 mmol, 92% yield).

¹H NMR (400 MHz, CDCl₃) δ 2.27 (s, 3H), 2.34 (s, 3H), 2.39 (s, 3H), 3.90 (s, 3H), 6.98 (dd, *J* =8.1, 2.1 Hz, 1H), 7.06 (d, *J =* 8.0 Hz, 1H), 7.18 (d, *J* =2.5 Hz, 1H), 8.07 (bs, 1H), 8.36 (s, 1H).

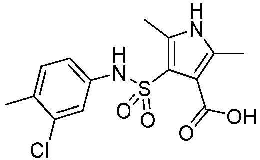

Methyl 4-(N-(3-chloro-4-methylphenyl)sulfamoyl)-2,5-dimethyl-1H-pyrrole-3-carboxylate (450 mg, 1.3 mmol) was dissolved in THF:MeOH (14 mL, 2:1) and lithium hydroxide (67.2 mg, 2.80 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford the acid product as a white solid (285 mg, 0.83 mmol, 66% yield).

¹H NMR (400 MHz, MeO-D₄) δ 2.24 (s, 3H), 2.26 (s, 3H), 2.37 (s, 3H), 6.91 (d, *J=* 8.2 Hz, 1H), 7.10 (d, *J=* 8 Hz, 1H), 7.16 (s, 1H).

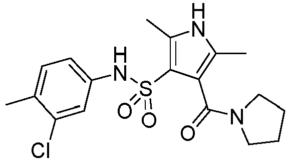

To a solution of 4-(N-(3-chloro-4-methylphenyl)sulfamoyl)-2,5-dimethyl-1H-pyrrole-3-carboxylic acid (285 mg, 0.83 mmol) in DMF (2 mL) was added PyBOP (648 mg, 1.25 mmol, 1.5 equiv.). After 10 mins, pyrrolidine (117 mg, 1.64 mmol, 2.0 equiv.) was added. The reaction was stirred at rt for 30 h, the solvent removed and the residue purified by flash chromatography (EtOAc/hexanes) to afford the amide product as a white solid (158 mg, 0.39 mmol, 48% yield).

¹H NMR (400 MHz, CDCl₃) 1.86 (bm, 2H), 1.94 (m, 2H), 2.13 (s, 3H), 2.16 (s, 3H), 2.25 (s, 3H), 3.36 (t, *J=* 6.6 Hz, 2H), 3.55 (bm. 2H).

### EXAMPLE 13. SYNTHESIS OF N-(3-CHLORO-4-(TRIFLUOROMETHYL)PHENYL)-2,5-DIETHYL-4-(PYRROLIDINE-1-CARBONYL)-1H-PYRROLE-3-SULFONAMIDE

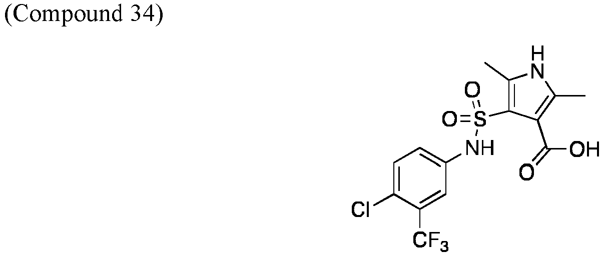

Methyl 4-(N-(4-chloro-3-(trifluoromethyl)phenyl)sulfamoyl)-2,5-dimethyl-1H-pyrrole-3-carboxylate (93 mg, 0.23 mmol) was dissolved in THF:MeOH (3 mL, 2:1) and lithium hydroxide (11 mg, 0.46 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford product as a white solid (58 mg, 0.15 mmol, 65% yield).

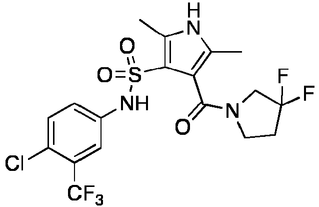

To a solution of 4-(*N*-(4-chloro-3-(trifluoromethyl)phenyl)sulfamoyl)-2,5-dimethyl-1*H*-pyrrole-3-carboxylic acid (50 mg, 0.13 mmol) in DMF (2 mL) was added PyBOP (104 mg, 0.20 mmol, 1.5 equiv.). After 10 mins, 3,3-difluoropyrrolidine (28 mg, 0.26 mmol, 2.0 equiv.) was added. The reaction was stirred at rt for 30 h, the solvent removed and the residue purified by flash chromatography (EtOAc/hexanes) to afford the amide product as a white solid (23 mg, 0.047 mmol, 38% yield).

MP = 92-106 °C; ¹H NMR (500 MHz, Acetone-D₆) δ 2.17 (s, 3H), 2.22 (s, 3H), 2.47 (bm, 2H), 3.66 (bm, 2H), 3.73-4.13 (bm, 2H), 7.49 (dd, *J =,* 1H), 7.55 (d, *J =,* 1H), 7.70 (d, *J =,* 1H), 8.52 (s, 1H), 10.50 (s, 1H); ¹⁹F NMR (376 MHz) δ -63.2; HPLC purity = 99%.

### EXAMPLE 14. SYNTHESIS OF N-(3,5-DICHLORO-4-(TRIFLUOROMETHYL)PHENYL)-4-(3,3-DIFLUOROPYRROLIDINE-1-CARBONYL)-2,5-DIMETHYL-1H-PYRROLE-3-SULFONAMIDE (Compound 35)

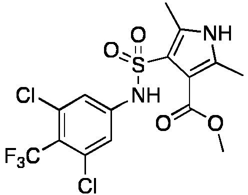

To a solution of methyl 4-(chlorosulfonyl)-2,5-dimethyl-1H-pyrrole-3-carboxylate (250 mg, 0.993 mmol) in DCE (12 mL) cooled in an ice bath was added 3,5-dichloro-4-(trifluoromethyl)aniline (228 mg, 0.993 mmol, 1 equiv.) and the reaction was stirred for 18 h, slowly warming to rt. The solvents were removed in vacuo and the residue purified by flash chromatography (EtOAc/hexanes) to afford the sulfonamide product as a white solid (413 mg, 0.928 mmol, 93% yield).

Mp = 202-228 °C; ¹H NMR (500 MHz, Acetone-D₆) δ 2.37 (s, 3H), 2.52 (s, 3H), 3.85 (s, 3H), 7.44 (s, 2H), 9.15 (s, 1H), 10.88 (bs, 1H); ¹³ C NMR (101 MHz, Acetone-D₆) δ 12.9, 13.5, 51.9, 110.2, 116.5, 120.6, 122.4, 125.1, 135.3, 136.4, 137.2, 144.1, 165.3; ¹⁹F NMR (376 MHz) δ -55.1

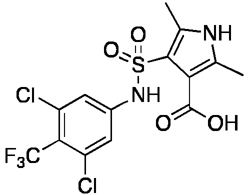

Methyl 4-(*N*-(3,5-dichloro-4-(trifluoromethyl)phenyl)sulfamoyl)-2,5-dimethyl-1*H*-pyrrole-3-carboxylate (262 mg, 0.6 mmol) was dissolved in THF:MeOH (9 mL, 2:1) and lithium hydroxide (28.8 mg, 1.20 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford the carboxylic acid product as a white solid (113 mg, 0.26 mmol, 44% yield).

Mp = 214-218 °C; ¹H NMR (400 MHz, MeO-D₄) δ 2.37 (s, 3H), 2.45 (s, 3H), 7.28 (s, 2H); ¹³ C NMR (101 MHz, MeO-D₆) δ 12.8, 13.4, 111.1, 116.2, 120.9 (q, ), 121.3, 123.0, 125.2, 127.4, 135.8, 136.8, 138.0, 144.3, 167.8; ¹⁹F NMR (376 MHz) δ -56.3; FTIR (neat): 3307, 2916, 2848, 1639, 1291, 1117.

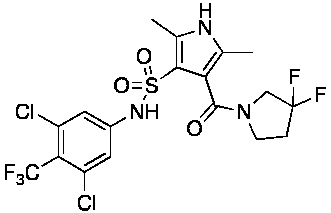

To a solution of 4-(N-(3,5-dichloro-4-(trifluoromethyl)phenyl)sulfamoyl)-2,5-dimethyl-1H-pyrrole-3-carboxylic acid (56 mg, 0.13 mmol) in DMF (2 mL) was added PyBOP (104 mg, 0.20 mmol, 1.5 equiv.). After 10 mins, 3,3-difluoropyrrolidine (28 mg, 0.26 mmol, 2.0 equiv.) was added. The reaction was stirred at rt for 30 h, the solvent removed and the residue purified by flash chromatography (EtOAc/hexanes) to afford the amide product as a white solid (16 mg, 0.031 mmol, 24% yield).

Mp = 119-128 °C; ¹H NMR (500 MHz, Acetone-D₆) δ 2.16 (s, 3H), 2.43 (s, 3H), 3.62 (bm, 2H), 3.69-4.09 (bm, 4H), 7.46 (s, 2H), 10.62 (bs, 1H).

### EXAMPLE 15. SYNTHESIS OF N-(3-CCHLORO-4-CYCLOPROPYLPHENYL)-4-(3,3-DIFLUOROPYRROLIDINE-1-CARBONYL)-2,5-DIMETHYL-1H-PYRROLE-3-SULFONAMIDE (Compound 36)

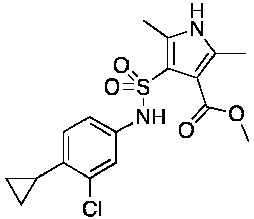

To a solution of methyl 4-(chlorosulfonyl)-2,5-dimethyl-1H-pyrrole-3-carboxylate (98 mg, 0.39 mmol) in DCE (8 mL) cooled in an ice bath was added 3-chloro-4-cyclopropylaniline (65 mg, 0.39 mmol, 1 equiv.) and the reaction was stirred for 18 h, slowly warming to rt. The solvents were removed in vacuo and the residue purified by flash chromatography (EtOAc/hexanes) to afford the sulfonamide product as a white solid (120 mg, 0.31 mmol, 80% yield.

Mp =119-135 °C; ¹H NMR (400 MHz, CDCl₃) δ 0.60 (m, 2H), 0.95 (m, 2H), 2.07 (m, 1H), 2.32 (s, 3H), 2.38 (s, 3H), 3.88 (s, 3H), 6.55 (d, J= , 1H), 6.97 (dd, J=, 1H), 7.17 (d, J=, 1H), 8.28 (s, 1H), 8.38 (s, 1H); ³C NMR (101 MHz) δ 8.0, 12.9, 14.2, 51.9, 109.7, 117.1, 120.2, 122.0, 126.6, 134.4, 135.4, 136.6, 137.4, 165.3; FTIR (neat): 3275 ,3160, 2950, 1683, 1448, 1230, 1190 cm⁻¹.

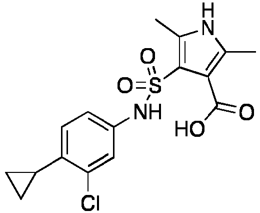

Methyl 4-(N-(3-chloro-4-cyclopropylphenyl)sulfamoyl)-2,5-dimethyl-1H-pyrrole-3-carboxylate (65 mg, 0.17 mmol) was dissolved in THF:MeOH (3 mL, 2:1) and lithium hydroxide (8.2 mg, 0.34 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford the carboxylic acid product as a white solid (34 mg, 0.092 mmol, 54% yield).

Mp = 178-190 °C (Dec); ¹H NMR (400 MHz, MeO-D₄) δ 0.57 (m, 2H), 0.92 (m, 2H), 2.04 (m, 1H), 2.27 (s, 3H), 2.36 (s, 3H), 6.75 (d, *J=* , 1H), 6.90 (dd, J= , 1H), 7.18 (d, J-, 1H).

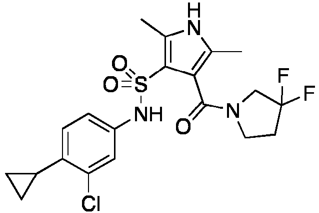

To a solution of 4-(N-(3-Chloro-4-cyclopropylphenyl)sulfamoyl)-2,5-dimethyl-1H-pyrrole-3-carboxylic acid (17 mg, 0.05 mmol) in DMF (2 mL) was added PyBOP (39 mg, 0.075 mmol, 1.5 equiv.). After 10 mins, 3,3-difluoropyrrolidine (11 mg, 0.10 mmol, 2.0 equiv.) in THF (0.5 mL) was added. The reaction was stirred at rt for 30 h, the solvent removed and the residue purified by flash chromatography (EtOAc/hexanes) to afford the amide product as a white solid (13 mg, 0.028 mmol, 62% yield).

Mp = 90-107 °C ¹H NMR (500 MHz, Acetone-D₆) ; ¹³C NMR (101 MHz, CDCl₃) δ ¹⁹F NMR (376 MHz) δ FTIR (neat): HRMS (m/z): calcd; (98% purity).

### EXAMPLE 16. SYNTHESIS OF N-(3-CHLORO-4-(TRIFLUOROMETHYL)PHENYL)-2,5-DIETHYL-4-(PYRROLIDINE-1-CARBONYL)-1H-PYRROLE-3-SULFONAMIDE

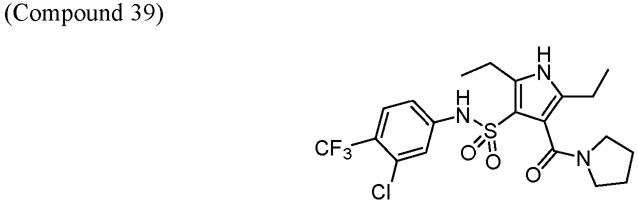

To a solution of 4-(*N*-(3-chloro-4-(trifluoromethyl)phenyl)sulfamoyl)-2,5-diethyl-1*H*-pyrrole-3-carboxylic acid (27 mg, 0.064 mmol) in DMF (2 mL) was added PyBOP (50 mg, 0.096 mmol, 1.5 equiv.). After 10 mins, pyrrolidine (9.1 mg, 0.128 mmol, 2.0 equiv.) in THF (0.5 mL) was added. The reaction was stirred at rt for 30 h, the solvent removed and the residue purified by flash chromatography (EtOAc/hexanes) to afford the amide product as a white solid (14 mg, 0.029 mmol, 46% yield).

### EXAMPLES 17. SYNTHESIS OF N-(3-CHLORO-5-(TRIFLUOROMETHYL)PHENYL)-4-(3,3-DIFLUOROPYRROLIDINE-1-CARBONYL)-2,5-DIMETHYL-1H-PYRROLE-3-SULFONAMIDE (Compound 40)

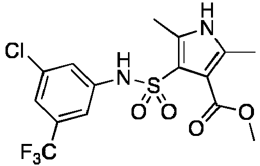

To a solution of Methyl 4-(chlorosulfonyl)-2,5-dimethyl-1H-pyrrole-3-carboxylate (90 mg, 0.36 mmol, 1 equiv.) in DCE (4 mL) cooled in an ice bath was added 3-chloro-4-cyclopropylaniline (65 mg, 0.36 mmol, 1 equiv.) and the reaction was stirred for 18 h, slowly warming to rt. The solvents were removed in vacuo and the residue purified by flash chromatography (EtOAc/hexanes) to afford the sulfonamide product as a white solid (132 mg, 0.321 mmol, 90% yield).

Mp = 226-235 °C; ¹HNMR (400 MHz, Acetone-D₆) δ 2.36 (s, 3H), 2.43 (s, 3H), 3.86 (s, 3H), 7.39 (s, 1H), 7.49 (s, 1H), 7.54 (s, 1H) 8.94 (s, 1H), 10.79 bs, 1H); ¹³C NMR (101 MHz) δ 12.7, 13.5, 51.9, 110.2, 115.3, 116.8, 122.9, 123.6, 125.6, 132.9, 133.3, 135.9, 142.4, 165.6; ¹⁹F NMR (376 MHz) -63.6; FTIR (neat): 3266, 2922, 1672, 1448, 1319 cm⁻¹.

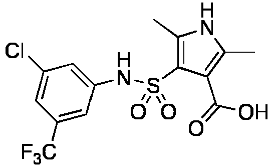

Methyl 4-(*N*-(3-chloro-5-(trifluoromethyl)phenyl)sulfamoyl)-2,5-dimethyl-1*H*-pyrrole-3-carboxylate (92 mg, 0.22 mmol, 1 equiv.) was dissolved in THF:MeOH (3 mL, 2:1) and lithium hydroxide (10.6 mg, 0.44 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford the carboxylic acid product as a white solid (40 mg, 0.22 mmol, quantitative yield).

Mp = 194-206 °C (Dec); ¹H NMR (400 MHz, MeO-D₄) δ 2.34 (s, 3H), 7.33 (s, 1H), 7.36 (t, *J=* , 1H), 7.40 (s, 1H) ¹³C NMR (101 MHz, MeO-D₄) δ; ¹⁹F NMR (376 MHz) δ -64.6; FTIR (neat): 3303, 2917, 2848, 1451, 1321, 1126 cm⁻¹; HRMS (ESI) *m*/*z* calcd for C₁₄H₁₁N₂O₄SF₃Cl [M-H]⁻ 395.01584; found 395.00848.

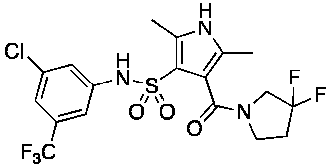

To a solution of 4-(*N*-(3-chloro-5-(trifluoromethyl)phenyl)sulfamoyl)-2,5-dimethyl-1*H*-pyrrole-3-carboxylic acid (25 mg, 0.063 mg, 1 equiv.) in DMF (2 mL) was added PyBOP (50 mg, 0.094 mmol, 1.5 equiv.). After 10 mins, 3,3-difluoropyrrolidine (13.5 mg, 0.126 mmol, 2.0 equiv.) in THF (0.5 mL) was added. The reaction was stirred at rt for 30 h, the solvent removed and the residue purified by flash chromatography (EtOAc/hexanes) to afford the amide product as a white solid (14 mg, 0.029 mmol, 48% yield).

Mp = 182-185°C (Dec); ¹H NMR (500 MHz, Acetone-D₆) δ 2.17 (s, 3H), 2.28 (s, 3H), 2.48 (bm, 2H), 3.54 (bm, 1H), 7.43 (s, 1H), 7.54 (s, 1H), 7.56 (s, 1H); ¹³C NMR (126 MHz, Acetone-D₆) δ 11.6, 11.7, 33.5, 35.1, 43.9, 46.7, 53.1, 55.1, 115.0, 116.4, 121.2, 123.1, 124.7, 125.3, 132.7, 134.3, 135.7, 142.4, 167.0; ¹⁹F NMR (376 MHz) δ -63.6; FTIR (neat): 3386, 3162, 2916, 2848, 1602, 1576, 1319, 1124; HRMS (ESI) *m*/*z* calcd for C₁₈H₁₆N₃O₃SF₅Cl [M-H]- 484.05993; found 484.05272.

### EXAMPLE 18. SYNTHESIS OF N-(2-BROMO-5-CHLORO-4-(TRIFLUOROMETHYL)PHENYL)-4-(3,3-DIFLUOROPYRROLIDINE-1-CARBONYL)-2.5-DIMETHYL-1H-PYRROLE-3-SULFONAMIDE (Compound 41)

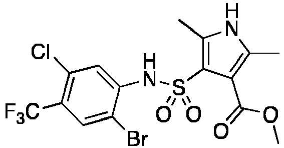

Methyl 4-(chlorosulfonyl)-2,5-dimethyl-1*H*-pyrrole-3-carboxylate (90 mg, 0.36 mmol) in DCE (4 mL) cooled in an ice bath was added 2-bromo-5-chloro-4-(trifluoromethyl)aniline (99 mg, 0.36 mmol, 1 equiv.) and the reaction was stirred for 18 h, slowly warming to rt. The solvents were removed in vacuo and the residue purified by flash chromatography (EtOAc/hexanes) to afford the sulfonamide product as a white solid (129 mg, 0.263 mmol, 74% yield).

Mp = 168 -204 °C (Dec); ¹H NMR (400 MHz, CDCl₃) δ 2.41 (s, 3H), 2.56 (s, 3H), 3.87 (s, 3H), 7.76 (s, 1H), 7.80 (s, 1H), 8.46 (s, 1H), 8.76 (s, 1H); ¹³C NMR (101 MHz) δ 13.2, 14.1, 51.9, 110.1, 116.6, 120.3, 120.8, 123.5, 123.9, 131.8, 132.3, 135.2, 136.2, 140.6, 164.1; ¹⁹F NMR (376 MHz) δ -62.2; FTIR (neat): 3199, 2117, 2923, 2852, 1690, 1577, 1306, 1107 cm⁻¹; HRMS (ESI) *m*/*z* calcd for C₁₅H₁₂N₂O₄SF₃ClBr [M-H]⁻ 486.94200; found 486.93491.

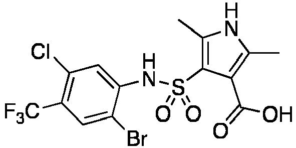

Methyl 4-(*N*-(2-bromo-5-chloro-4-(trifluoromethyl)phenyl)sulfamoyl)-2,5-dimethyl-1*H*-pyrrole-3-carboxylate (96 mg, 0.20 mmol, 1 equiv.) was dissolved in THF:MeOH (3 mL, 2:1) and lithium hydroxide (9.6 mg, 0.40 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford the carboxylic acid product as a white solid (35 mg, 0.074 mmol, 38% yield).

¹H NMR (400 MHz, MeO-D₄) δ 2.39 (s, 3H), 2.50 (s, 3H), 7.80 (s, 1H), 7.88 (s, 1H); ¹⁹F NMR (376 MHz) δ -63.5; HRMS (ESI) *m*/*z* calcd for C₁₄H₁₂N₂O₄SF₃ClBr [M+H]+ 473.92635; found 474.93413.

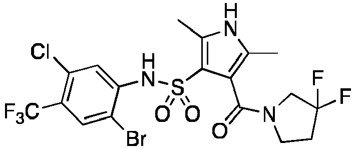

To a solution of 4-(*N*-(2-bromo-5-chloro-4 (trifluoromethyl)phenyl)sulfamoyl)-2,5-dimethyl-1*H*-pyrrole-3-carboxylic acid (18 mg, 0.038 mmol, 1 equiv.) in DMF (2 mL) was added PyBOP (30 mg, 0.057 mmol, 1.5 equiv.). After 10 mins, difluoropyrrolidine (8.1 mg, 0.076 mmol, 2.0 equiv.) in THF (0.5 mL) was added. The reaction was stirred at rt for 30 h, the solvent removed and the residue purified by flash chromatography (EtOAc/hexanes) to afford the amide product as a white solid (8 mg, 0.01 mmol, 40% yield).

### EXAMPLE 19. SYNTHESIS OF N-(4-CHLORO-2-(TRIFLUOROMETHYL)PHENYL)-4-(2,2-DIFLUOROPYRROLIDINE-1-CARBONYL)-2,5-DIMETHYL-1H-PYRROLE-3-SULFONAMIDE (Compound 42)

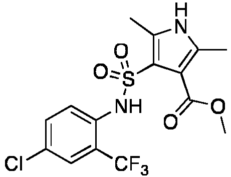

To a solution of methyl 4-(chlorosulfonyl)-2,5-dimethyl-1*H*-pyrrole-3-carboxylate (93 mg, 0.37 mmol, 1 equiv.) in DCE (4 mL) cooled in an ice bath was added 4-chloro-2-(trifluoromethyl)aniline (72.3 mg, 0.37 mmol, 1 equiv.) and the reaction was stirred for 18 h, slowly warming to rt. The solvents were removed in vacuo and the residue purified by flash chromatography (EtOAc/hexanes) to afford the sulfonamide product as a white solid (65 mg, 0.16 mmol, 43% yield).

Mp= 159-173 (Dec); ¹H NMR (400 MHz, Acetone-D₄) δ 2.41 (s, 3H), 2.47 (s, 3H), 3.83 (s, 3H), 7.63 (m, 3H), 8.73 (s, 1H), 10.97 (s, 1H); ¹³C NMR (101 MHz, Acetone-D₆) δ; 12.6, 13.6, 51.8, 110.1, 118.4, 122.7, 124.2, 125.4, 127.4, 129.6, 134.0, 134.8, 136.4, 137.2, 165.5; ¹⁹F NMR (376 MHz) δ -61.2; FTIR (neat): 3247, 2954, 2922, 2851, 1681, 1311, 1116 cm⁻¹; HRMS (ESI) *m*/*z* calcd for C₁₅H₁₃N₂O₄SF₃Cl [M-H]⁻ 409.03149; found 409.02426.

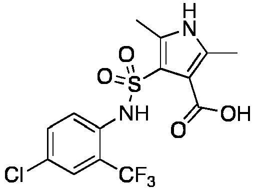

Methyl 4-(*N*-(4-chloro-2-(trifluoromethyl)phenyl)sulfamoyl)-2,5-dimethyl-1*H*-pyrrole-3-carboxylate (42 mg, 0.10 mmol, 1 equiv.) was dissolved in THF:MeOH (3 mL, 2:1) and lithium hydroxide (4.8 mg, 0.20 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford the carboxylic acid product as a white solid (28 mg, 0.071 mmol, 69% yield).

Mp = 188-204 (Dec); ¹H NMR (400 MHz, Acetone-D₆) δ 2.45 (s, 3H), 2.47 (s, 3H), 7.57-7.67 (m, 3H), 10.86 (s, 1H); ¹³C NMR (101 MHz, Acetone-D₆) δ 12.6, 13.6, 118.7, 122.6, 124.3, 125.4, 127.4, 129.5, 133.9, 134.6, 136.7, 137.4, 166.3; ¹⁹F NMR (376 MHz) δ -61.2; FTIR (neat): 3300, 2918, 2849, 1641, 1314, 1120 cm⁻¹.

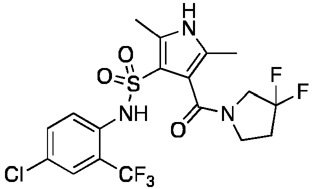

To a solution of 4-(*N*-(4-Chloro-2-(trifluoromethyl)phenyl)sulfamoyl)-2,5-dimethyl-1*H*-pyrrole-3-carboxylic acid (21 mg, 0.053 mmol, 1 equiv.) in DMF (1 mL) was added PyBOP (41 mg, 0.079 mmol, 1.5 equiv.). After 10 mins, difluoropyrrolidine (11.4 mg, 0.106 mmol, 2.0 equiv.) in THF (0.5 mL) was added. The reaction was stirred at rt for 30 h, the solvent removed and the residue purified by flash chromatography (EtOAc/hexanes) to afford the amide product as a white solid (15 mg, 0.031 mmol, 58% yield) .

¹H NMR (400 MHz, Acetone-D₆) δ 2.24 (s, 3H), 2.32 (s, 3H), 2.45 (m, 2H), 3.60-4.00 (m, 4H), 7.47 (d, *J* =8.99 Hz, 1H), 7.60 (dd, *J =* 8.88 Hz, 2.49 Hz, 1H), 7.67 (d, *J=* 2.56 Hz, 1H), 8.31 (s, 1H), 10.59 (s, 1H); ¹⁹F NMR (376 MHz, Acetone-D₆) δ -71.8, -71.9, - 60.5; HPLC purity = 99 %.

### EXAMPLE 20. SYNTHESIS OF N-(2-CHLORO-6-(TRIFLUOROMETHYL)PHENYL)-4-(2,2-DIFLUOROPYRROLIDINE-1-CARBONYL)-2,5-DIMETHYL-1H-PYRROLE-3-SULFONAMIDE (Compound 43)

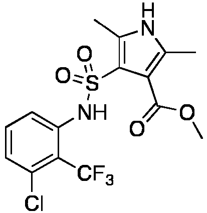

To a solution of methyl 4-(chlorosulfonyl)-2,5-dimethyl-1*H*-pyrrole-3-carboxylate (93 mg, 0.37 mmol, 1 equiv.) in DCE (2 mL) cooled in an ice bath was added 3-chloro-2-(trifluoromethyl)aniline (72.3 mg, 0.37 mmol, 1 equiv.) and the reaction was stirred for 18 h, slowly warming to rt. The solvents were removed in vacuo and the residue purified by flash chromatography (EtOAc/hexanes) to afford the sulfonamide product as a white solid (135 mg, 0.32 mmol, 89% yield).

Mp = 181-211 °C (Dec); ¹H NMR (400 MHz, Acetone-D₆) δ 2.38 (s, 3H), 2.40 (s, 3H) 3.83 (s, 3H) 7.36 (d, *J =* 7.9 Hz, 1H), 7.48 (m, 2H), 8.86 (s, 1H), 10.84 (bs, 1H); ¹³C NMR (101 MHz, Acetone-D₆) δ; 12.6, 13.6, 51.8, 110.1, 117.7, 119.9, 120.2, 122.1, 122.3, 123.1, 125.9, 128.5, 134.0, 135.4, 137.1, 139.6, 165.6; ¹⁹F NMR (376 MHz) δ -54.6; FTIR (neat): 3281, 1679, 1450, 1285, 1157,1111 cm⁻¹; HRMS (ESI) *m*/*z* calcd for C₁₅H₁₃N₂O₄SF₃Cl [M-H]⁻ 409.03149; found 409.02430.

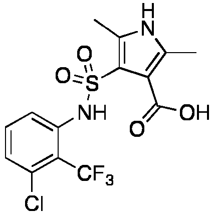

Methyl 4-(N-(2-chloro-6-(trifluoromethyl)phenyl)sulfamoyl)-2,5-dimethyl-1*H*-pyrrole-3-carboxylate (83 mg, 0.2 mmol, 1 equiv.) was dissolved in THF:MeOH (3 mL, 2:1) and lithium hydroxide (9.6 mg, 0.40 mmol, 2.0 equiv.) added and the reaction stirred at 60 °C for 72 h then cooled to rt. The reaction was acidified with 1 M aqueous HCl and the mixture extracted with EtOAc. The organic layer was concentrated and purified by flash chromatography (MeOH/DCM) to afford the carboxylic acid product as a white solid (27 mg, 0.068 mmol, 34% yield). Mp = 197-198 °C (Dec); ¹H NMR (400 MHz, Acetone-D₆) δ 2.40 (s, 3H), 2.45 (s, 3H), 7.35 (d, *J=* 7.6 Hz, 1H), 7.44-7.52 (m, 2H), 10.84 (s, 1H); ¹³C NMR (101 MHz, Acetone-D₆) δ 12.6, 13.6, 110.3, 117.9, 122.1, 123.1, 125.8, 128.4, 133.9, 134.3, 135.2, 137.4, 139.8, 166.3; ¹⁹F NMR (376 MHz) δ -54.6; FTIR (neat): 3287, 2916, 2848, 1643, 1456, 1201 cm⁻¹.

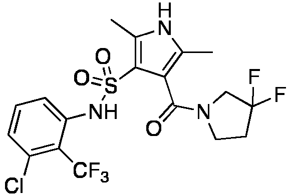

To a solution of 4-(*N*-(2-chloro-6-(trifluoromethyl)phenyl)sulfamoyl)-2,5-dimethyl-1*H*-pyrrole-3-carboxylic acid (24 mg, 0.060 mmol, 1 equiv.) in DMF (1 mL) was added PyBOP (47 mg, 0.09 mmol, 1.5 equiv.). After 10 mins, difluoropyrrolidine (12.9 mg, 0.12 mmol, 2.0 equiv.) in THF (0.5 mL) was added. The reaction was stirred at rt for 30 h, the solvent removed and the residue purified by flash chromatography (EtOAc/hexanes) to afford the amide product as a white solid (25 mg, 0.051 mmol, 85% yield). Mp = 167-174 °C (Dec); ¹H NMR (400 MHz, (CD₃)₂CO) δ 2.12 (s, 3H), 2.24 (s, 3H), 2.47 (m, 2H), 3.62-4.02 (m, 4H), 7.21 (m, 1H), 7.47 (m, 2H), 8.40 (m, 1H), 10.53 (m, 1H); ¹⁹F NMR (376 MHz, (CD₃)₂CO) δ -73.7, -71.9, -54.9; FTIR (neat): 3264, 2919, 2849, 1615, 1447, 1286 cm⁻¹; HPLC purity = 99%.

### EXAMPLE 21. SYNTHESIS OF ETHYL 2-OXO-5-(TRIFLUOROMETHOXY)-4'H-SPIRO[INDOLINE-3,5'-ISOXAZOLE]-3'-CARBOXYLATE (Compound 25)

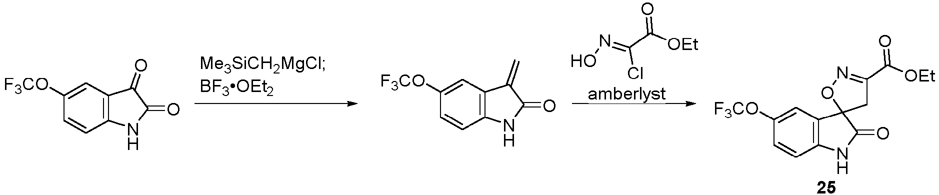

### Step I: Synthesis of 3-Hydroxy-5-(trifluoromethoxy)-3-((trimethylsilyl)methyl)indolin-2-one

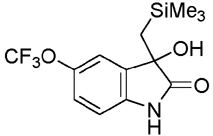

A solution of (trimethylsilyl)methyl)magnesium chloride (1 molar (M) in THF, 2.60 mL, 2.60 mmol, 2.0 equiv.) was added to a cold (-78 °C) suspension of 5-(trifluoromethoxy)indoline-2,3-dione (300.0 mg, 1.30 mmol) in diethyl ether (15 mL). The reaction was stirred at -78 °C then allowed to warm to rt. Once warmed to rt, the reaction was quenched with methanol and the reaction adsorbed onto Celite and purified by flash chromatography to afford the product as an off-white solid (237 mg, 0.742 mmol, 42% yield). *R_{f}* = 0.4 (50% EtOAc in hexanes).

¹H NMR (400 MHz, chloroform-d) δ 1.73 (s, 2H), 7.10 (d, *J =* 8.4 Hz, 1H), 7.36 (d, *J =* 8.0 Hz, 1H), 8.01 (s, 1H);

### Step II: Synthesis of 3-Methylene-5-(trifluoromethoxy)indolin-2-one

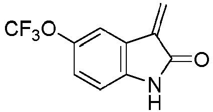

To a cold (-78 °C) solution of 3-hydroxy-5-(trifluoromethoxy)-3-((trimethylsilyl)methyl)indolin-2-one (225 mg, 0.705 mmol) in CH₂Cl₂ (15 mL) was added boron trifluoride diethyl etherate (0.45 mL, 3.52 mmol, 5.0 equiv.). The reaction was stirred at -78 °C for 2 h then warmed to 0 °C for 1 h, poured into saturated, aqueous NaHCO₃ (25 mL) and extracted with diethyl ether (3 × 20 mL). The combined organic layers were dried with anhydrous MgSO₄, evaporated and used without further purification (160 mg, 0.698 mmol, 99% yield).

¹H NMR (400 MHz, Chloroform-d) δ 6.12 (d, *J* = 0.9 Hz, 1H), 6.41 (s, 1H), 6.77 (d, *J =* 8.5 Hz, 1H), 7.06 (s, 1H), 7.27 (s, 1H).

### Step III: Synthesis of Ethyl 2-oxo-5-(trifluoromethoxy)-4'H-spiro[indoline-3,5'-isoxazole]-3'-carboxylate (Compound 25)

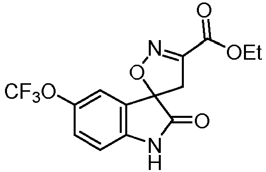

A solution of 3-methylene-5-(trifluoromethoxy)indolin-2-one (150 mg, 0.655 mmol) in CH₂Cl₂ (1.5 mL) was added to flamed-dried and cooled (rt) molecular sieves (230 mg). A solution of (Z)-ethyl 2-chloro-2-(hydroxyimino)acetate (298 mg, 1.96 mmol, 3.0 equiv.) in CH₂Cl₂ (15 mL) was added to the reaction through a tube containing Aberlyst A21 resin (2.4 g) at a rate of 0.16 mL/min. After complete addition, the reaction was stirred at rt for 15 h, adsorbed onto Celite and purified by flash chromatography to afford the spirocyclic product as a yellow oil (100 mg, 0.290 mmol, 44% yield). Compound 25 is obtained as a 1:1 racemic mixture.

¹H NMR (400 MHz, chloroform-d) δ 1.62 (t, *J =* 7.1 Hz, 3H), 3.75 (d, *J =* 18.3 Hz, 1H), 4.02 (d, *J =* 18.3 Hz, 1H), 4.62 (q, *J =* 7.1 Hz, 2H), 7.13 (d, *J =* 9.1 Hz, 1H), 7.38-7.46 (m, 2H), 8.07 (br s, 1H).

### EXAMPLE 22. D3 DAR β-ARRESTIN RECRUITMENT ASSAY

A D3 DAR β-arrestin cell line from DiscoverX (Fremont, CA) was used as described in the protocol below (Table 2). A CHO cell line engineered to overexpress the D3 DAR fused with a small 42-amino acid fragment of β-galactosidase called ProLink^{™} and a fusion protein consisting of β-arrestin and a larger N-terminal deletion mutant of β-galactosidase was used (DiscoverX catalogue number 93-0579C2). When the D3 DAR is activated by dopamine, it stimulates binding of β-arrestin to ProLink-tagged D₃ DARs, and the two complementary parts of β-galactosidase form a functional enzyme. When substrate (PathHunter^{®} Detection reagent, DiscoverX 93-0001) is added, β-galactosidase hydrolyzes it and generates a chemiluminescent signal.

| Table 2. Protocol for D3 DAR β-arrestin recruitment assay | | | |
|---|---|---|---|
| Sequence | Parameter | Value | Description |
| 1 | Cells | 7.5 µL | 2,625 cells/well |
| 2 | Time | 16-20 hr | Incubate at 37 °C and 5% CO₂ |
| 3 | Reagent | 2.5 µL | Test compound in the presence of an EC₈₀ concentration of DA (30 nM), sulpiride as control (in DMSO) |
| 4 | Time | 90 min | Incubate at 37 °C and 5% CO₂ |
| 5 | Detection Reagent | 10 µL | 1:5:19 Galacton Star Substrate: Emerald II solution: PathHunter buffer |
| 6 | Time | 30 min | Room temperature incubation |
| 7 | Detector | 2 sec | Luminescent settings, µcell plate reader |

### EXAMPLE 23. D2 DAR β-ARRESTIN RECRUITMENT ASSAY

For a secondary-screen and selectivity assays, DAR PathHunter^{®} β-arrestin GPCR cell lines from DiscoverX (Fremont, CA) were used. In the D2 DAR PathHunter^{®} β-arrestin GPCR cell line, the D2 DAR is overexpressed and fused with a small 42-amino acid fragment of β-galactosidase called ProLink^{™} on a CHO cellular background expressing a fusion protein of β-arrestin and a larger *N*-terminal deletion mutant of β -galactosidase ("enzyme acceptor"). When the D2 DAR is activated by dopamine, it stimulates binding of β-arrestin to the ProLink-tagged DAR and the two complementary parts of β-galactosidase form a functional enzyme. When substrate (PathHunter^{™} Detection reagent) is added, β-galactosidase hydrolyzes it and generates a chemiluminescent signal. Table 3 summarizes the protocol for the D2 DAR β-arrestin recruitment assay.

| Table 3. Protocol for the D2 DAR β-arrestin recruitment assay | | | |
|---|---|---|---|
| Sequence | Parameter | Value | Description |
| 1 | Cells | 7.5 µL | 2,625 cells/well |
| 2 | Time | 16-20 hr | Incubate at 37 °C and 5% CO₂ |
| 3 | Reagent | 2.5 µL | Test compound in the presence of an EC₈₀ concentration of DA (1 µM), sulpiride as control (in DMSO) |
| 4 | Time | 90 min | Incubate at 37 °C and 5% CO₂ |
| 5 | Detection Reagent | 10 µL | 1:5:19 Galacton Star Substrate: Emerald II solution: PathHunter buffer |
| 6 | Time | 30 min | Room temperature incubation |
| 7 | Detector | 2 sec | Luminescent settings, µcell plate reader |

### EXAMPLE 24. ADDITIONAL COMPOUNDS

Table 4 shows compounds of Examples 1-21 with pharmacological data derived from the DiscoverX β-arrestin recruitment functional assay in Examples 22 and 23, and shows additional compounds prepared by the methods shown in Example 1. Table 4 shows further additional compounds which were prepared by the methods shown in Examples 1-21. Routine changes in starting materials and reaction conditions, readily apparent to those of one skilled in the art, were used to make the particular compounds disclosed in Table 4. A "*" is used to denote compounds with an IC₅₀ greater than 100 micromolar, a "**" indicates compound with an IC₅₀ between 50 micromolar and 100 micromolar, a "***" denotes compounds with an IC₅₀ between 10 micromolar and 50 micromolar, a "****" denotes compounds with an IC₅₀ less than 10 micromolar. A "+" is used to denote compounds with D3 DAR Imax (Imax = maximum inhibition) between 80% to 100%, "NA" indicates not active.

| Table 4: Compound Activity in the DiscoverX β-Arrestin Recruitment Assay | | | | | |
|---|---|---|---|---|---|
| | Structure | D3 DAR IC₅₀ | D3 DAR Imax | D2 DAR IC₅₀ | D2 DAR Imax |
| 1 | 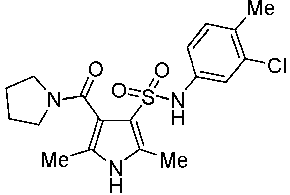 | *** | + | NA | NA |
| 2 | 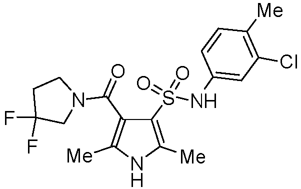 | *** | + | NA | NA |
| 3 | 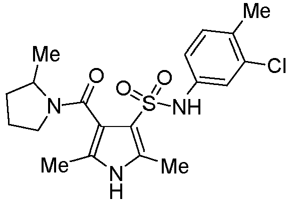 | *** | + | NA | NA |
| 4 | 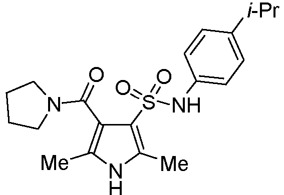 | *** | + | NA | NA |
| 5 | 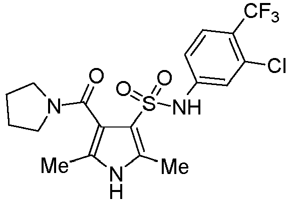 | **** | + | NA | NA |
| 6 | 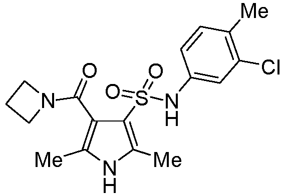 | *** | + | NA | NA |
| 7 | 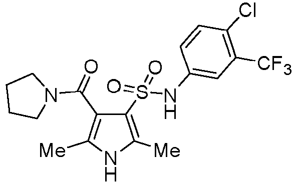 | **** | + | NA | NA |
| 8 | 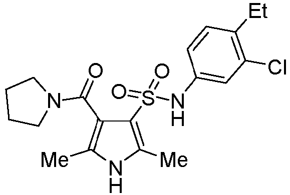 | *** | + | NA | NA |
| 9 | 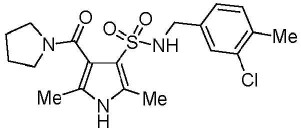 | ** | Curve Incomplete | NA | NA |
| 10 | 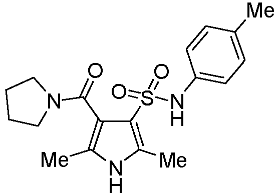 | *** | + | NA | NA |
| 11 | 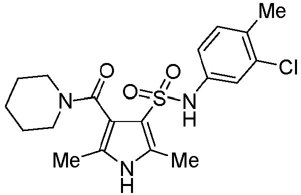 | *** | + | NA | NA |
| 12 | 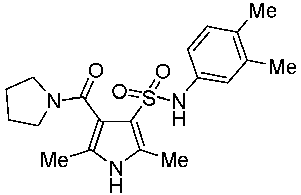 | *** | + | NA | NA |
| 13 | 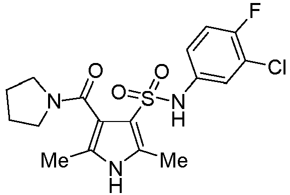 | *** | + | NA | NA |
| 14 | 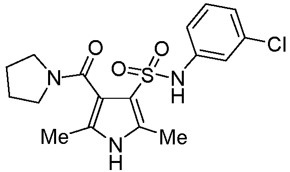 | *** | + | NA | NA |
| 15 | 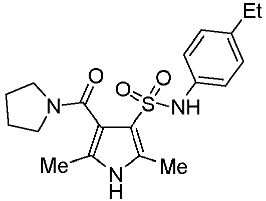 | **** | + | NA | NA |
| 16 | 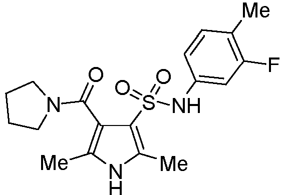 | *** | + | NA | NA |
| 17 | 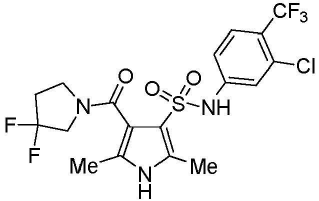 | **** | + | NA | NA |
| 18 | 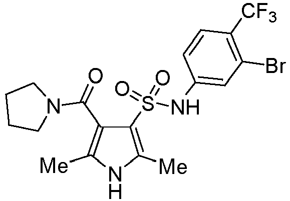 | **** | + | NA | NA |
| 19 | 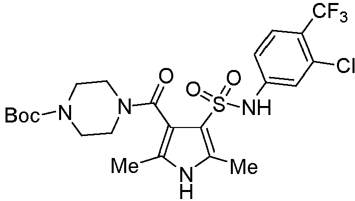 | ** | + | NA | NA |
| 20 | 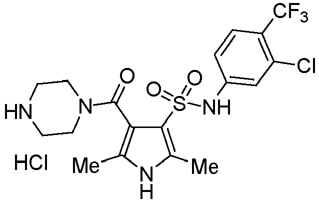 | ** | Curve Incomplete | NA | NA |
| 21 | 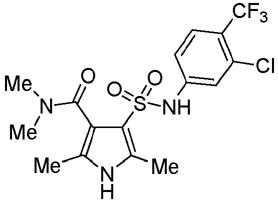 | **** | + | NA | NA |
| 22 | 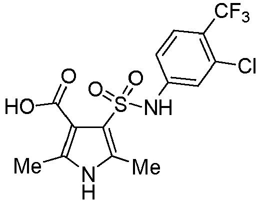 | ** | Curve Incomplete | NA | NA |
| 23 | 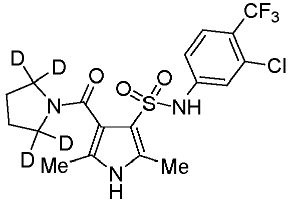 | **** | + | NA | NA |
| 24 | 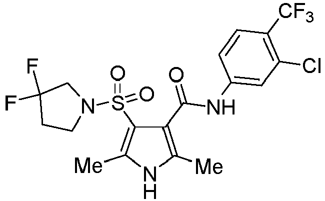 | ** | Curve Incomplete | NA | NA |
| 25 | 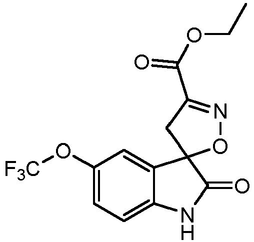 | ** * | + | NA | NA |
| 26 | 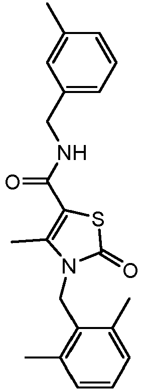 | ** * | + | NA | NA |
| 27 | 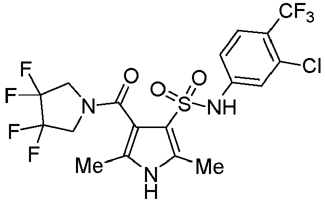 | *** | + | NA | NA |
| 28 | 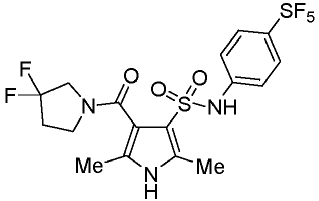 | **** | + | NA | NA |
| 29 | 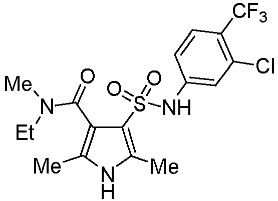 | *** | + | NA | NA |
| 30 | 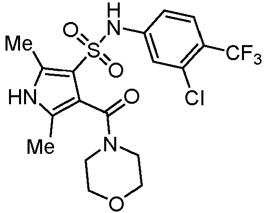 | * | Curve Incomplete | NA | NA |
| 31 | 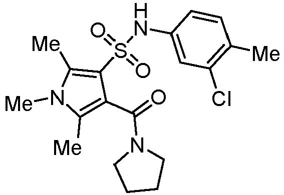 | * | Curve Incomplete | * | Curve Incomple te |
| 32 | 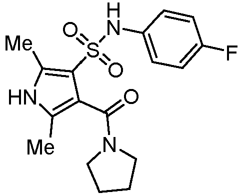 | * | Curve Incomplete | NA | NA |
| 33 | 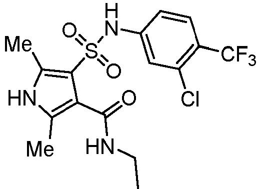 | **** | + | NA | NA |
| 34 | 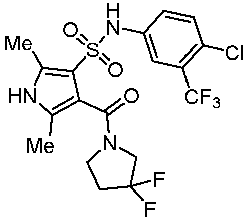 | *** | + | NA | NA |
| 35 | 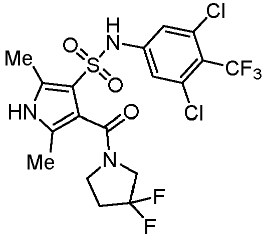 | **** | + | NA | NA |
| 36 | 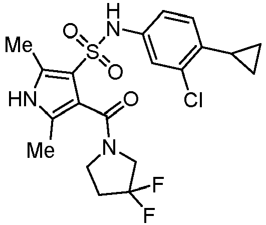 | *** | + | NA | NA |
| 37 | 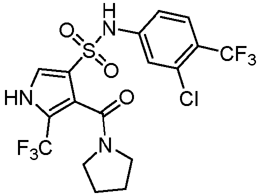 | *** | + | NA | NA |
| 38 | 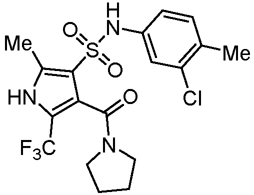 | **** | + | NA | NA |
| 39 | 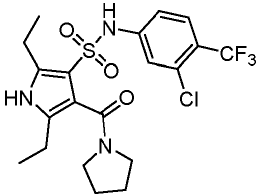 | *** | + | NA | NA |
| 40 | 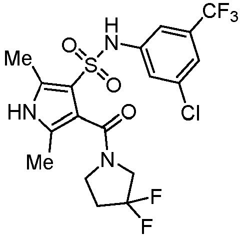 | *** | + | NA | NA |
| 41 | 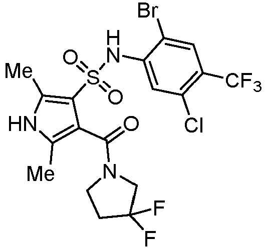 | **** | + | NA | NA |
| 42 | 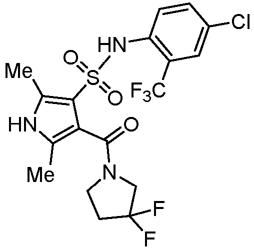 | *** | + | NA | NA |
| 43 | 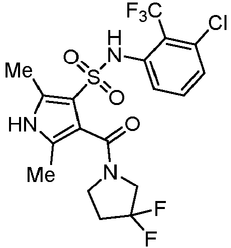 | ** | + | NA | NA |
| 44 | 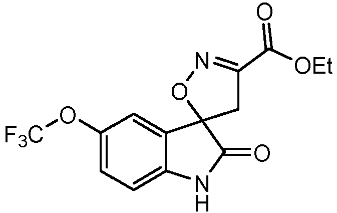 (+ enantiomer) | **** | + | NA | NA |
| 45 | 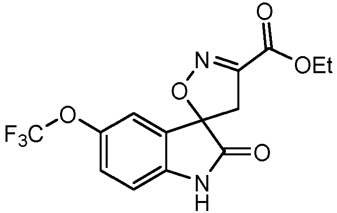 (- enantiomer) | *** | + | NA | NA |

### EXAMPLE 25. D3 RADIOLIGAND BINDING ASSAY

Compounds were tested for their ability to compete with the orthosteric radioligand [³H]-methylspiperone for binding to the D3 DAR using HEK cells transiently transfected with the D3 DAR as described in the detailed protocol below presented in Table 5. Cells were cultured in Dulbecco's modified Eagle's Medium (Corning, catalogue no. 10-013) containing 10% FBS, 1,000 units/mL Penicillin, 1,000 mg/mL Streptomycin, 100 mM sodium pyruvate, 1 µg/mL Gentamicin, and 250 mg/mL G418. All cells were maintained at 37 °C in 5% CO₂ and 90% humidity. For radioligand binding assays cells were removed mechanically using calcium-free Earle's balanced salt solution (EBSS). Intact cells were collected by centrifugation and then lysed with 5 mM Tris-HCl and 5 mM MgCl₂ at pH 7.4. Homogenates were centrifuged at 30,000 x g for 30 minutes. The membranes were resuspended in EBSS (US Biological, catalogue no. E0249-05) pH 7.4 to a concentration of 16 µg/mL. For competition binding studies, membrane preparations were incubated for 90 minutes at room temperature with various concentrations of compound and a single concentration of [³H]-methylspiperone (Perkin Elmer, NET856) in a reaction volume of 250 µL. Non-specific binding was determined in the presence of 4 µM (+)-butaclamol (Sigma-Aldrich, catalogue no. D033). Bound ligand was separated from unbound by filtration through GF/C filters using a PerkinElmer cell harvester and quantified on a Top-count (PerkinElmer). Ki values were determined using Cheng-Prusoff equation from observed IC₅₀ values and ligand Kd values from separate saturation experiments.

| Table 5. Protocol for the [³H]-methylspiperone radioligand binding assay | | | |
|---|---|---|---|
| Sequence | Parameter | Value | Description |
| 1 | Cells | 25 mL | 2 x 10⁷ cells/flask |
| 2 | Time | 24 h | Incubate at 37 °C and 5% CO₂ |
| 3 | Reagent | 10 mL | EBSS (-) |
| 4 | Time | 10 min | Room Temperature |
| 5 | Centrifuge | 900 x g | Pellet cells |
| 6 | Lysis | 6 mL | Re-suspend and homogenize in lysis buffer |
| 7 | Centrifuge | 30,000 x g | Pellet homogenate |
| 8 | Buffer | 10 mL | Re-suspend in EBSS to 16 µg protein/mL |
| 9 | Reagent | 25 µL | Buffer/Butaclamol/Test compound per assay well |
| 10 | Reagent | 125 µL | Radioligand per assay well |
| 11 | Lysate | 100 µL | Membrane preparation per assay well |
| 12 | Time | 90 min | Incubate at room temperature with shaking |
| 13 | Filter | 4 washes | Filter membranes onto GF/C filter plates |
| 14 | Reagent | 50 µL | Perkin Elmer scintillation cocktail |

### EXAMPLE 26. ORTHOGONAL SCREENING ASSAYS

**BRET β-arrestin recruitment assay:** As an orthogonal test using an unrelated assay of D2- or D3 DAR-mediated β-arrestin recruitment, an arrestin BRET assay was conducted. HEK293T cells transiently expressing D2-Rluc8, Arrestin3-mvenus and GRK2, or D3-Rluc8, Arrestin3-mvenus, and GRK3 were harvested with EBSS, plated in 96-well plates in Dulbecco's phosphate-buffered saline (DPBS) and incubated at rt for 45 min. Cells were incubated with 5 µM coelenterazine H (the substrate of Rluc8, Nanolight Technology, Pinetop AZ) for 5 min, then stimulated with the indicated concentrations of either sulpiride or a test compound of Formulae I, II or III in the presence of an EC₈₀ concentration of DA (1 µM for D2 DAR and 30 nM for D3 DAR) for 5 min. BRET signal was determined by quantifying and calculating the ratio of the light emitted by mVenus (525 nm) over that emitted by RLuc8 (485 nm) using a PHERAstar FSX Microplate Reader (BMG Labtech).

**Gₒ BRET activation assay:** To determine if a test compound of Formulae I, II, or III displays functional selectivity (the ability to selectively activate one signaling pathway versus another), a Gₒ BRET activation assay was utilized as described in the protocol below (Table 6). Briefly, HEK293T cells transiently expressing either the D2 DAR or D3 DAR and Gα_{oA}-Rluc8, untagged-β₁, and mVenus-γ₂ were harvested with EBSS-, plated in 96-well white plates at 20,000 cells/well in DPBS and incubated at RT for 45 min. Cells were incubated with 5 µM coelenterazine h (Nanolight Technology, Pinetop, AZ) for 5 min, then stimulated with the indicated concentrations of either sulpiride or a test compound of Formulae I, II, or III in the presence of an EC₈₀ concentration of DA (1 µM for D2 DAR and 30 nM for D3 DAR) for 5 min. BRET signal was determined by quantifying and calculating the ratio of the light emitted by mVenus (525 nm) over that emitted by RLuc8 (485 nm) using a PHERAstar FSX Microplate Reader (BMG Labtech).

| Table 6. Protocol for the Gₒ BRET activation assay | | | |
|---|---|---|---|
| Sequence | Parameter | Value | Description |
| 1 | Cells | 100 µL | 200,000 cells/mL in 96 well plate |
| 2 | Time | 45 min | Incubate at room temperature |
| 3 | Reagent | 50 µL | 25 µM coelenterazine |
| 4 | Time | 5 min | Incubate at room temperature |
| 5 | Reagent | 100 µL | Test compound |
| 6 | Time | 5 min | Incubate at room temperature |
| 7 | Detector | BRET signal | 525 nM/485 nM ratio using PHERAstar FSX Microplate Reader (BMG Labtech) |

Certain compounds have been tested in the D3 Go BRET activation assay and their activity is present in Table 7. A "*" is used to denote compounds with an IC₅₀ greater than 50 micromolar, a "**" denotes compounds with an IC₅₀ between 10 micromolar and 50 micromolar, a "***" denotes compounds with an IC₅₀ between 1 micromolar and 10 micromolar, and "****" indicates and IC₅₀ of less than 1 micromolar. A "+" indicates a detectable Imax of between 80% and 100%, a "++" indicates a detectable Imax of more than 100%, indicative of possible inverse agonist activity. NA means not active.

| Table 7. D3 Go BRET IC₅₀ for exemplified compounds | | |
|---|---|---|
| Cmpd. # | D3 Go BRET IC₅₀ | D3 Go BRET Imax |
| 1 | ** | ++ |
| 2 | *** | ++ |
| 3 | *** | ++ |
| 4 | ** | ++ |
| 5 | *** | + |
| 6 | ** | ++ |
| 7 | *** | ++ |
| 8 | *** | + |
| 9 | NA | NA |
| 10 | * | Curve Incomplete |
| 11 | ** | ++ |
| 12 | * | Curve Incomplete |
| 13 | ** | ++ |
| 14 | *** | ++ |
| 15 | ** | + |
| 16 | ** | ++ |
| 17 | *** | ++ |
| 18 | *** | + |
| 19 | ** | ++ |
| 20 | * | Curve Incomplete |
| 21 | ** | ++ |
| 22 | ** | ++ |
| 23 | *** | ++ |
| 24 | * | Curve Incomplete |
| 25 | * | Curve Incomplete |
| 26 | Not Determined | Not Determined |
| 27 | *** | ++ |
| 28 | *** | + |
| 29 | ** | ++ |
| 31 | ** | ++ |
| 32 | *** | ++ |
| 33 | ** | ++ |
| 34 | ** | + |
| 35 | **** | + |
| 36 | *** | + |
| 37 | * | Curve Incomplete |
| 38 | *** | ++ |
| 39 | *** | ++ |
| 40 | ** | ++ |
| 41 | Not antagonist | Not antagonist |
| 42 | ** | ++ |
| 43 | * | Curve Incomplete |
| 44 | NA | NA |
| 45 | Not Determined | Not Determined |

**cAMP CAMYEL biosensor assay:** HEK293 cells transiently expressing the D2 DAR or D3 DAR and the CAMYEL cAMP biosensor (yellow fluorescence protein-Epac-Rluc) were harvested with EBSS-, plated in 96-well white plates at 20,000 cells/well in DPBS and incubated at room temperature for 45 min. Cells were pretreated for 5 min with 10 µM forskolin and 10 µM propranolol (to block endogenous β-adrenergic receptors), then incubated with 5 µM coelenterazine h (Nanolight Technology, Pinetop, AZ) for 5 min, then stimulated with the indicated concentrations of either sulpiride or a test compound of Formulae I, II or III in the presence of an EC₈₀ concentration of DA (1 µM for D2 DAR and 30 nM for D3 DAR) for 5 min. BRET signal was determined by quantifying and calculating the ratio of the light emitted by mVenus (525 nm) over that emitted by RLuc8 (485 nm) using a PHERAstar FSX Microplate Reader (BMG Labtech).

**ERK1/2 Phosphorylation assay:** ERK1/2 phosphorylation was measured using the Alphascreen SureFire Ultra ERK kit (PerkinElmer, Waltham, USA). CHO-K1 cells stably expressing either the D2 DAR or D3 DAR were seeded into 384-well small volume white plates at a density of 40,000 cells/well in serum-free Ham's F12 media overnight. Cells were stimulated with the indicated concentrations of either sulpiride or a test compound of Formulae I, II or III in the presence of an EC₈₀ concentration of DA (1 µM for D2 DAR and 30 nM for D3 DAR) for 15 min, followed by cell lysis as specified by manufacture's protocol. The plate was shaken for 10 min at RT, followed by the addition of Surefire activation buffer, Surefire reaction buffer, Alphascreen acceptor beads, and Alphascreen donor beads in ratios specified by the manufacturer. The plate was incubated in the dark for 2 h, then read using a PHERAstar FSX Microplate Reader (BMG Labtech).

**Mouse Plasma and Brain Tissue Sampling:** The pharmacokinetic study was conducted by CRO Pharmaron (Beijing, China). The levels of cmp 17 in mouse plasma and brain tissue samples were assessed as follows. A single IP dose (40 mg/kg) of cmp 17 was administered to male CD1 mice. The formulation consisted of NMP/Solutol/PEG-400/normal saline (v/v/v/v, 10:5:30:55). Plasma and brain samples were collected 0.5 h, 1 h, 2 h, 4 h and 8 h post dose. Brain samples were homogenized at a ratio of 1:3 with PBS (W/V, 1:3). The desired serial concentrations of working solutions were achieved by diluting stock solution of analyte with 50% acetonitrile in water solution. 10 µL of working solutions (0.5, 1, 2, 5, 10, 50, 100, 500, 1000, 10000 ng/mL) were added to 10 µL of the blank CD1 mouse plasma/brain homogenate to achieve calibration standards of 0.5~10000 ng/mL (0.5, 1, 2, 5, 10, 50, 100, 500, 1000, 10000 ng/mL) in a total volume of 20µL. Five quality control samples at 1 ng/mL, 2 ng/mL, 5 ng/mL, 100 ng/mL and 8000 ng/mL for plasma/brain homogenate were prepared independently of those used for the calibration curves. 20µL standards, 20 µL QC samples and 20 µL unknown samples (10 µL plasma/brain homogenate with 10 µL blank solution) were added to 200 µL of acetonitrile containing IS mixture for precipitating protein respectively. Then the samples were vortexed for 30 s. After centrifugation at 4 °C, 4700 rpm for 15 min, the supernatant was diluted at a ratio of 1:2 with water, and 10 µL of diluted supernatant was injected into the LC/MS/MS system (SCIEX AB API 4000) for quantitative analysis. Three mice were used for each time point collected, and the data represent means ± SD.

## Claims

1. A compound of Formula I 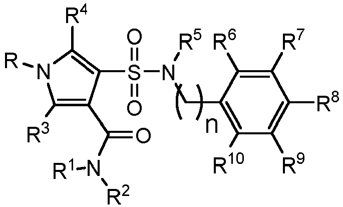 or a pharmaceutically acceptable salt thereof, wherein:
n is 0 or 1;
R is H;
R¹ is chosen from hydrogen, methyl, and ethyl, and R² is chosen from methyl and ethyl; or
R¹ and R² are joined to form a heterocycloalkyl ring optionally containing 1 additional heteroatom chosen from N, O, and S, and the heterocycloalkyl ring is unsubstituted or substituted by 1 or more substituents independently chosen from halogen, hydroxyl, amino, C₁-C₃alklyl, C₁-C₃alkoxy, C₁-C₂haloalkyl, and C₁-C₂haloalkoxy;
R³ and R⁴ are independently chosen from hydrogen, methyl, ethyl, and trifluoromethyl
R⁵ is chosen from hydrogen, methyl, and ethyl; and
R⁶, R⁷, R⁸, R⁹, and R¹⁰ are independently chosen from hydrogen, halogen, hydroxyl, amino, -SF₅, C₁-C₄alkyl, C₁-C₄alkoxy, C₁-C₄alkylester, C₃-C₆cycloalkyl, C₁-C₂haloalkyl, and C₁-C₂haloalkoxy; wherein at least one of R⁷, R⁸, and R⁹ is not hydrogen.

2. The compound or salt of claim 1, wherein R¹ and R² are both methyl, R¹ is methyl and R² is ethyl, or R¹ is hydrogen and R² is ethyl.

3. The compound or salt of claim 1, wherein R¹ and R² are joined to form a heterocycloalkyl ring selected from an azetidinyl, pyrrolidinyl, piperidinyl, or piperazinyl ring and the heterocycloalkyl ring is unsubstituted or substituted by 1 or more substituents independently chosen from halogen, hydroxyl, amino, C₁-C₃alkyl, C₁-C₃alkoxy, C₁-C₂haloalkyl, and C₁-C₂haloalkoxy, optionally wherein the azetidinyl, pyrrolidinyl, piperidinyl, or piperazinyl ring is unsubstituted or substituted with one or two halogen or methyl substituents.

4. The compound or salt of any one of claims 1 to 3, wherein at least one of the following conditions is met
(i) R³ and R⁴ are both methyl;
(ii) R⁵ is hydrogen;
(iii) R⁶, R⁹, and R¹⁰ are all hydrogen; and/or
(iv) wherein R⁷ and R⁸ are independently chosen from hydrogen, fluoro, chloro, bromo, methyl, ethyl, and trifluoromethyl, where at least one of R⁷ and R⁸ is not hydrogen.

5. The compound or salt of any one of claims 1 to 4, wherein n is 1.

6. The compound or salt of any one of claims 1 to 4, wherein n is 0.

7. The compound or salt of claim 1, of Formula I-A

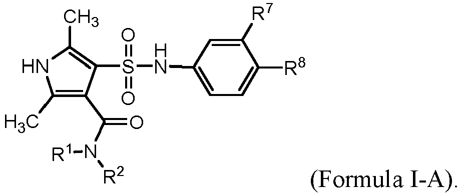

8. The compound or salt of claim 7, wherein
R¹ and R² are both methyl, or R¹ is hydrogen and R² is ethyl, or R¹ is methyl and R² is ethyl; or
R¹ and R² are joined to form a heterocycloalkyl ring selected from an azetidinyl, pyrrolidinyl, piperidinyl, or piperazinyl ring, which ring is unsubstituted or substituted with or more methyl or fluoro substituents; and
R⁷ and R⁸ are chosen from hydrogen, halogen, methyl, ethyl, trifluoromethyl and cyclopropyl, wherein at least one of R⁷ and R⁸ is not hydrogen.

9. The compound or salt of claim 1, of Formula I-B 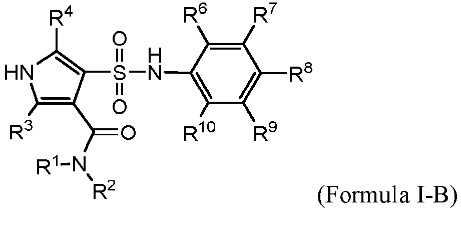
R¹ is methyl and R² is ethyl, or R¹ is hydrogen and R² is ethyl, or R¹ is methyl and R² is ethyl; or
R¹ and R² are joined to form a heterocycloalkyl ring selected from an azetidinyl, pyrrolidinyl, piperidinyl, or piperazinyl ring, which ring is unsubstituted or substituted with or more methyl or fluoro substituents; and
R³ and R⁴ are both methyl, or one of R³ and R⁴ is hydrogen and the other is trifluoromethyl, or oner of R³ and R⁴ is methyl and the other is trifluoromethyl; and
R⁶, R⁷, R⁸, R⁹, and R¹⁰ are independently chosen from hydrogen, halogen, C₁-C₂alkyl, C₁-C₂alkoxy, cyclopropyl, SF₅, and CF₃, wherein at least one of R⁶, R⁷, R⁸, R⁹, and R¹⁰ is not hydrogen and no more than 4 of R⁶, R⁷, R⁸, R⁹, and R¹⁰ are non-hydrogen.

10. The compound or salt of claim 9, wherein
R¹ and R² are both methyl, or
R¹ and R² are joined to form a heterocycloalkyl ring selected from an azetidinyl, pyrrolidinyl, piperidinyl, or piperazinyl ring and the heterocycloalkyl ring is unsubstituted or substituted by 1 or more substituents independently chosen from halogen, hydroxyl, amino, C₁-C₃alkyl, C₁-C₃alkoxy, C₁-C₂haloalkyl, and C₁-C₂haloalkoxy; and
R⁷ and R⁸ are independently chosen from hydrogen, fluoro, chloro, bromo, methyl, ethyl, and trifluoromethyl, where at least one of R⁷ and R⁸ is not hydrogen.

11. A compound or salt of claim 1, wherein the compound is
| Cmp. No. | Structure | Cmp. No. | Structure |
|---|---|---|---|
| 1 | 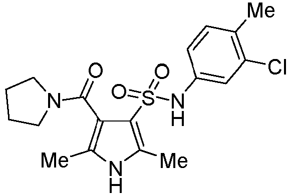 | 21 | 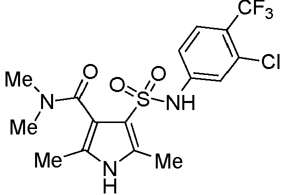 |
| 2 | 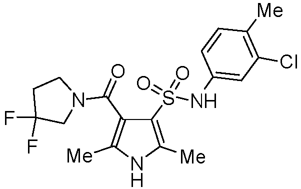 | 23 | 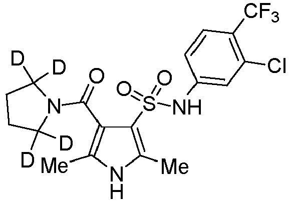 |
| 3 | 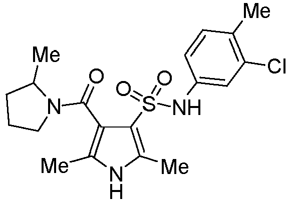 | 24 | 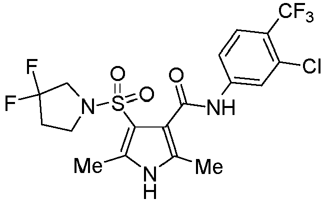 |
| 4 | 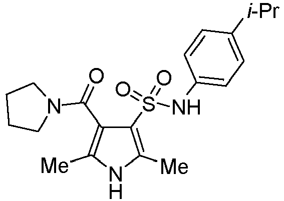 | 27 | 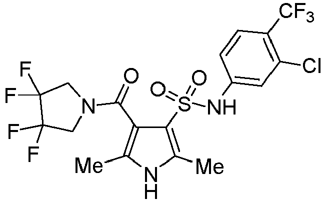 |
| 5 | 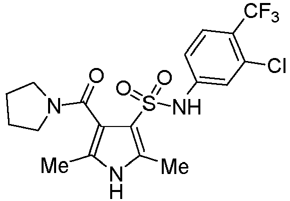 | 28 | 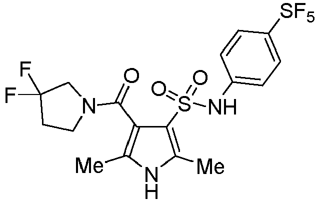 |
| 6 | 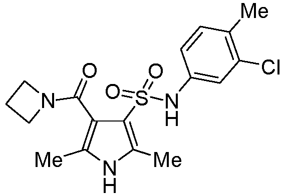 | 29 | 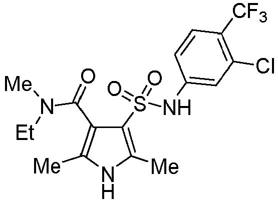 |
| 7 | 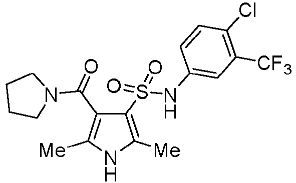 | 30 | 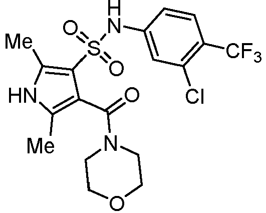 |
| 8 | 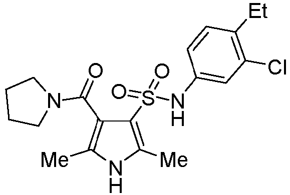 | | 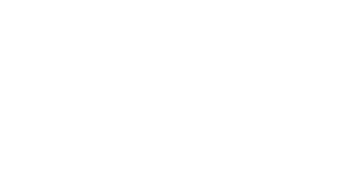 |
| 9 | 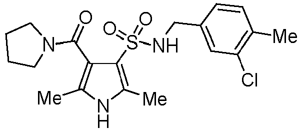 | 32 | 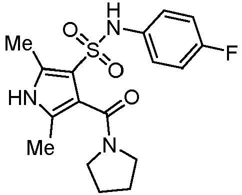 |
| 10 | 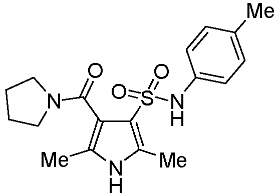 | 33 | 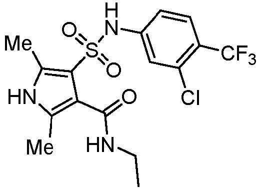 |
| 11 | 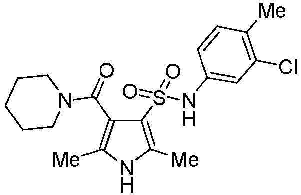 | 34 | 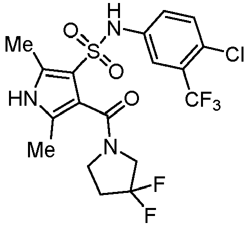 |
| 12 | 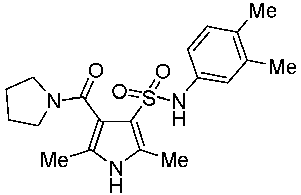 | 35 | 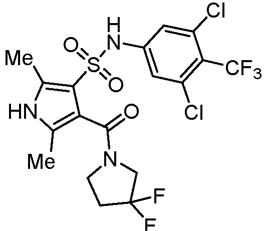 |
| 13 | 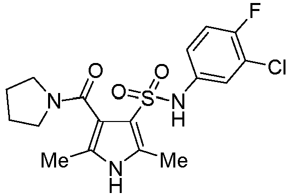 | 36 | 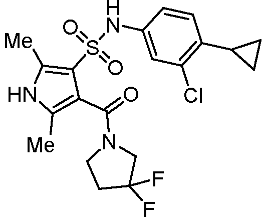 |
| 14 | 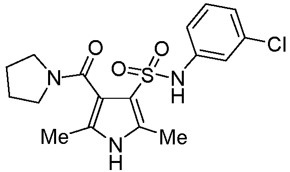 | 37 | 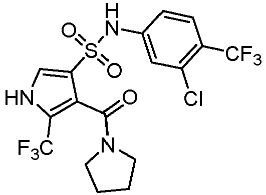 |
| 15 | 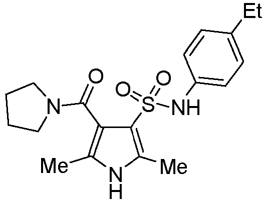 | 38 | 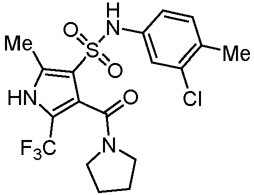 |
| 16 | 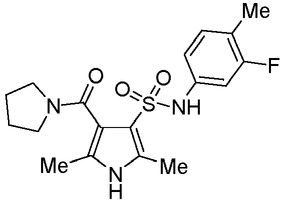 | 39 | 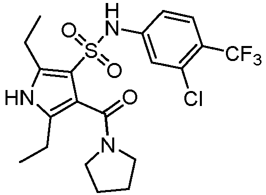 |
| 17 | 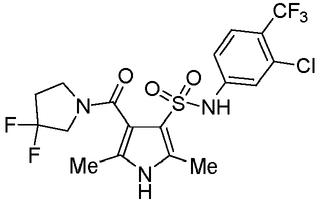 | 40 | 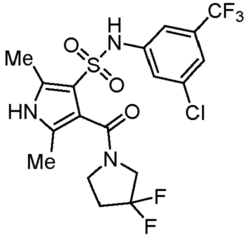 |
| 18 | 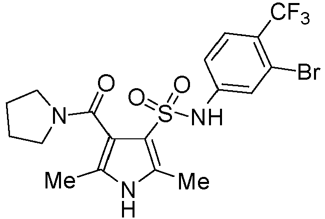 | 41 | 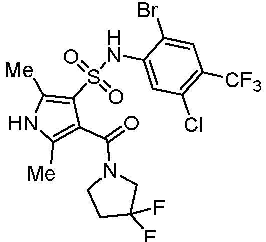 |
| 19 | 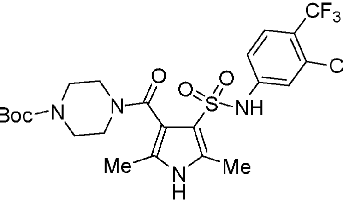 | 42 | 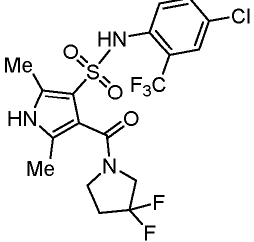 |
| 20 | 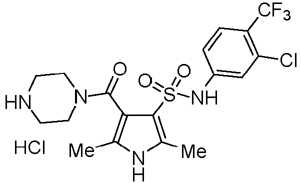 | 43 | 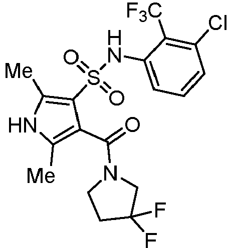 |

12. A pharmaceutical composition comprising a compound or pharmaceutically acceptable salt thereof of any one of claims 1 to 11, together with a pharmaceutically acceptable excipient.

13. A compound of any one of claims 1 to 11 or a pharmaceutically acceptable salt thereof for use in a method of treating a central nervous system disorder, wherein the central nervous system disorder is Parkinson's disease or a related syndrome, dyskinesia, a neurodegenerative disorder, schizophrenia, cognitive dysfunction, substance use disorder and/or withdrawal syndrome in a patient.

14. The compound for use according to claim 13, wherein the disorder is selected from obesity, anxiety, depression (optionally refractory depression, MDD, or bipolar depression), psychosis (optionally psychosis associated with dementia, optionally hallucinations in progressive Parkinson's disease or paranoid ideas), schizophrenia, a sleep disorder (optionally a sleep disorder associated with schizophrenia or another psychiatric or neurological disease), a sexual disorder, migraine, a condition associated with headaches, social phobias, agitation in dementia (e.g., agitation in Alzheimer's disease), agitation in autism and related autistic disorders, a gastrointestinal disorder such as gastrointestinal motility dysfunction, and dementia, such as dementia of Alzheimer's disease or Parkinson's disease; a mood disorder; or substance use disorder and/or withdrawal syndrome, for example, opiate dependence and / or alcohol dependence or withdrawal from drug or alcohol dependence (for example, opiate dependence); or compulsive overeating.

15. The compound for use according to claim 14, wherein the disorder is a substance use disorder and/or a withdrawal syndrome; optionally wherein the substance use disorder is related to substances comprising tobacco, alcohol, caffeine, opioids, cannabis, stimulants, sedatives, inhalants, hypnotics or anxiolytics, or hallucinogen.

## Patentansprüche

1. Verbindung der Formel I 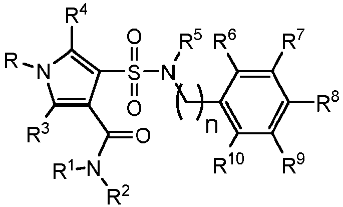 oder pharmazeutisch verträgliches Salz davon, wobei:
n gleich 0 oder 1 ist;
R gleich H;
R¹ ausgewählt ist aus Wasserstoff, Methyl und Ethyl, und R² ausgewählt ist aus Methyl und Ethyl; oder
R¹ und R² zusammengefügt sind, um einen Heterocycloalkylring zu bilden, der gegebenenfalls 1 zusätzliches Heteroatom enthält, das ausgewählt ist aus N, O und S, und der Heterocycloalkylring unsubstituiert oder durch 1 oder mehrere Substituenten substituiert ist, die unabhängig voneinander ausgewählt sind aus Halogen, Hydroxyl, Amino, C₁-C₃Alklyl, C₁-C₃Alkoxy, C₁-C₂Halogenalkyl und C₁-C₂Halogenalkoxy;
R³ und R⁴ unabhängig voneinander ausgewählt sind aus Wasserstoff, Methyl, Ethyl und Trifluormethyl,
R⁵ ausgewählt ist aus Wasserstoff, Methyl und Ethyl; und
R⁶, R⁷, R⁸, R⁹ und R¹⁰ unabhängig voneinander ausgewählt sind aus Wasserstoff, Halogen, Hydroxyl, Amino, -SF₅, C₁-C₄Alkyl, C₁-C₄Alkoxy, C₁-C₄Alkylester, C₃-C₆Cycloalkyl, C₁-C₂Halogenalkyl und C₁-C₂Halogenalkoxy; wobei mindestens eines von R⁷, R⁸ und R⁹ nicht Wasserstoff ist.

2. Verbindung oder Salz nach Anspruch 1, wobei R¹ und R² beide Methyl sind, R¹ Methyl ist und R² Ethyl ist, oder R¹ Wasserstoff ist und R² Ethyl ist.

3. Verbindung oder Salz nach Anspruch 1, wobei R¹ und R² zusammengefügt sind, um einen Heterocycloalkylring ausgewählt aus einem Azetidinyl-, Pyrrolidinyl-, Piperidinyl- oder Piperazinylring zu bilden, und der Heterocycloalkylring unsubstituiert oder durch einen oder mehrere Substituenten substituiert ist, die unabhängig voneinander ausgewählt sind aus Halogen, Hydroxyl, Amino, C₁-C₃Alkyl, C₁-C₃Alkoxy, C₁-C₂Halogenalkyl und C₁-C₂Halogenalkoxy, wobei der Azetidinyl-, Pyrrolidinyl-, Piperidinyl- oder Piperazinylring gegebenenfalls unsubstituiert oder mit einem oder zwei Halogen- oder Methylsubstituenten substituiert ist.

4. Verbindung oder Salz nach einem der Ansprüche 1 bis 3, wobei mindestens eine der folgenden Bedingungen erfüllt ist:
(i) R³ und R⁴ sind beide Methyl;
(ii) R⁵ ist Wasserstoff;
(iii) R⁶, R⁹ und R¹⁰ sind alle Wasserstoff; und/oder
(iv) wobei R⁷ und R⁸ unabhängig voneinander ausgewählt sind aus Wasserstoff, Fluor, Chlor, Brom, Methyl, Ethyl und Trifluormethyl, wobei mindestens eines von R⁷ und R⁸ nicht Wasserstoff ist.

5. Verbindung oder Salz nach einem der Ansprüche 1 bis 4, wobei n gleich 1 ist.

6. Verbindung oder Salz nach einem der Ansprüche 1 bis 4, wobei n gleich 0 ist.

7. Verbindung oder Salz nach Anspruch 1 der Formel I-A

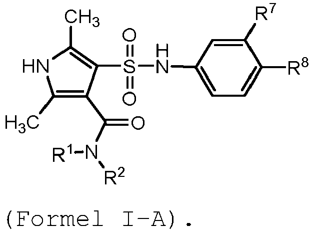

8. Verbindung oder Salz nach Anspruch 7, wobei
R¹ und R² beide Methyl sind, oder R¹ Wasserstoff ist und R² Ethyl ist, oder R¹ Methyl ist und R² Ethyl ist; oder
R¹ und R² zusammengefügt sind, um einen Heterocycloalkylring ausgewählt aus einem Azetidinyl-, Pyrrolidinyl-, Piperidinyl- oder Piperazinylring zu bilden, wobei der Ring unsubstituiert oder mit einem oder mehreren Methyl- oder Fluorsubstituenten substituiert ist; und
R⁷ und R⁸ ausgewählt sind aus Wasserstoff, Halogen, Methyl, Ethyl, Trifluormethyl und Cyclopropyl, wobei mindestens eines von R⁷ und R⁸ nicht Wasserstoff ist.

9. Verbindung oder Salz nach Anspruch 1 der Formel I-B 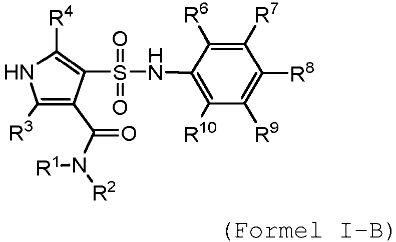
R¹ Methyl ist und R² Ethyl ist, oder R¹ Wasserstoff ist und R² Ethyl ist, oder R¹ Methyl ist und R² Ethyl ist; oder
R¹ und R² zusammengefügt sind, um einen Heterocycloalkylring ausgewählt aus einem Azetidinyl-, Pyrrolidinyl-, Piperidinyl- oder Piperazinylring zu bilden, wobei der Ring unsubstituiert oder mit einem oder mehreren Methyl- oder Fluorsubstituenten substituiert ist; und
R³ und R⁴ beide Methyl sind, oder eines von R³ und R⁴ Wasserstoff ist und das andere Trifluormethyl ist, oder eines von R³ und R⁴ Methyl ist und das andere Trifluormethyl ist; und
R⁶, R⁷, R⁸, R⁹ und R¹⁰ unabhängig voneinander ausgewählt sind aus Wasserstoff, Halogen, C₁-C₂Alkyl, C₁-C₂Alkoxy, Cyclopropyl, SF₅ und CF₃, wobei mindestens eines von R⁶, R⁷, R⁸, R⁹ und R¹⁰ nicht Wasserstoff ist und nicht mehr als 4 von R⁶, R⁷, R⁸, R⁹ und R¹⁰ Nicht-Wasserstoff sind.

10. Verbindung oder Salz nach Anspruch 9, wobei
R¹ und R² beide Methyl sind, oder
R¹ und R² zusammengefügt sind, um einen Heterocycloalkylring aus einem Azetidinyl-, Pyrrolidinyl-, Piperidinyl- oder Piperazinylring zu bilden, und der Heterocycloalkylring unsubstituiert oder durch 1 oder mehrere Substituenten substituiert ist, die unabhängig voneinander ausgewählt sind aus Halogen, Hydroxyl, Amino, C₁-C₃Alklyl, C₁-C₃Alkoxy, C₁-C₂Halogenalkyl und C₁-C₂Halogenalkoxy; und
R⁷ und R⁸ unabhängig voneinander ausgewählt sind aus Wasserstoff, Fluor, Chlor, Brom, Methyl, Ethyl und Trifluormethyl, wobei mindestens eines von R⁷ und R⁸ nicht Wasserstoff ist.

11. Verbindung oder Salz nach Anspruch 1, wobei die Verbindung eine Verbindung Folgende ist:
| Verbindung Nr. | Struktur | Verbindung Nr. | Struktur |
|---|---|---|---|
| 1 | 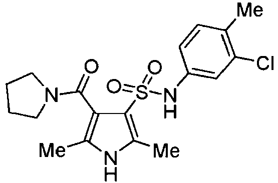 | 21 | 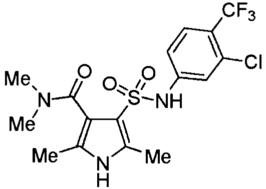 |
| 2 | 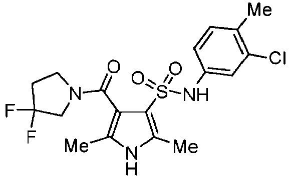 | 23 | 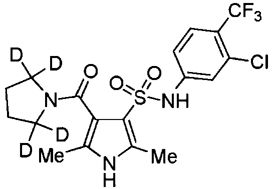 |
| 3 | 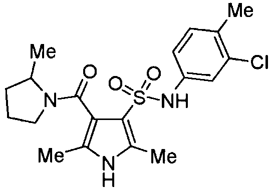 | 24 | 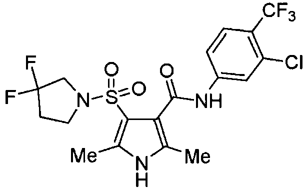 |
| 4 | 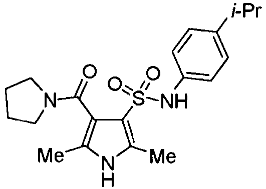 | 27 | 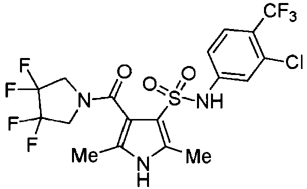 |
| 5 | 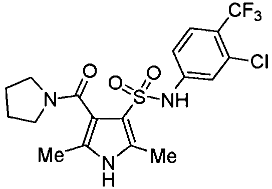 | 28 | 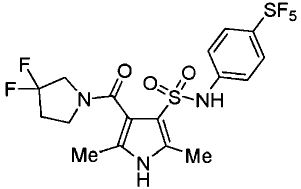 |
| 6 | 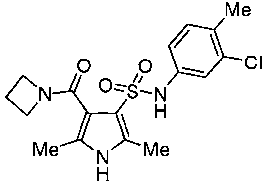 | 29 | 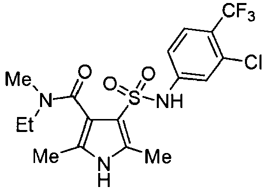 |
| 7 | 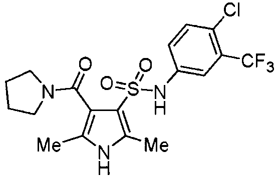 | 30 | 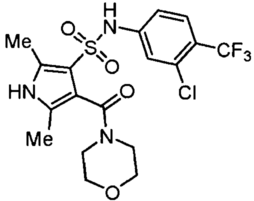 |
| 8 | 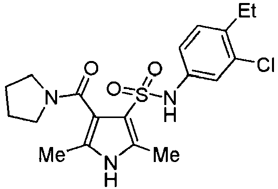 | | |
| 9 | 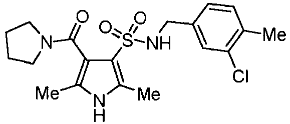 | 32 | 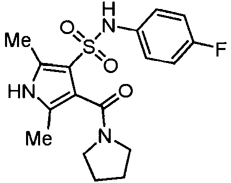 |
| 10 | 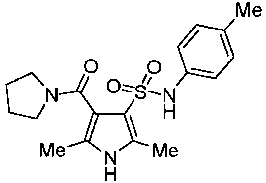 | 33 | 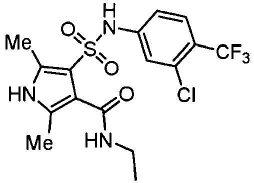 |
| 11 | 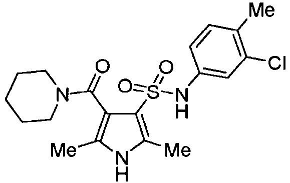 | 34 | 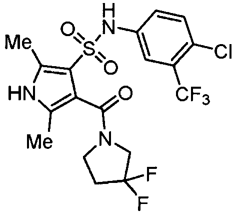 |
| 12 | 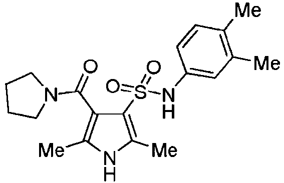 | 35 | 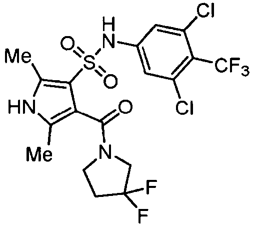 |
| 13 | 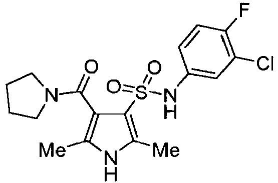 | 36 | 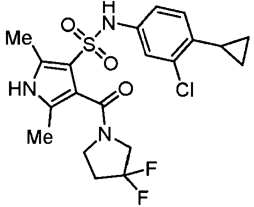 |
| 14 | 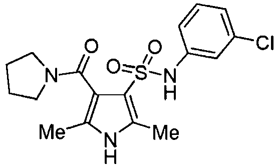 | 37 | 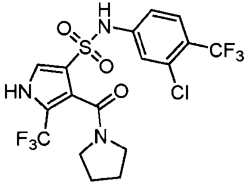 |
| 15 | 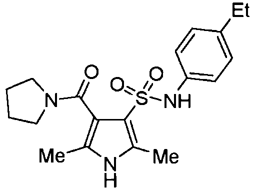 | 38 | 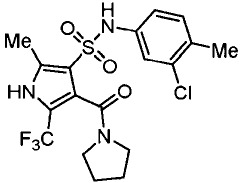 |
| 16 | 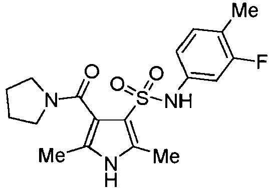 | 39 | 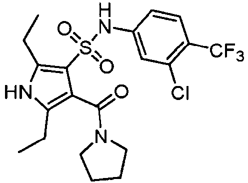 |
| 17 | 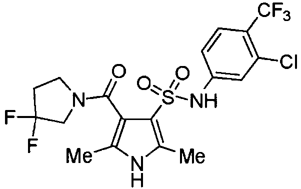 | 40 | 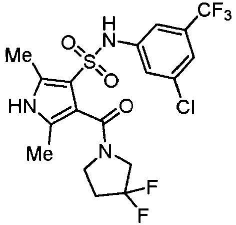 |
| 18 | 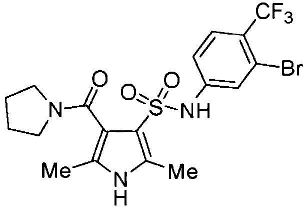 | 41 | 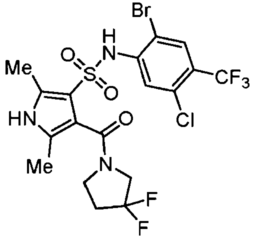 |
| 19 | 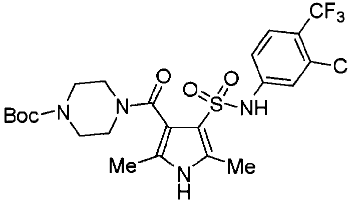 | 42 | 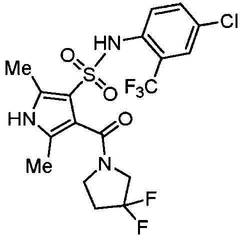 |
| 20 | 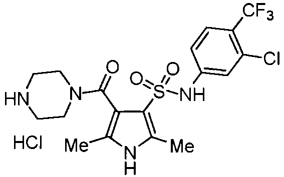 | 43 | 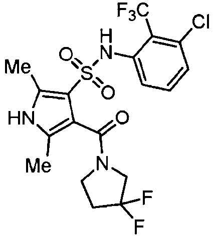 |

12. Pharmazeutische Zusammensetzung, die eine Verbindung oder ein pharmazeutisch verträgliches Salz davon nach einem der Ansprüche 1 bis 11 zusammen mit einem pharmazeutisch verträglichen Hilfsstoff umfasst.

13. Verbindung nach einem der Ansprüche 1 bis 11 oder ein pharmazeutisch verträgliches Salz davon zur Verwendung in einem Verfahren zur Behandlung einer Störung des zentralen Nervensystems, wobei die Störung des zentralen Nervensystems die Parkinson-Krankheit oder ein verwandtes Syndrom, Dyskinesie, eine neurodegenerative Störung, Schizophrenie, kognitive Dysfunktion, eine Störung infolge von Substanzgebrauch und/oder Entzugssyndrom bei einem Patienten ist.

14. Verbindung zur Verwendung nach Anspruch 13, wobei die Störung ausgewählt ist aus Fettleibigkeit, Angst, Depression (optional refraktäre Depression, MDD oder bipolare Depression), Psychose (optional Psychose in Verbindung mit Demenz, optional Halluzinationen bei progressiver Parkinson-Krankheit oder paranoide Ideen), Schizophrenie, einer Schlafstörung (optional einer Schlafstörung in Verbindung mit Schizophrenie oder einer anderen psychiatrischen oder neurologischen Erkrankung), einer sexuellen Störung, Migräne, einem Zustand in Verbindung mit Kopfschmerzen, sozialen Phobien, Unruhe bei Demenz (z. B. Unruhe bei der Alzheimer-Krankheit), Unruhe bei Autismus und verwandten autistischen Störungen, einer gastrointestinalen Störung, wie gastrointestinale Motilitätsstörungen und Demenz, wie Demenz bei der Alzheimer-Krankheit oder der Parkinson-Krankheit; einer Stimmungsstörung; oder einer Störung infolge von Substanzgebrauch und/oder einem Entzugssyndrom, z. B. Opiatabhängigkeit und/oder Alkoholabhängigkeit oder Entzug bei Drogen- oder Alkoholabhängigkeit (z. B. Opiatabhängigkeit); oder zwanghaftem Überessen.

15. Verbindung zur Verwendung nach Anspruch 14, wobei die Störung eine Störung infolge von Substanzgebrauch und/oder ein Entzugssyndrom ist; optional, wobei die Störung infolge von Substanzgebrauch mit Substanzen zusammenhängt, die Tabak, Alkohol, Koffein, Opioide, Cannabis, Stimulanzien, Sedativa, Inhalationsmittel, Hypnotika oder Anxiolytika oder Halluzinogene umfassen.

## Revendications

1. Composé de formule I 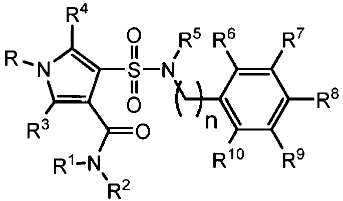 ou un sel pharmaceutiquement acceptable de celui-ci, dans lequel :
n vaut 0 ou 1 ;
R est H ;
R¹ est choisi parmi l'hydrogène, méthyle et éthyle, et R² est choisi parmi méthyle et éthyle ; ou
R¹ et R² sont joints pour former un cycle hétérocycloalkyle contenant éventuellement 1 hétéroatome additionnel choisi parmi N, O et S, et le cycle hétérocycloalkyle est non substitué ou substitué par 1 ou plusieurs substituants indépendamment choisis parmi les halogènes, hydroxyle, amino, alkyle en C₁ à C₃, alcoxy en C₁ à C₃, halogénoalkyle en C₁ à C₂ et halogénoalcoxy en C₁ à C₂ ;
R³ et R⁴ sont indépendamment choisis parmi l'hydrogène, méthyle, éthyle et trifluorométhyle ;
R⁵ est choisi parmi l'hydrogène, méthyle et éthyle ; et
R⁶, R⁷, R⁸, R⁹ et R¹⁰ sont indépendamment choisis parmi l'hydrogène, les halogènes, hydroxyle, amino, -SF₅, alkyle en C₁ à C₄, alcoxy en C₁ à C₄, esters alkyliques en C₁ à C₄, cycloalkyle en C₃ à C₆, halogénoalkyle en C₁ à C₂, et halogénoalcoxy en C₁ à C₂ ; parmi lesquels au moins l'un de R⁷, R⁸ et R⁹ n'est pas l'hydrogène.

2. Composé ou sel selon la revendication 1, dans lequel R¹ et R² sont tous deux méthyle, R¹ est méthyle et R² est éthyle, ou R¹ est l'hydrogène et R² est éthyle.

3. Composé ou sel selon la revendication 1, dans lequel R¹ et R² sont joints pour former un cycle hétérocycloalkyle choisi parmi les cycles azétidinyle, pyrrolidinyle, pipéridinyle et pipérazinyle, et le cycle hétérocycloalkyle est non substitué ou substitué par 1 ou plusieurs substituants indépendamment choisis parmi les halogènes, hydroxyle, amino, alkyle en C₁ à C₃, alcoxy en C₁ à C₃, halogénoalkyle en C₁ à C₂, et halogénoalcoxy en C₁ à C₂, éventuellement dans lequel le cycle azétidinyle, pyrrolidinyle, pipéridinyle ou pipérazinyle est non substitué ou substitué par un ou deux substituants halogènes ou méthyle.

4. Composé ou sel selon l'une quelconque des revendications 1 à 3, dans lequel au moins l'une des conditions suivantes est satisfaite :
(i) R³ et R⁴ sont tous deux méthyle ;
(ii) R⁵ est l'hydrogène ;
(iii) R⁶, R⁹ et R¹⁰ sont tous des hydrogènes ; et/ou
(iv) R⁷ et R⁸ sont indépendamment choisis parmi l'hydrogène, fluoro, chloro, bromo, méthyle, éthyle et trifluorométhyle, et au moins l'un de R⁷ et R⁸ n'est pas l'hydrogène.

5. Composé ou sel selon l'une quelconque des revendications 1 à 4, dans lequel n vaut 1.

6. Composé ou sel selon l'une quelconque des revendications 1 à 4, dans lequel n vaut 0.

7. Composé ou sel selon la revendication 1, de formule I-A

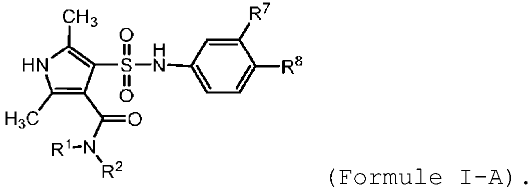

8. Composé ou sel selon la revendication 7, dans lequel R¹ et R² sont tous deux méthyle, ou R¹ est l'hydrogène et R² est éthyle, ou R¹ est méthyle et R² est éthyle ; ou
R¹ et R² sont joints pour former un cycle hétérocycloalkyle choisi parmi les cycles azétidinyle, pyrrolidinyle, pipéridinyle et pipérazinyle, lequel cycle est non substitué ou substitué par un ou plusieurs substituants méthyle ou fluoro ; et
R⁷ et R⁸ sont choisis parmi l'hydrogène, les halogènes, méthyle, éthyle, trifluorométhyle et cyclopropyle, et au moins l'un de R⁷ et R⁸ n'est pas l'hydrogène.

9. Composé ou sel selon la revendication 1, de formule I-B 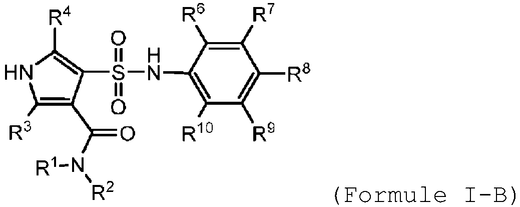
R¹ est méthyle et R² est éthyle, ou R¹ est l'hydrogène et R² est éthyle, ou R¹ est méthyle et R² est éthyle ; ou
R¹ et R² sont joints pour former un cycle hétérocycloalkyle choisi parmi les cycles azétidinyle, pyrrolidinyle, pipéridinyle et pipérazinyle, lequel cycle est non substitué ou substitué par un ou plusieurs substituants méthyle ou fluoro ; et
R³ et R⁴ sont tous deux méthyle, ou l'un de R³ et R⁴ est l'hydrogène et l'autre est trifluorométhyle, ou l'un de R³ et R⁴ est méthyle et l'autre est trifluorométhyle ; et
R⁶, R⁷, R⁸, R⁹ et R¹⁰ sont indépendamment choisis parmi l'hydrogène, les halogènes, alkyle en C₁ à C₂, alcoxy en C₁ à C₂, cyclopropyle, SF₅ et CF₃, parmi lesquels au moins l'un de R⁶, R⁷, R⁸, R⁹ et R¹⁰ n'est pas l'hydrogène et au plus 4 de R⁶, R⁷, R⁸, R⁹ et R¹⁰ ne sont pas des hydrogènes.

10. Composé ou sel selon la revendication 9, dans lequel
R¹ et R² sont tous deux méthyle, ou
R¹ et R² sont joints pour former un cycle hétérocycloalkyle choisi parmi les cycles azétidinyle, pyrrolidinyle, pipéridinyle et pipérazinyle et le cycle hétérocycloalkyle est non substitué ou substitué par 1 ou plusieurs substituants indépendamment choisis parmi les halogènes, hydroxyle, amino, alkyle en C₁ à C₃, alcoxy en C₁ à C₃, halogénoalkyle en C₁ à C₂ et halogénoalcoxy en C₁ à C₂ ; et
R⁷ et R⁸ sont indépendamment choisis parmi l'hydrogène, fluoro, chloro, bromo, méthyle, éthyle et trifluorométhyle, et au moins l'un de R⁷ et R⁸ n'est pas l'hydrogène.

11. Composé ou sel selon la revendication 1, dans lequel le composé est
| Comp. N° | Structure | Comp. N° | Structure |
|---|---|---|---|
| 1 | 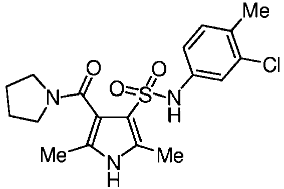 | 21 | 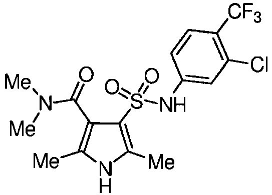 |
| 2 | 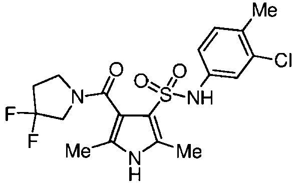 | 23 | 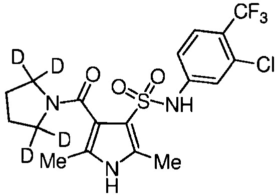 |
| 3 | 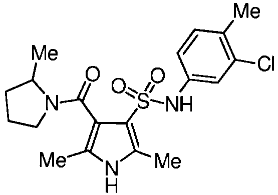 | 24 | 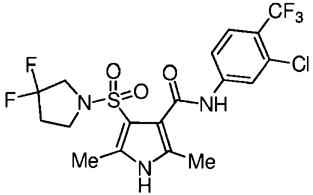 |
| 4 | 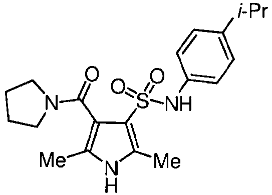 | 27 | 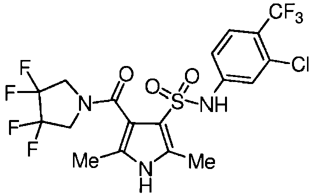 |
| 5 | 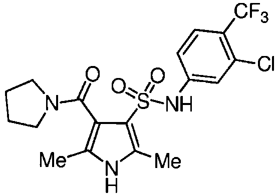 | 28 | 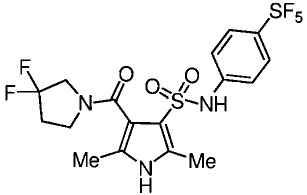 |
| 6 | 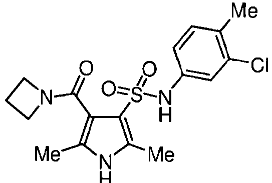 | 29 | 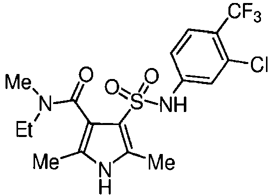 |
| 7 | 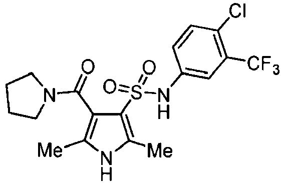 | 30 | 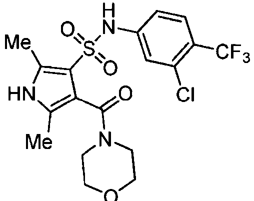 |
| 8 | 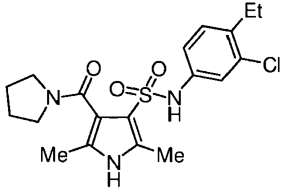 | | |
| 9 | 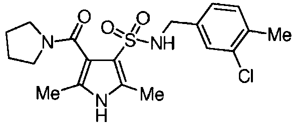 | 32 | 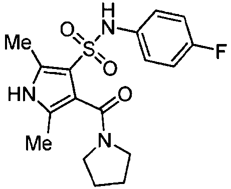 |
| 10 | 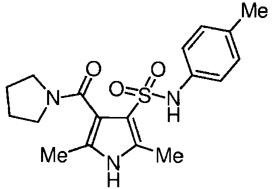 | 33 | 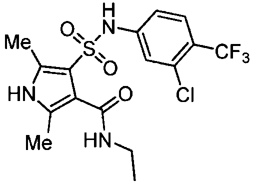 |
| 11 | 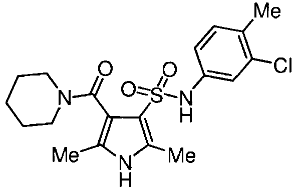 | 34 | 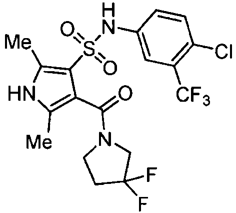 |
| 12 | 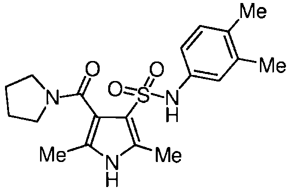 | 35 | 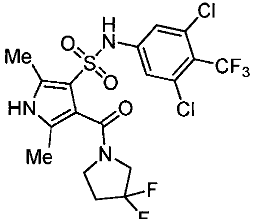 |
| 13 | 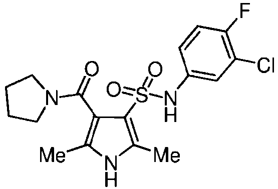 | 36 | 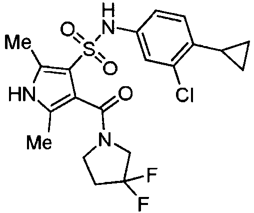 |
| 14 | 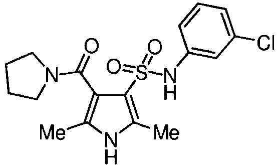 | 37 | 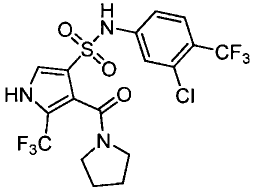 |
| 15 | 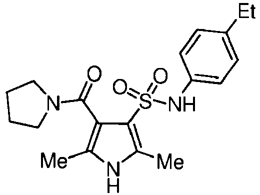 | 38 | 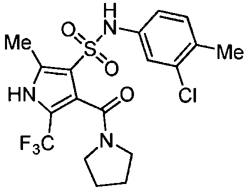 |
| 16 | 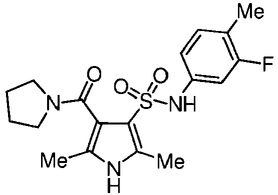 | 39 | 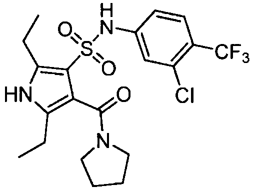 |
| 17 | 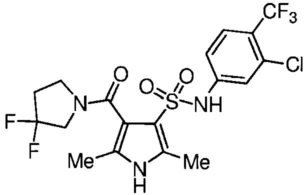 | 40 | 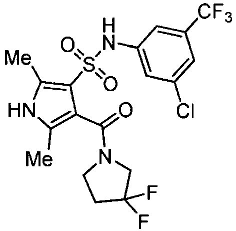 |
| 18 | 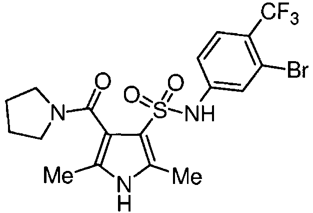 | 41 | 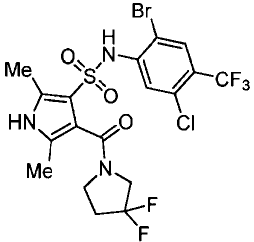 |
| 19 | 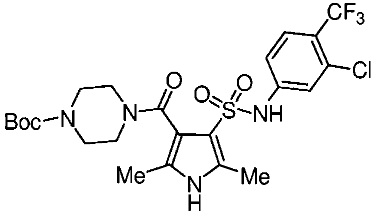 | 42 | 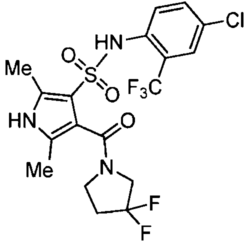 |
| 20 | 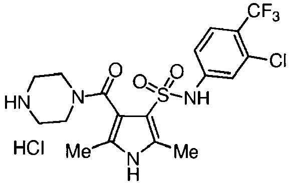 | 43 | 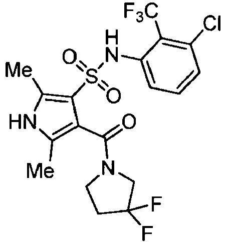 |

12. Composition pharmaceutique comprenant un composé ou un sel pharmaceutiquement acceptable de celui-ci de l'une quelconque des revendications 1 à 11, conjointement avec un excipient pharmaceutiquement acceptable.

13. Composé selon l'une quelconque des revendications 1 à 11 ou sel pharmaceutiquement acceptable de celui-ci, pour une utilisation dans une méthode de traitement d'un trouble du système nerveux central, dans lequel le trouble du système nerveux central est une maladie de Parkinson ou un syndrome apparenté, une dyskinésie, un trouble neurodégénératif, une schizophrénie, un dysfonctionnement cognitif, un trouble lié à l'usage de substances psychoactives et/ou un syndrome de sevrage chez un patient.

14. Composé pour une utilisation selon la revendication 13, dans lequel le trouble est choisi parmi une obésité, une anxiété, une dépression (éventuellement une dépression réfractaire, un trouble dépressif majeur, ou une dépression bipolaire), une psychose (éventuellement une psychose associée à une démence, éventuellement des hallucinations dans le contexte d'une maladie de parkinson ou des idées paranoïdes), une schizophrénie, un trouble du sommeil (éventuellement un trouble du sommeil associé à une schizophrénie ou à une autre maladie psychiatrique ou neurologique), un trouble sexuel, une migraine, un état associé à des céphalées, des phobies sociales, une agitation dans le contexte d'une démence (par exemple une agitation dans le contexte d'une maladie d'Alzheimer), une agitation dans le contexte d'un autisme et de troubles autistiques apparentés, un trouble gastrointestinal tel qu'un dysfonctionnement de la motilité gastrointestinale, et une démence, telle qu'une démence dans le contexte d'une maladie d'Alzheimer ou d'une maladie de Parkinson ; un trouble de l'humeur ; un trouble lié à l'usage de substances psychoactives et/ou un syndrome de sevrage, par exemple une dépendance aux opiacés et/ou un alcoolisme ou un sevrage d'une toxicomanie ou d'un alcoolisme (par exemple d'une dépendance aux opiacés) ; ou une hyperphagie compulsive.

15. Composé pour une utilisation selon la revendication 14, dans lequel le trouble est un trouble lié à l'usage de substances psychoactives et/ou à un syndrome de sevrage ; éventuellement dans lequel le trouble lié à l'usage de substances psychoactives est associé à des substances comprenant le tabac, l'alcool, la caféine, les opioïdes, le cannabis, les stimulants, les sédatifs, les inhalants, les hypnotiques ou anxiolytiques, ou les hallucinogènes.
